# EUROPEAN PATENT SPECIFICATION

(11) **EP 0 911 334 B1**
(45) Date of publication and mention of the grant of the patent: **21.05.2008**
(21) Application number: 98911102.6
(22) Date of filing: 30.03.1998
(51) Int. Cl.: C07D 491/20, A61K 31/485

(54) **QUINOLINOMORPHINANE DERIVATIVES AND MEDICINAL USE THEREOF**
CHINOLINMORPHINAN-DERIVATE UND IHRE MEDIZINISCHE ANWENDUNG
DERIVES DE QUINOLINOMORPHINANE ET LEUR USAGE MEDICAL

(30) Priority: 31.03.1997 JP 8175697
(43) Date of publication of application: 28.04.1999
(73) Proprietor: TORAY INDUSTRIES, INC., Tokyo 103-8666 (JP)
(72) Inventor: NAGASE, Hiroshi, Kamakura-shi, Kanagawa 247-0063 (JP); IMAMURA, Yoshifumi, Kamakura-shi, Kanagawa 248-0034 (JP); OHNO, Hiroshi, Kamakura-shi, Kanagawa 248-0034 (JP); KANEEDA, Masanobu, Kamakura-shi, Kanagawa 248-0031 (JP); MATSUDA, Susumu, Fujisawa-shi, Kanagawa 251-0038 (JP); MIYAUCHI, Yasushi, Kamakura-shi, Kanagawa 248-0034 (JP)
(74) Representative: Kador & Partner
(86) International application number: PCT/JP1998/001443
(87) International publication number: WO 1998/043977

(56) References cited:
- WO-A-89/00995
- WO-A-94/07896
- JP-A- 3 218 313
- JP-A- 3 223 288

## Description

### Technical Field

The present invention relates to quinolinomorphinan derivatives or pharmacologically acceptable acid addition salts thereof, and a medical composition comprising one of the derivatives or salts thereof.

### Background Art

In recent years, cases of diseases in the cerebral region such as various cerebrovascular diseases have increased with the arrival of the aging society. Cerebrovascular diseases are possibly caused by aging, hypertension, arterial sclerosis, hyperlipidemia, and the like, and are generally referred to as "cerebral stroke". In a broad sense, cerebral vascular diseases possibly include functional disorders of the brain due to head trauma.

Cerebral stroke is roughly classified into ischemic (infarcted) diseases and hemorrhagic diseases. Examples of the former include cerebral infarction (cerebral thrombosis, cerebral embolism), and the like, and examples of the latter include cerebral hemorrhage, subarachnoid hemorrhage, and the like. In these diseases, the blood flow is clogged due to a cerebrovascular disorder, and thus glucose and oxygen, which are energy sources of the action of the cerebral nerve cells, are insufficiently supplied, resulting in various damages of the nerve cells. These diseases are fundamentally caused by death of the cerebral nerve cells of a damage area and the periphery thereof. Such cerebrovascular diseases cause occurrence of various aftereffects such as cerebrovascular dementia, which are critical medical and social problems at present.

Medicines which have been developed as agents for curing such cerebrovascular diseases in Japan are mainly used for ameliorating aftereffects such as psychoneurosis and the like, and main medicines have the function to increase the amount of the blood flow to the brain to promote the supply of glucose and oxygen to an ischemic area. From the viewpoint of the functional mechanism, these medicines are expressed by vague terms such as medicines for ameliorating the cerebral blood flow, medicines for activating cerebral metabolism, and medicines for ameliorating cerebral function. However, almost all of these medicines are effective in ameliorating marginal symptoms such as volition disorders, affective disorders, behavioral abnormality, and the like, while the activity to the nucleus symptoms of dementia such as memory disorders and the like is considered as doubtful. Also some anti cerebral edema agents, antithrombotic agents, and thrombolytic agents are clinically used, particularly, in the acute stage of a cerebrovascular disease. These agents also have no direct action on the cerebral nerve cells, but are used only for symptomatic therapy. In any case, the above present medicines have substantially no effect on damage of the cerebral nerve cells in cerebrovascular diseases, and have no action to inhibit directly the death of the cerebral nerve cells.

As described above, there is now no medicine effective against damage of the cerebral nerve cells which are fundamental causes of cerebrovascular diseases. It is known that the degree of such damage has correlation to the ischemia time the cerebral blood flow is clogged, and a long ischemia time causes organic damage of the cerebral nerve cells which are not ameliorated even by recovery of the blood flow. For such cerebrovascular disorders, it is important to cure the disorders in the acute stage within 24 hours from the occurrence of the diseases. Therefore, there is now demand for developing, as early as possible, a safe medicine which has a secure protective effect on damages of the cerebral nerve cells and which is easy to use.

In addition to such cerebrovascular disorders, an increase in cerebral neurodegenerative diseases such as Alzheimer's disease is also a problem, and approach for elucidating causes and developing a therapeutic method is actively carried out in various fields. Although the main object of the approach is to activate, particularly, the acetylcholine nervous system, approach is also carried out by employing the neuroprotective action by a substance related to a nerve growth factor, a neurotrophic factor for the death of the nerve cells due to cerebral neurodegenerative diseases. Also the effect of a medicine having the cerebral neuroprotective action is expected.

The present invention relates to an agent for curing• preventing cerebral disorders, and an object of the present invention is to provide a medicine useful for ameliorating various cerebral diseases and aftereffects thereof, and preventing the recurrence thereof. Particularly, the present invention provides a medicine useful for curing• preventing cerebral stroke, traumatic cerebral diseases, cerebral edema, and cerebral neurodegenerative diseases by inhibiting various ischemic, hemorrhagic or traumatic cerebral disorders and damage of the cerebral nerve cells caused by various nerve degeneration, to protect the cerebral nerve cells.

### Disclosure of Invention

The present invention relates to quinolinomorphinan derivatives and pharmacologically acceptable acid addition salts thereof according to claim 1.

### Best Mode for Carrying Out the Invention

Preferred embodiments of the quinolinomorphinan derivative or a pharmacologically acceptable acid addition salt thereof represented by formula (II) of the present invention are as follows.

R¹ is preferably hydrogen, alkyl having 1 to 5 carbon atoms, cycloalkylmethyl having 4 to 7 carbon atoms, cycloalkenylmethyl having 5 to 7 carbon atoms, phenyl, naphthyl, phenylalkyl having 7 to 13 carbon atoms, alkenyl having 2 to 7 carbon atoms, alkanoyl having 1 to 5 carbon atoms, furan-2-ylalkyl having 1 to 5 carbon atoms (wherein the number of carbons indicates the number of the carbons of the alkyl moiety of furan-2-ylalkyl), or thiophene-2-ylalkyl having 1 to 5 carbon atoms (wherein the number of carbons indicates the number of the carbons of the alkyl moiety of thiophene-2-ylalkyl), and more preferably hydrogen, methyl, ethyl, propyl, butyl, cyclopropylmethyl, cyclobutylmethyl, cyclopentylmethyl, benzyl, phenethyl, allyl, 2-butenyl, 3-butenyl, 2-methyl-2-butenyl, 3-methyl-2-butenyl, 3-methyl-3-butenyl, acetyl, furan-2-ylmethyl, or thiophene-2-ylmethyl. Of these groups, hydrogen, methyl, cyclopropylmethyl, cyclobutylmethyl, benzyl, phenethyl, ally and acetyl are preferred.

R² and R³ are preferably hydrogen, hydroxy, methoxy, ethoxy, propoxy, acetoxy, benzyloxy, or benzoyloxy. Of these groups, R² is more preferably hydroxy, methoxy, or acetoxy, and R³ is more preferably hydrogen, hydroxy or methoxy.

When R⁵ does not form the fused ring structure A, R⁵ represents R¹⁸ which is preferably fluoro, chloro, bromo, iodo, nitro, hydroxy, methyl, ethyl, propyl, butyl, methoxy, ethoxy, isothiocyanato, trifluoromethyl, trifluoromethoxy, cyano, phenyl, hydroxymethyl, SR⁶, SOR⁶, SO₂R⁶, CO₂R⁷, CH₂CO₂R⁷, (CH₂)₂CO₂R⁷, SO₂NR⁷R⁸, CONR⁷R⁸, NR⁷R⁸, CH₂NR⁷R⁸, (CH₂)₂NR⁷R⁸, N(R⁷)COR⁸, CH₂N (R⁷) COR⁸, or (CH₂)₂N(R⁷)COR⁸, wherein R⁶ is preferably methyl or ethyl, R⁷ is preferably hydrogen or methyl, and R⁸ is preferably hydrogen, methyl, ethyl, propyl, butyl, cyclopropylmethyl, When two groups R⁵ form together a fused ring structure A, the residual 0 to 2 groups R⁵ are the above R¹⁸, or the residual two R⁵ form together another fused ring structure A. The fused ring structure A is preferably benzo, indeno, naphtho, pyrido or cyclohexeno, which is substituted by 0 or 2 groups R⁹, or unsubstituted dioxoleno. Particularly, benzo, indeno and cyclohexeno which are substituted by 0 to 1 R⁹, and unsubstituted dioxoleno are preferable. For example, when two R⁵ groups form together a fused ring structure A which is benzo, examples of compounds of formula (II) include compounds represented by the following formulae (IVa), (IVb), (IVc) and (IVd). However, the compounds of formula (II) are not limited to these compounds.

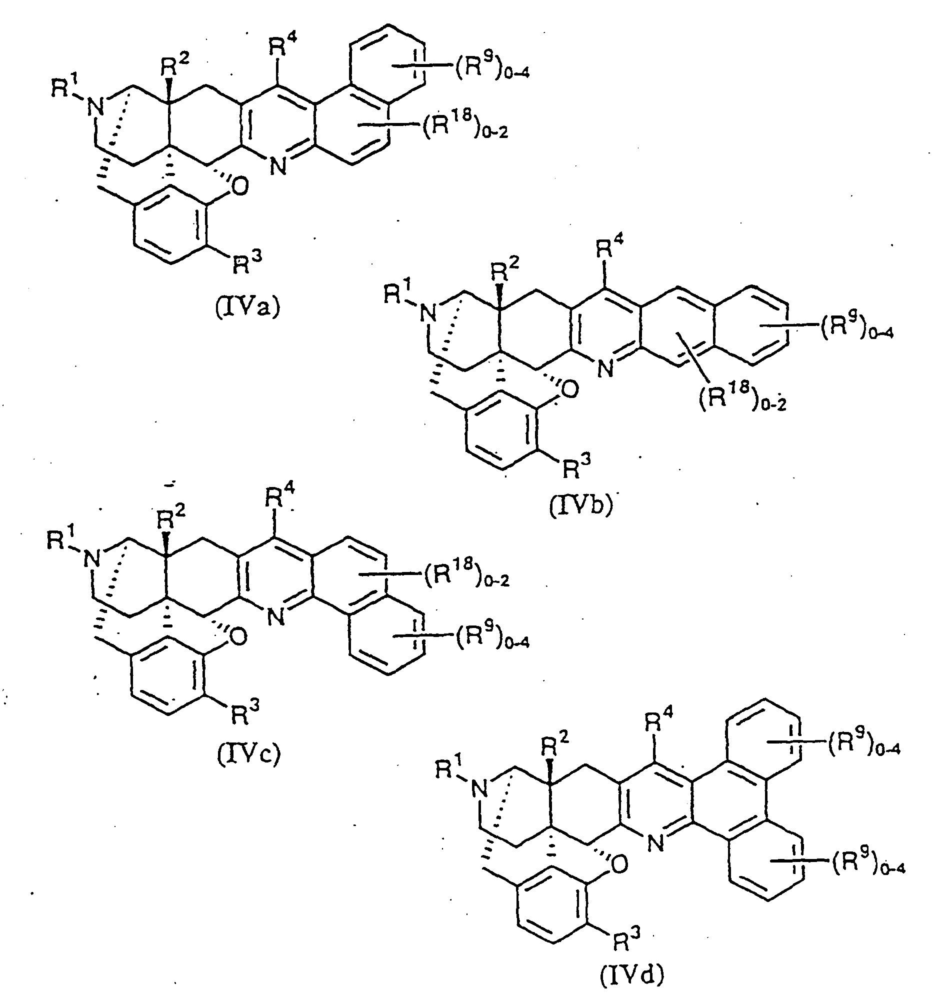

When R⁹ does not form a bridged structure R⁹-R¹⁸ together with R¹⁸, R⁹ is preferably fluoro, chloro, bromo, iodo, nitro, hydroxy, methyl, ethyl, propyl, butyl, methoxy, ethoxy, isothiocyanato, trifluoromethyl, trifluoromethoxy, cyano, phenyl, hydroxymethyl, SR⁶, SOR⁶, SO₂R⁶, CH₂CO₂R³, (CH₂)2CO₂R⁷, SO₂NR⁷R⁸, CONR⁷R⁸, NR⁷R⁸, CH₂NR⁷R⁸, (CH₂)₂NR⁷R⁸, N (R⁷) COR⁸, CH₂N(R⁷)COR⁸, or (CH₂)₂N(R⁷)COR⁸, wherein R⁶ is preferably methyl or ethyl, R⁷ is preferably hydrogen or methyl, and R⁸ is preferably hydrogen, methyl, ethyl, propyl, butyl, cyclopropylmethyl, or cyclopropylbutyl. R⁹ and R¹⁸ may form a bridged structure of R⁹-R¹⁸ together. In this case, ethano or o-benzeno is preferable as the bridged structure. For example, the bridged structure is ethano, and the fused ring structure A is benzo, examples of compounds of formula (I) include compounds represented by the following formulae (Va), (Vb), (Vc) and (Vd). The compounds of formula (I) are not limited to these compounds.

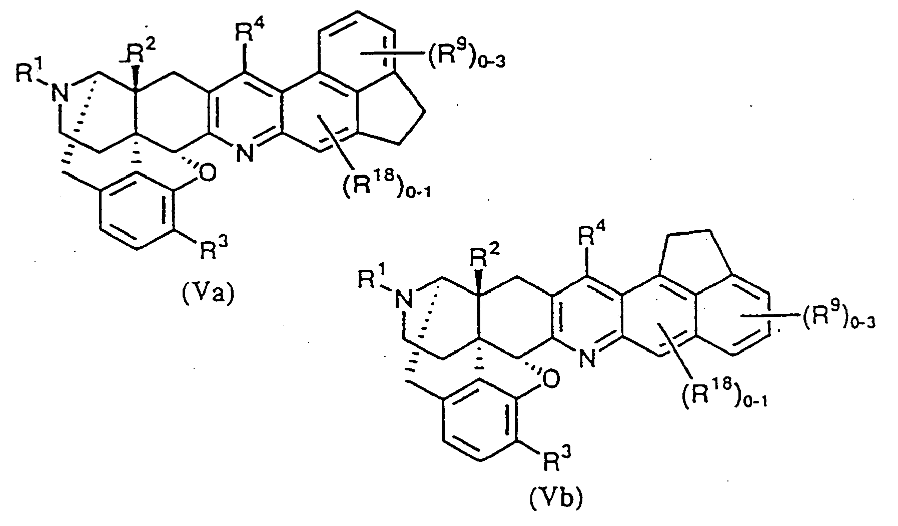

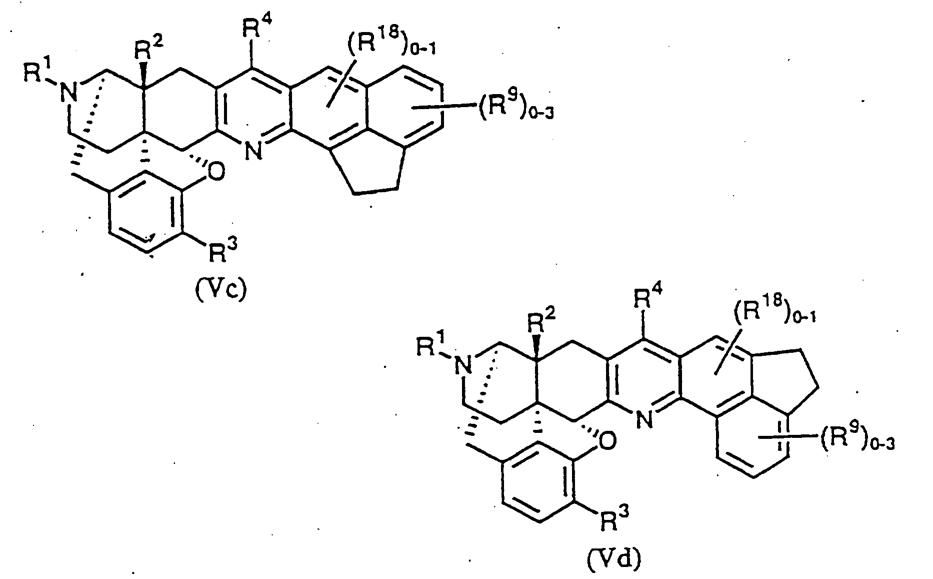

When R⁴ and R⁵ substituted at the peri position do not form together a bridged structure R⁴-R⁵, R⁴ is preferably hydrogen, methyl, ethyl, propyl, isopropyl, hydroxyalkyl having 1 to 3 carbon atoms, aryl having 6 to 12 carbon atoms (which may be substituted by at least one substituent R¹⁷), NR¹⁰R¹¹, OR¹², COOR¹³ or CONR¹⁴R¹⁵, wherein R¹⁰ is preferably hydrogen or methyl, R¹¹ is preferably hydrogen, alkyl having 1 to 5 carbon atoms, cycloalkylalkyl having 4 to 7 carbon atoms, benzyl, phenethyl or alkanoyl having 1 to 5 carbon atoms, R¹² is preferably hydrogen, methyl or acetyl, R¹³ is preferably hydrogen, methyl, ethyl, phenyl or benzyl, R¹⁴ is preferably hydrogen, methyl, ethyl, phenyl or benzyl, R¹⁵ is preferably hydrogen, methyl, ethyl, phenyl or benzyl, and R¹⁷ is preferably fluoro, chloro, bromo, amino, methyl or ethyl.

Of these groups, R⁴ is preferably hydrogen, methyl, ethyl, isopropyl, hydroxymethyl, hydroxyethyl, phenyl, naphthyl, amino, methylamino, dimethylamino, (cyclohexylmethyl)amino, benzylamino, phenethylamino, formylamino, acetylamino, propionylamino, hydroxy, methoxy, acetoxy, carboxy, methoxycarbonyl, ethoxycarbonyl, phenoxycarbonyl, benzyloxycarbonyl, carbamoyl, methylcarbamoyl, phenylcarbamoyl, benzylcarbamoyl or dimethylcarbamoyl; particularly hydrogen, methyl, ethyl, isopropyl, hydroxymethyl, phenyl, amino, methylamino, dimethylamino, (cyclohexylmethyl)amino, benzylamino, formylamino, acetylamino, hydroxy, methoxy, carboxy, methoxycarbonyl, carbamoyl, methylcarbamoyl or dimethylcarbamoyl. When R⁴ and R⁵ substituted at the peri position form together a bridged structure R⁴-R⁵, the bridged structure R⁴-R⁵ is preferably N(R¹⁶)CO, N(R¹⁶)C(=NH), N(R¹⁶)CH₂, o-benzeno, or propano; particularly N(R¹⁶)CO, N(R¹⁶)C(=NH), or N(R¹⁶)CH₂. In this case, R¹⁶ is preferably hydrogen, methyl, ethyl, benzyl, or acetyl. For example, R⁴ and R³ substituted at peri-position form together a bridge structure R⁴-R⁵ which is N(R¹⁶)CO, compounds of formula (II) are represented by the following formula (VI). However, compounds of formula (II) are not limited to these compounds.

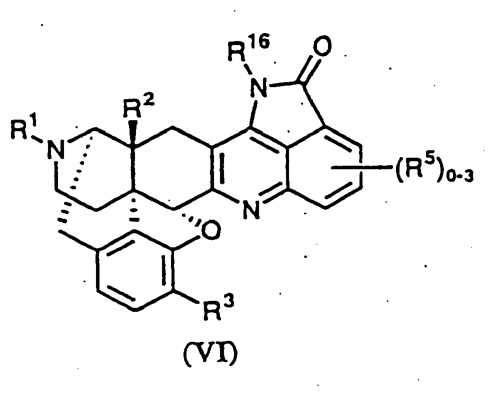

However, with hydrogen as R⁴, (1) when m is 1, R⁵ is hydroxy, and (2) when m is an integer of 2 to 4, R⁵ represents hydroxy, or two R⁵ groups necessarily form together a fused ring structure A, and the residual 0 or 2 R⁵ groups are independently R¹⁸ or must form another fused ring structure A. In this case, the fused ring structure A is preferably benzo, pyrido or cycloalkeno having 5 to 7 carbon atoms, which is substituted by 1 or 2 substituents R⁹, or indeno or naphtho unsubstituted by R⁹, or unsubstituted dioxoleno. Particularly, when the fused ring structure A is benzo, pyrido or cycloalkeno having 5 to 7 carbon atoms, which is substituted by 1 or 2 substituents R⁹, at least one sustituent R¹⁸ is hydroxy, or at least one R⁹ and one R¹⁸ substituted at adjacent carbons with a ring junction therebetween necessarily form a bridged structure R⁹-R¹⁸ together which is any one of ethano, propano, and o-benzeno structures. In this case, as the bridged structure R⁹-R¹⁸, ethano and o-benzeno are preferable.

Preferable examples of pharmacologically acceptable acid addition salts include inorganic salts such as a hydrochloride, a sulfate, a nitrate, a hydrobromide, a hydroiodide, a phosphate, and the like: organic carboxylates such as an acetate, a lactate, a citrate, an oxalate, a glutarate, a malate, a tartrate, a fumarate, a mandelate, a maleate, a benzoate, a phthalate, and the like; organic sulfonates such as a methanesulfonate, an ethanesulfonate, a benzenesulfonate, a p-toluenesulfonate, a camphorsulfonate, and the like. Particularly, a hydrochloride, a phosphate, a tartrate, a methanesulfonate, and the like are preferable, but, of course, the salts are not limited to these salts.

Of the compounds of formula (II) of the present invention, compound 1 is designated 17-cyclopropylmethyl-**6,7-dehydro-4,5α-epoxy-3,14β-dihydroxy-6,7,2',3'-**quinolinomorphinan, in which R¹ is cyclopropylmethyl, R² and R³ are each hydroxy, R⁴ is hydrogen, and m is 0.

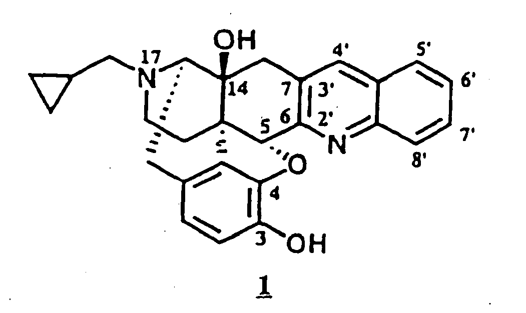

Of the compounds of formula (II) of the present invention, compound 36 is designated 17-cyclopropylmethyl-6.7-dehydro-4,5a.-epoxy-3.14p-dihydroxy-6,7.2',3'-(7',8'-benzoquinolino)morphinan, in which R¹ is cyclopropylmethyl, R² and R³ are each hydroxy, R⁴ is hydrogen, m is 2, and two R⁵ groups are substituents at the 7' and 8'-positions of the quinoline ring and form together the fused ring structure A which is benzo.

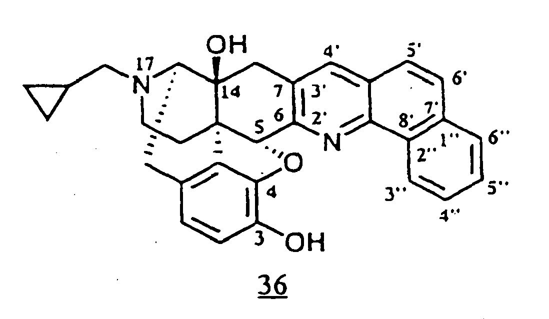

Of the compounds of formula (I) of the present invention, compound 44 is designated 17-cyclopropylmethyl-6,7-dehydro-4,5α-epoxy-3,14β-dihydroxy-6,7,2',3'-(6',6"-ethano-7',8'-benzoquinolino)morphinan, in which R¹ is cyclopropylmethyl, R² and R³ are each hydroxy, R⁴ is hydrogen, m is 3, two R⁵ groups are substituents at the 7' and 8'-positions of the quinoline ring and form together the fused ring structure A which is benzo substituted by one R⁹. and the residual one R⁵ is R¹⁸ substituted at the 6'-position of the quinoline ring and forming ethano as a bridged structure R⁹-R¹⁸ together with R⁹ substituted at the 6" -position of the benzene ring adjacent to R¹⁸ with the ring junction therebetween.

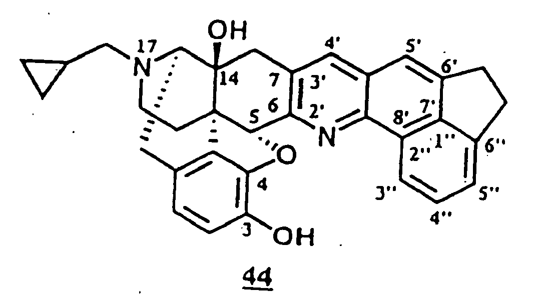

Of the compounds of formula (II) of the present invention, compound 39 is designated 17-cyclopropylmethyl-6,7-dehydro-4,5α-epoxy-3,14β-dihydroxy-67,2'3'-(7',8'-cyclohexenoquinolino)morphinan, in which R¹ is cyclopropylmethyl, R² and R³ are each hydroxy, R⁴ is hydrogen, m is 2, and two R⁵ groups are substituents at the 7' and 8'-positions of the quinoline ring and form together the fused **ring structure A which is cyclohexeno.**

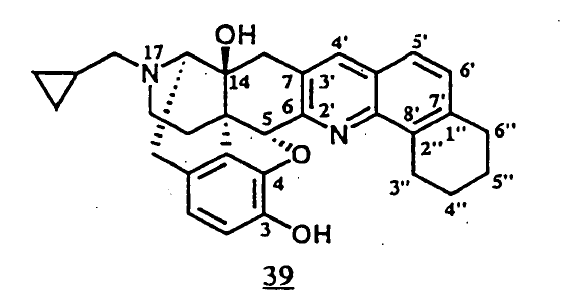

Of the compounds of formula (II) of the present invention, compound 29 is designated 17-cyclopropylmethyl-6,7-dehydro-4,5α-epoxy-3,14β-dihydroxy-6,7,2',3'-(4'-methyl-6',7'-dioxolenoquinolino)morphinan, in which R¹ is cyclopropylmethyl, R² and R³ are each hydroxy, R⁴ is methyl, m is 2, and two R⁵ groups are substituents at the 6' and 7'-positions of the quinoline ring and form together the fused ring structure A which is dioxoleno.

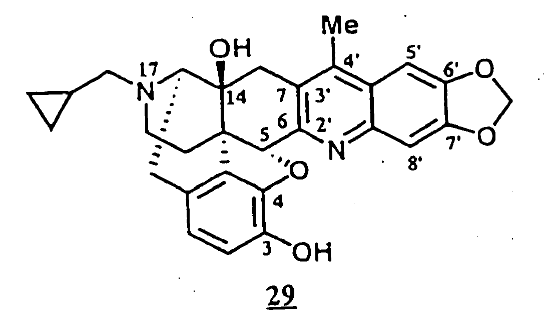

Of the compounds of formula (II) of the present invention, compound 34 is designated 17-cyclopropylmethyl-6,7-dehydro-4,5α-epoxy-3,14β-dihydroxy-6,7,2',3'-(4' ,5'-[imino(oxomethano)]quinolino]morphinan, in which R¹ is cyclopropylmethyl, R² and R³ are each hydroxy, and R⁴ forms a bridged structure R⁴-R⁵ together with R⁹ substituted at the 5'-position as the peri position of the quinoline ring which is N(R¹⁶)CO wherein R¹⁶ is hydrogen.

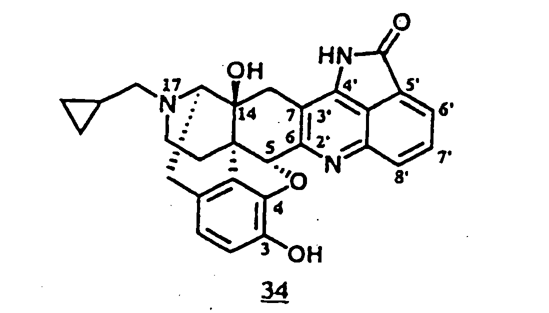

The quinolino morphinan derivatives represented by formula (II) include the following compounds listed in the table below. The compounds on page 21 for which R₄ = H are not part of the claimed invention.

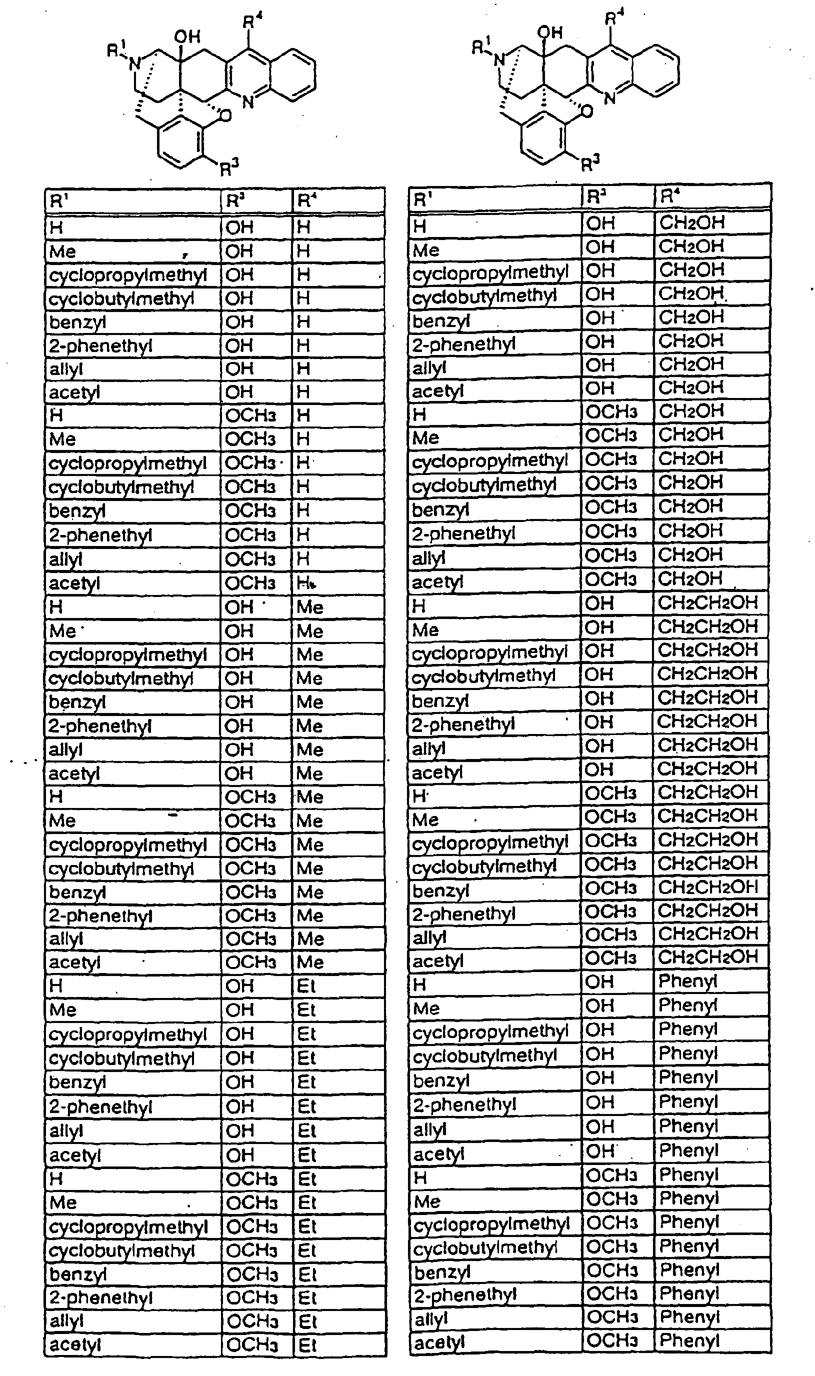

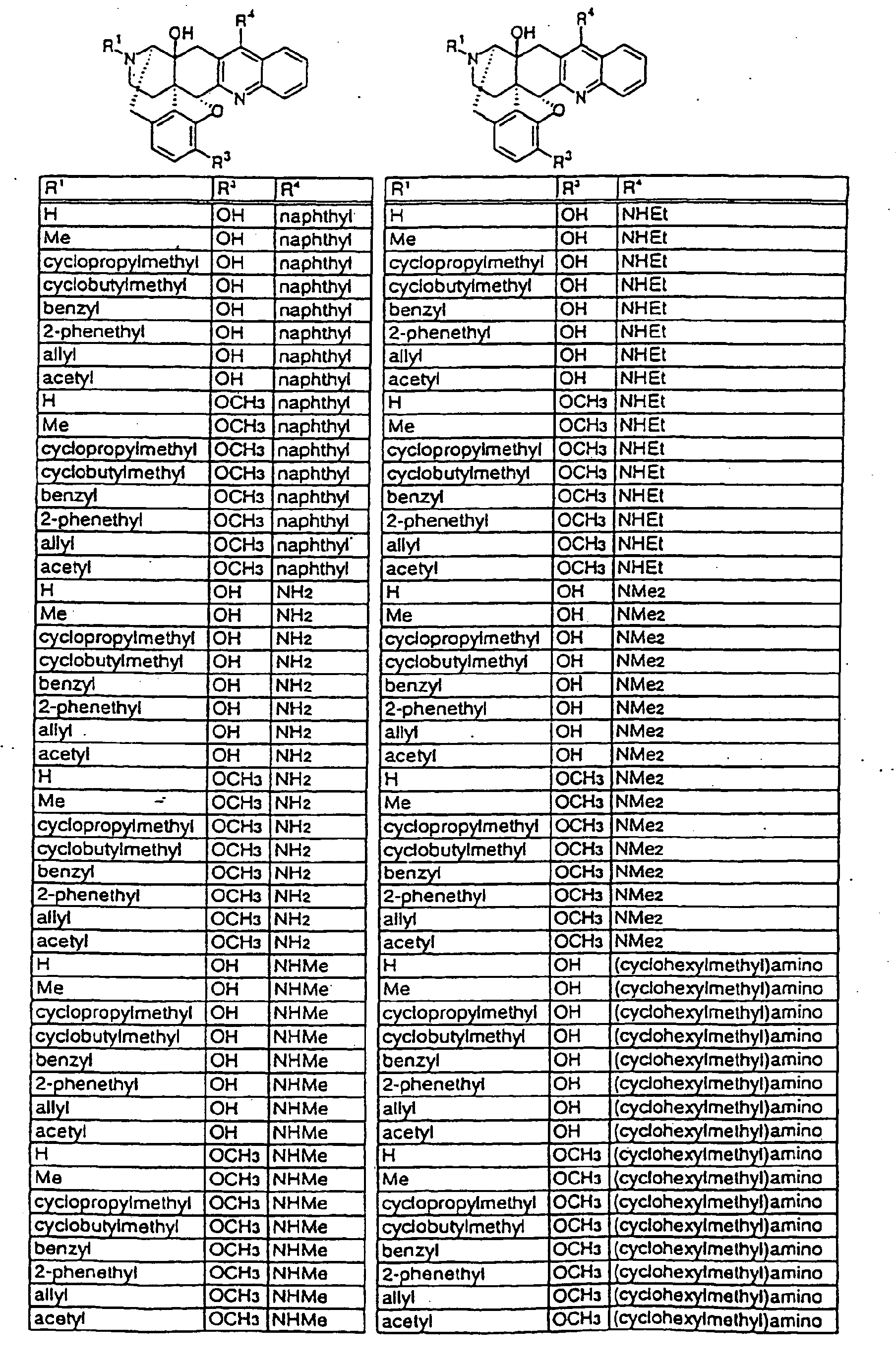

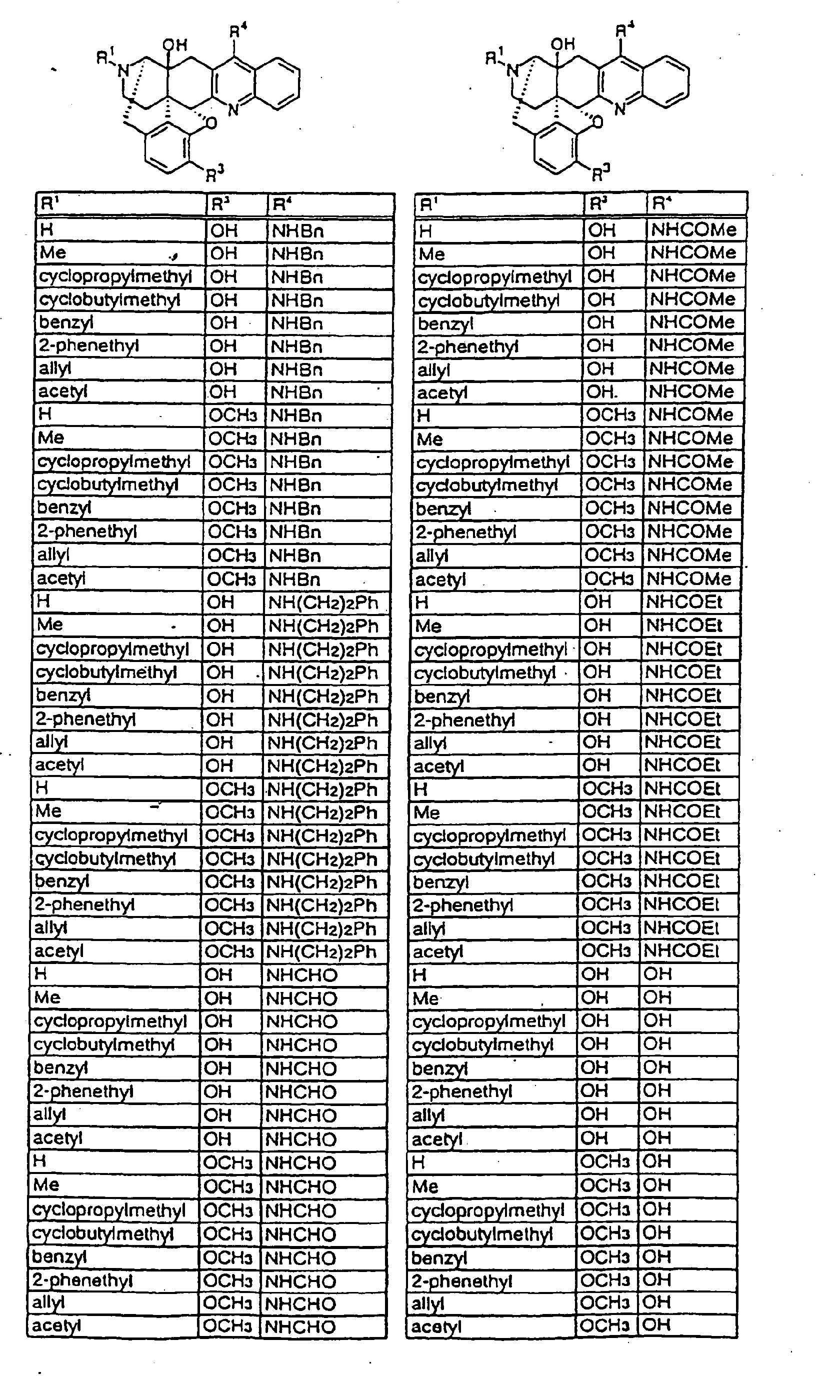

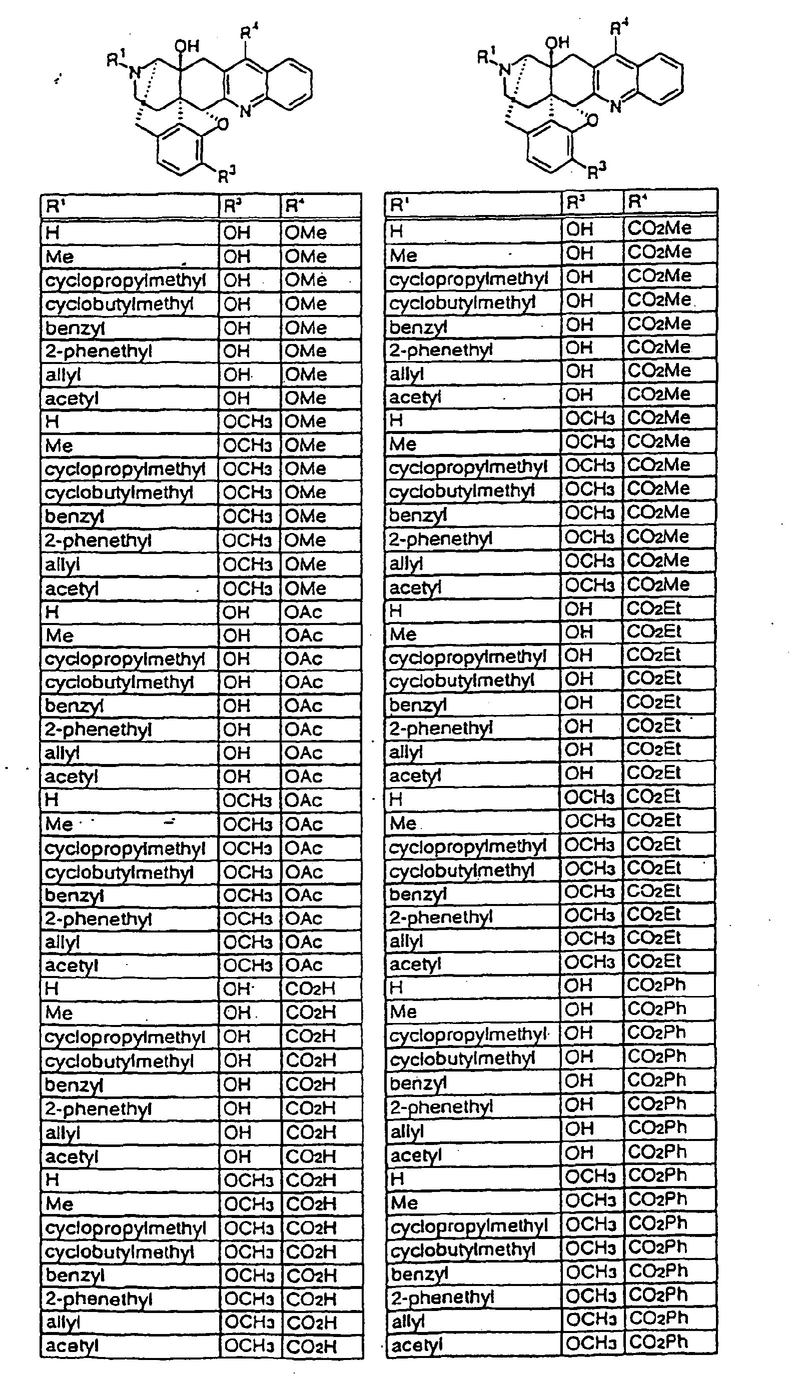

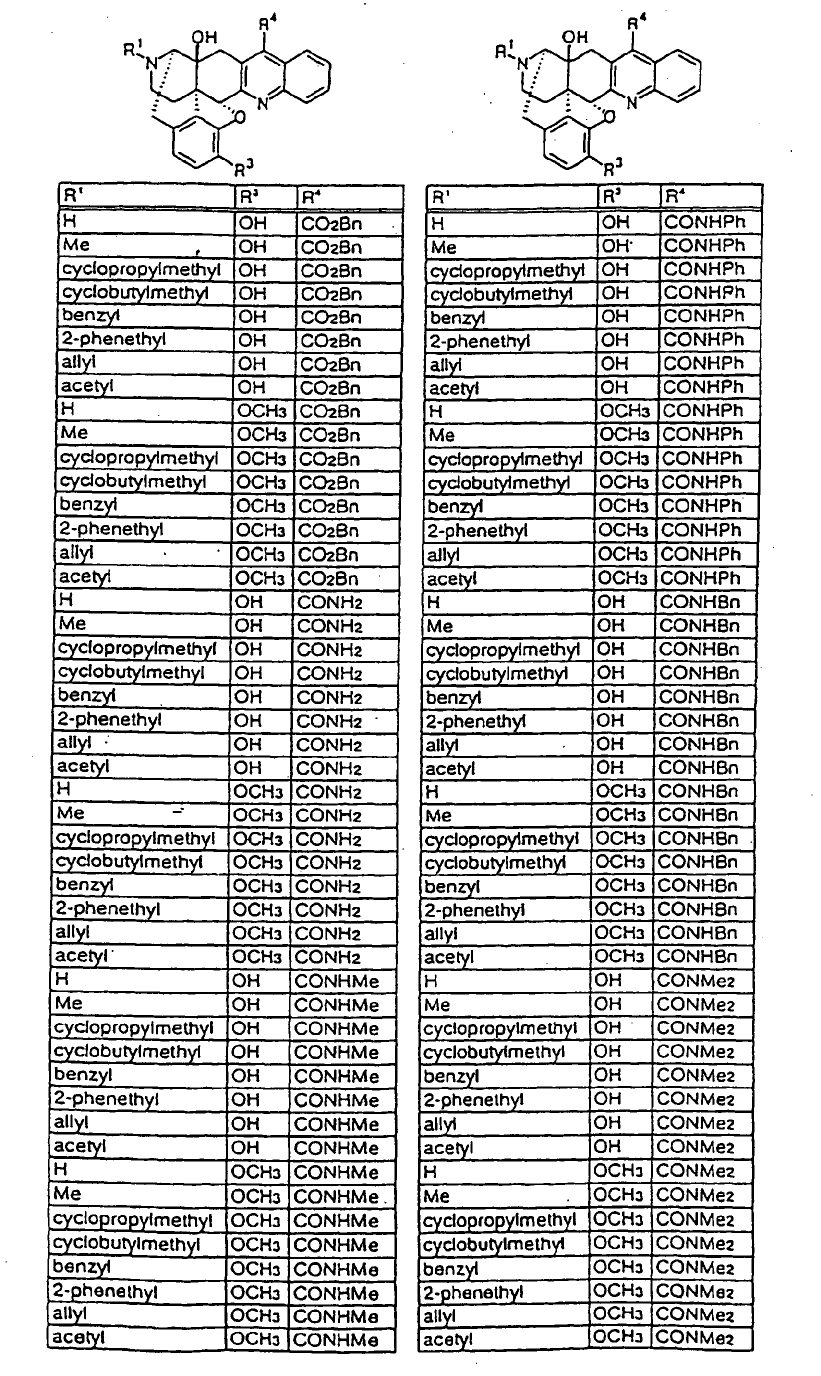

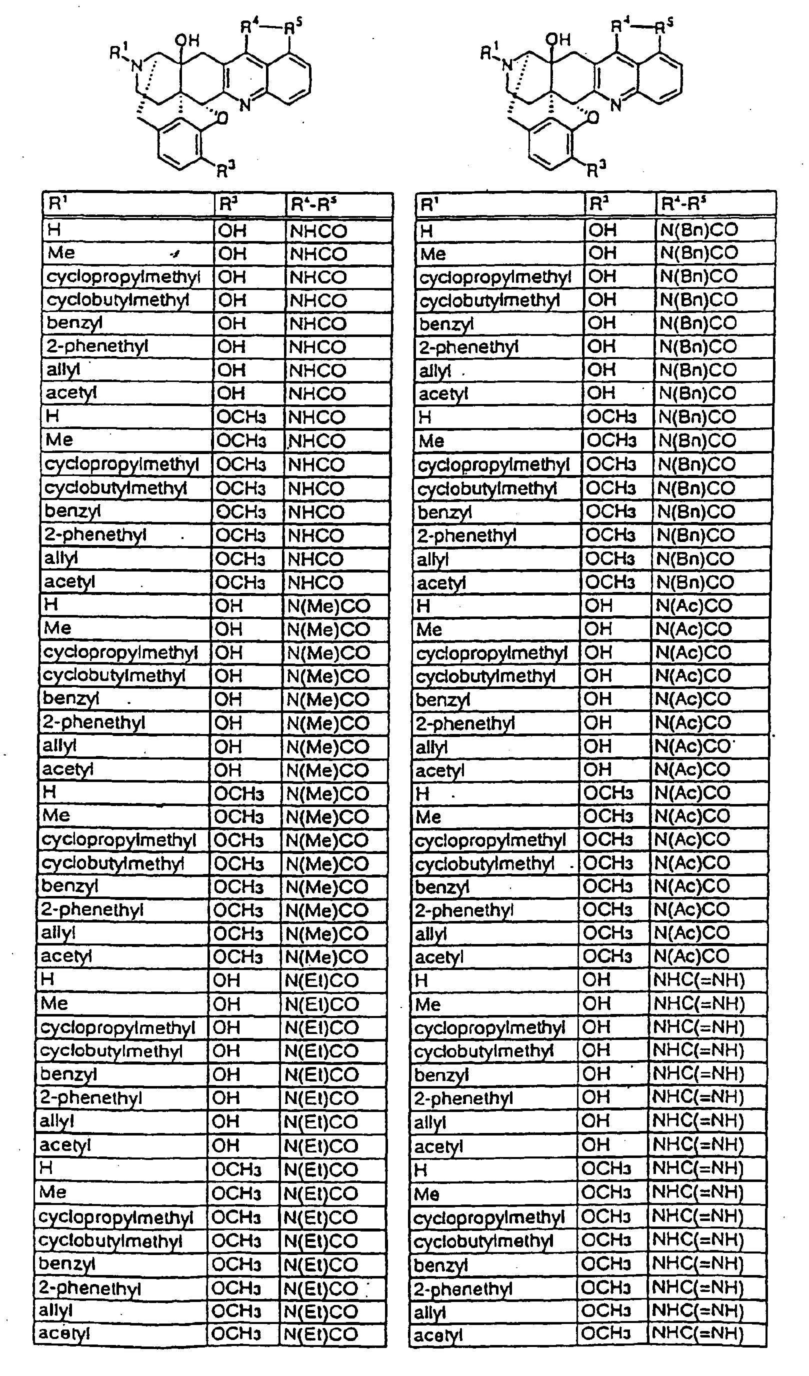

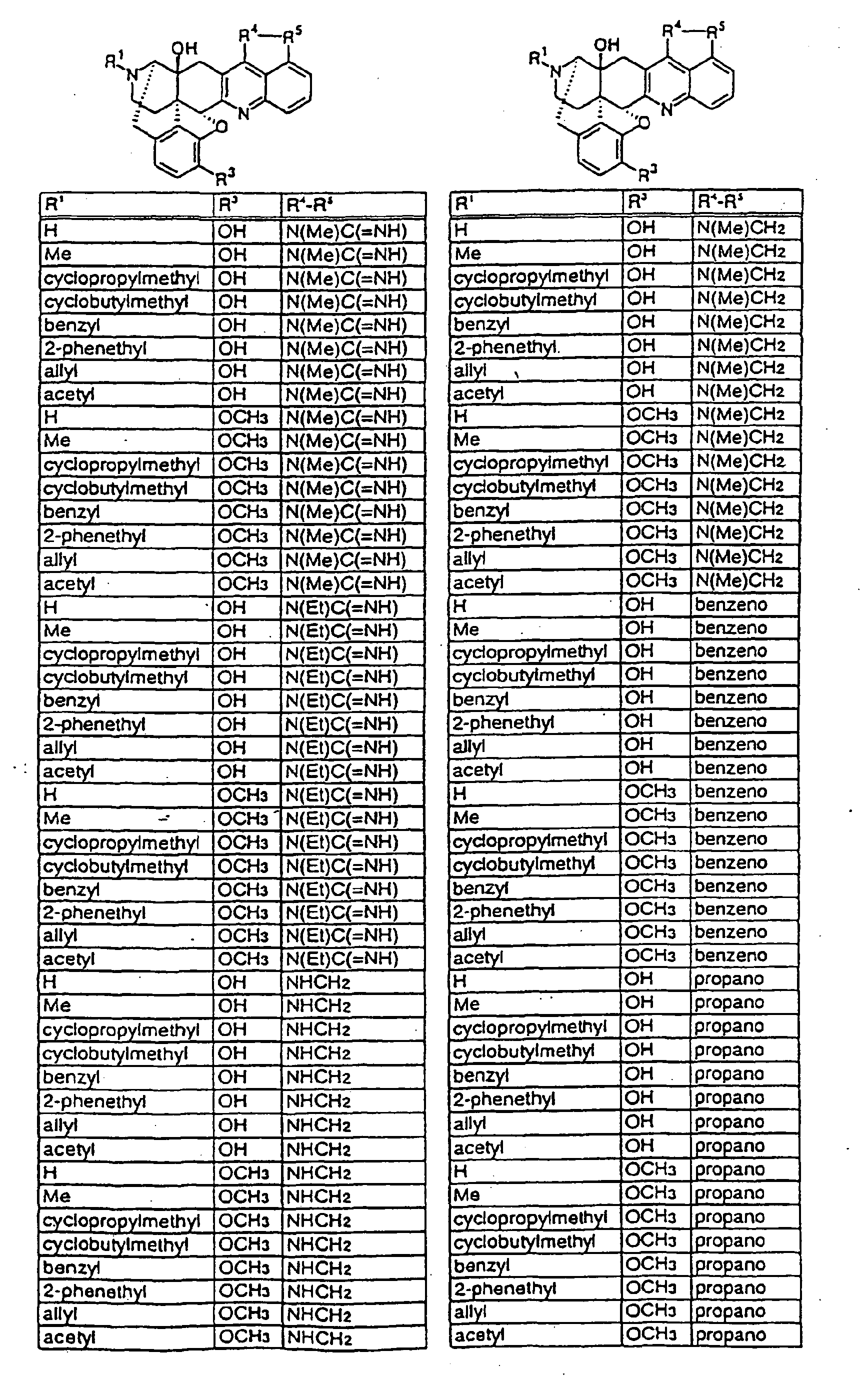

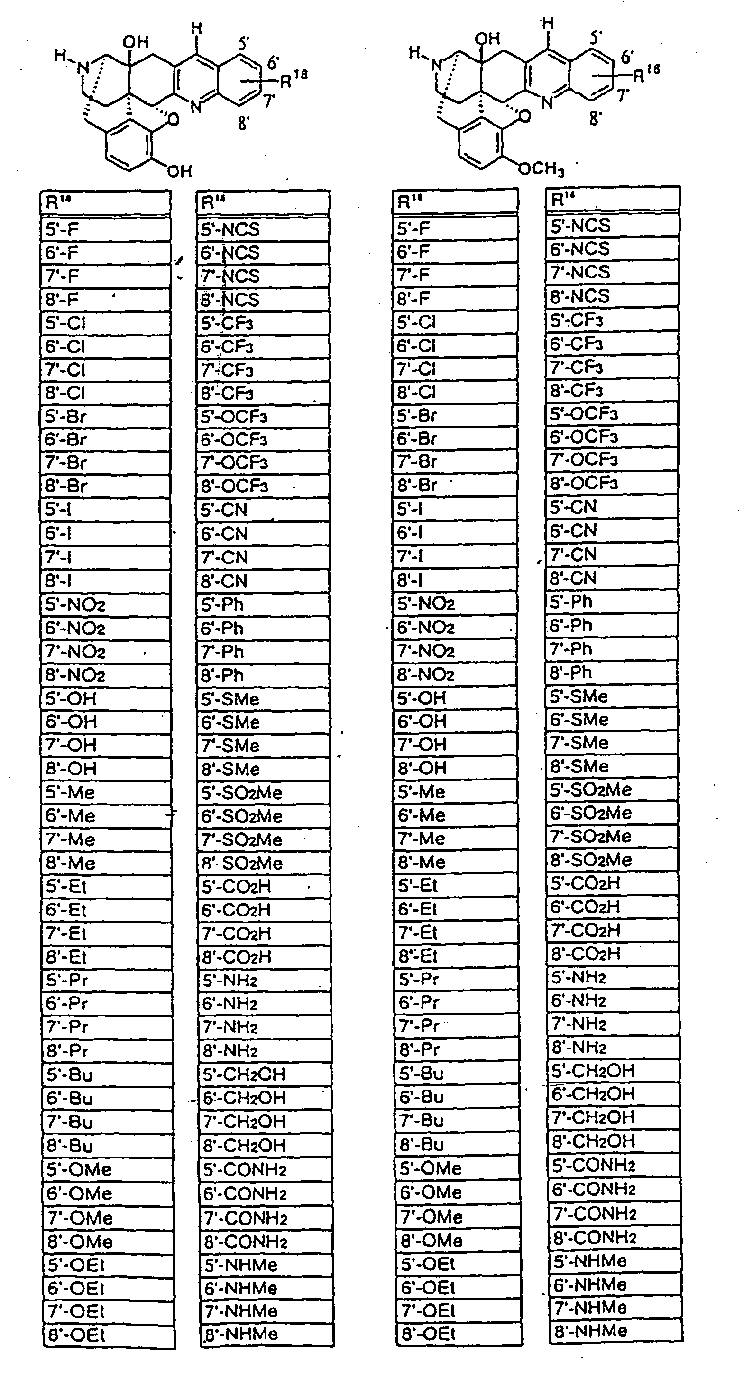

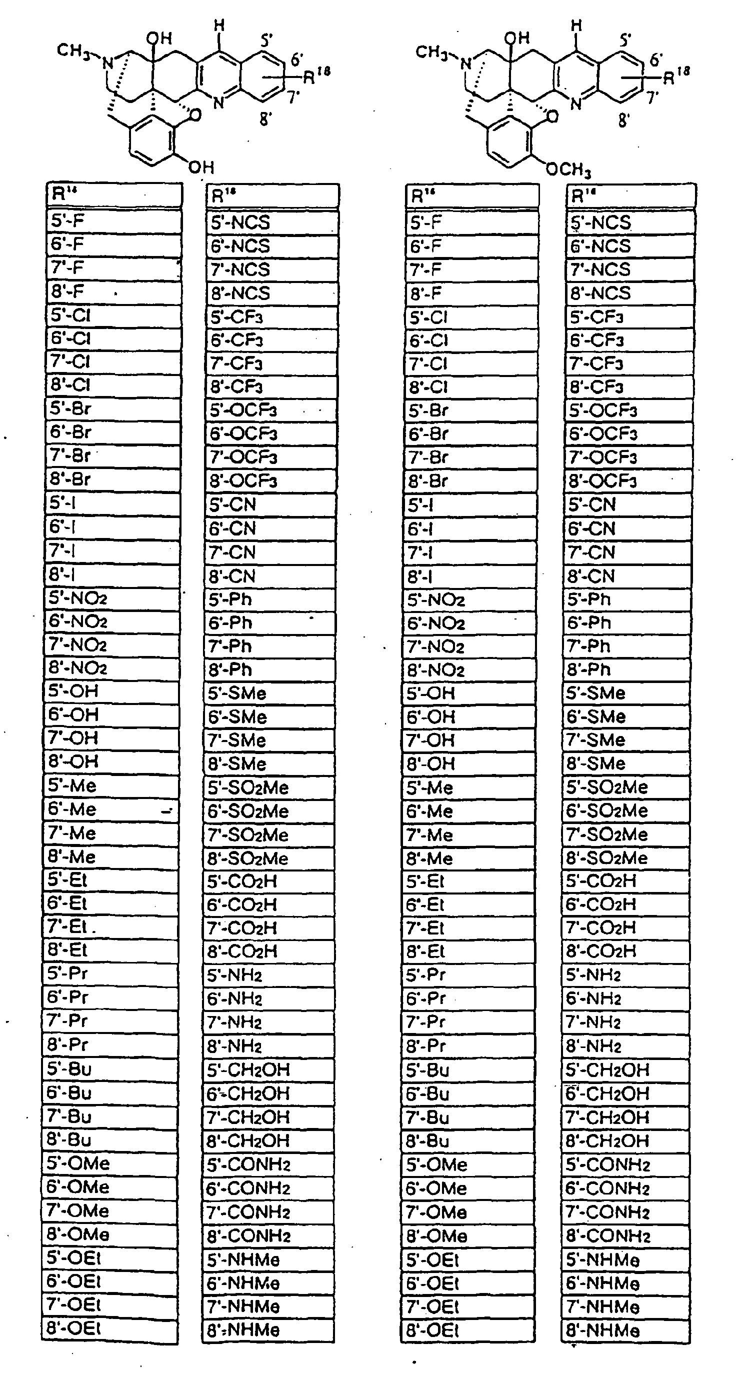

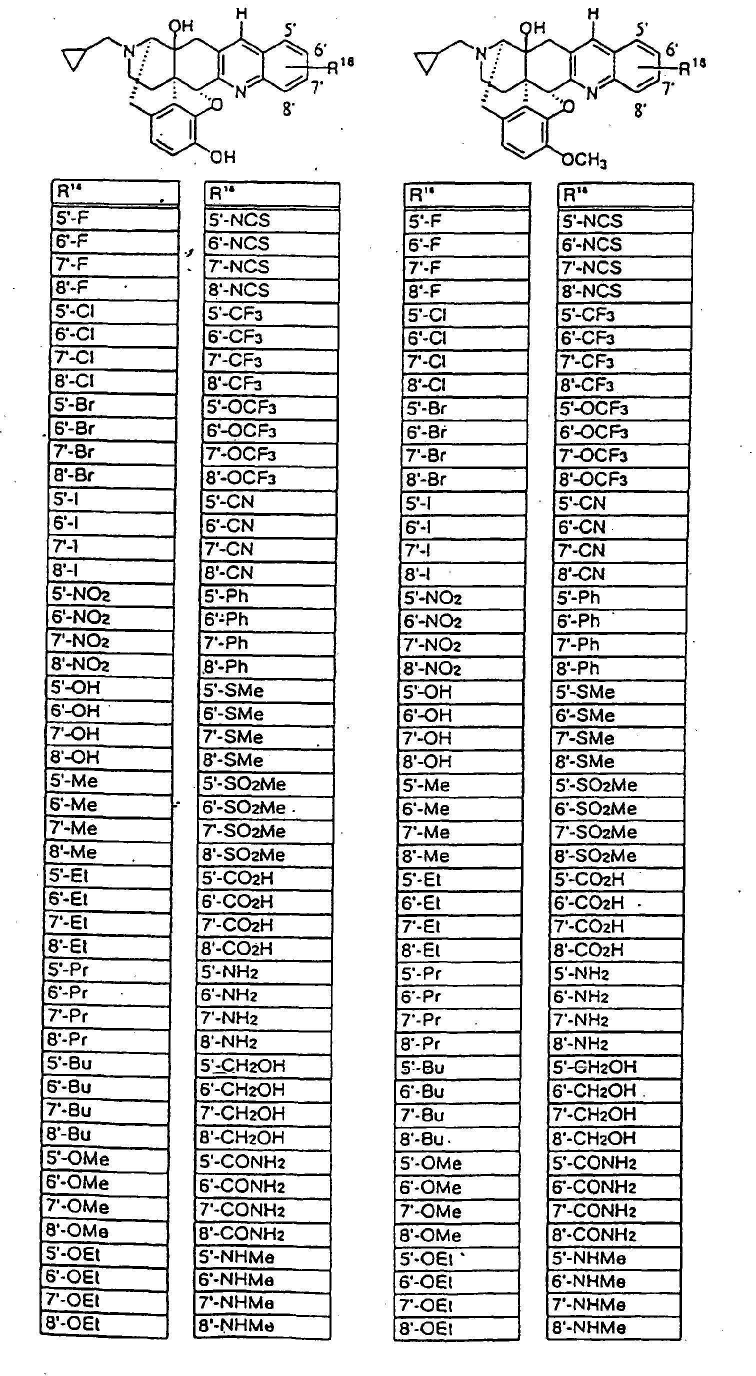

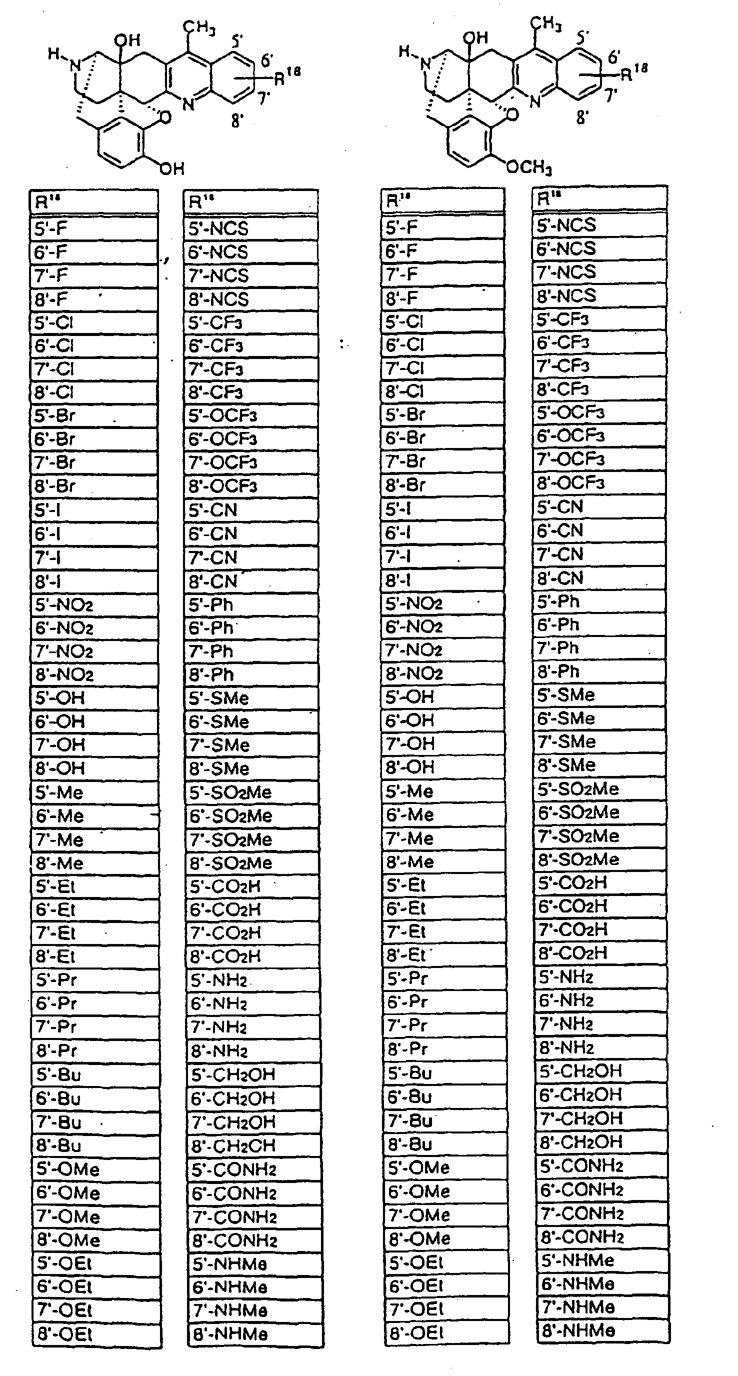

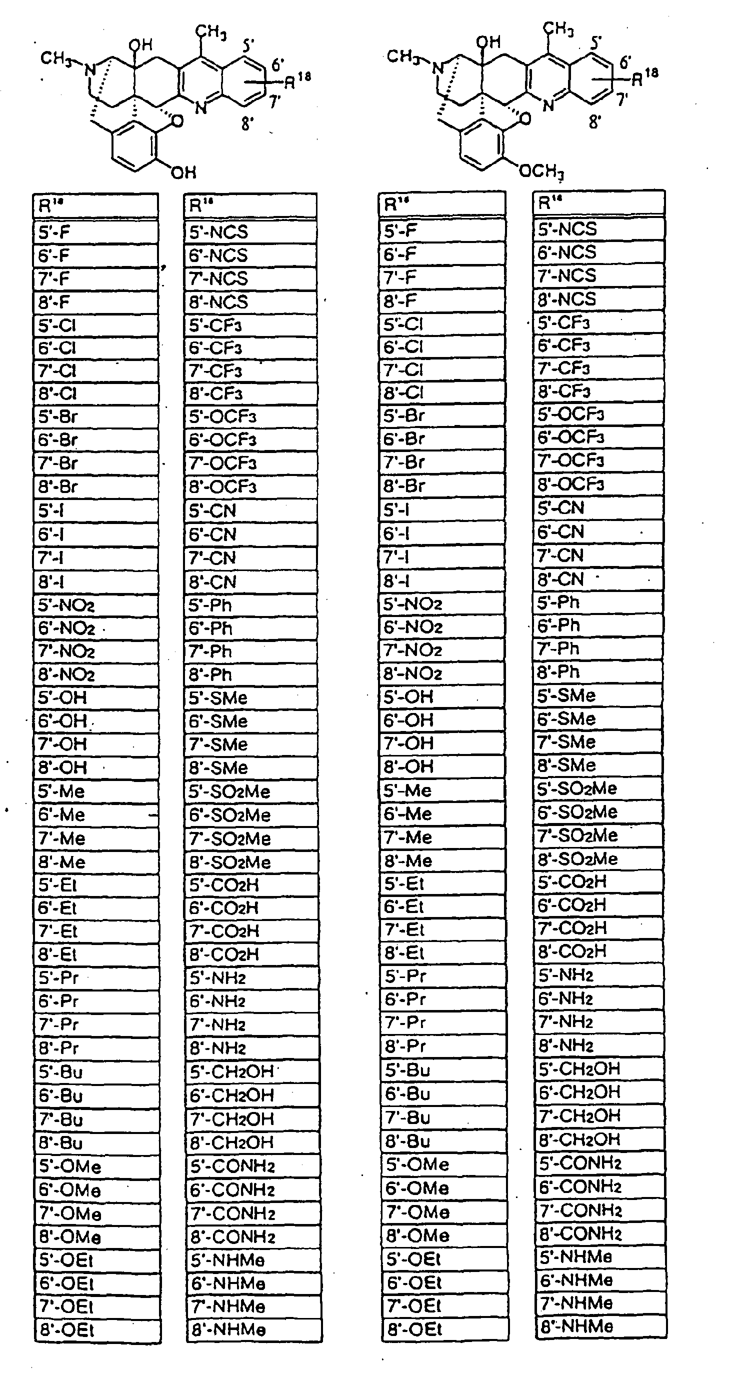

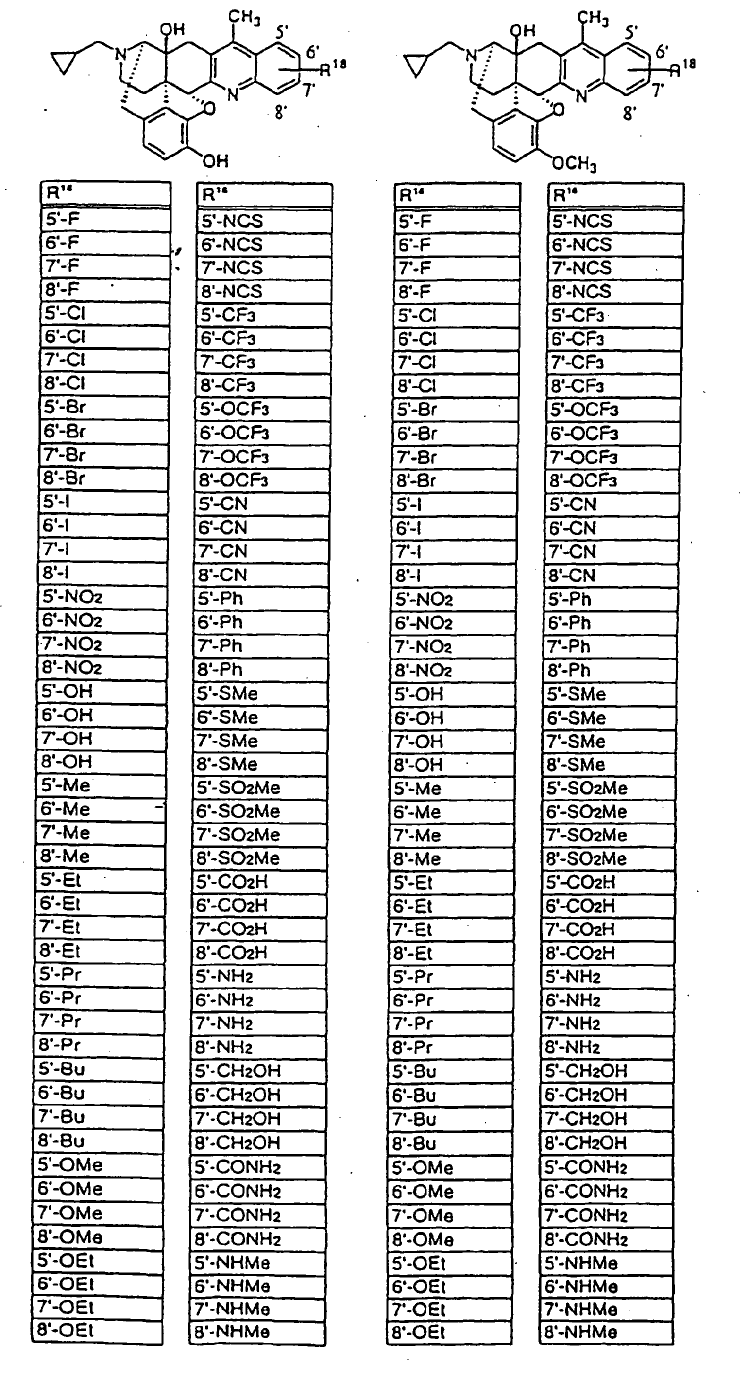

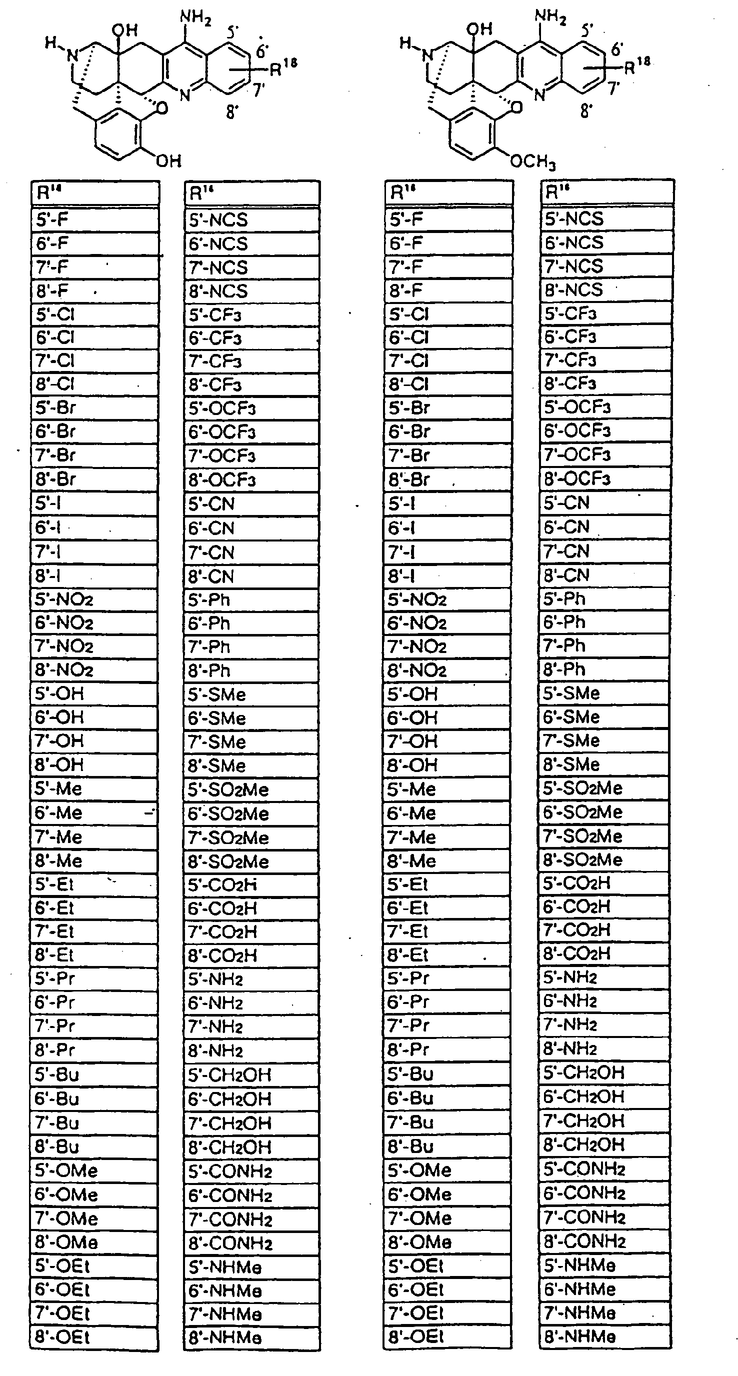

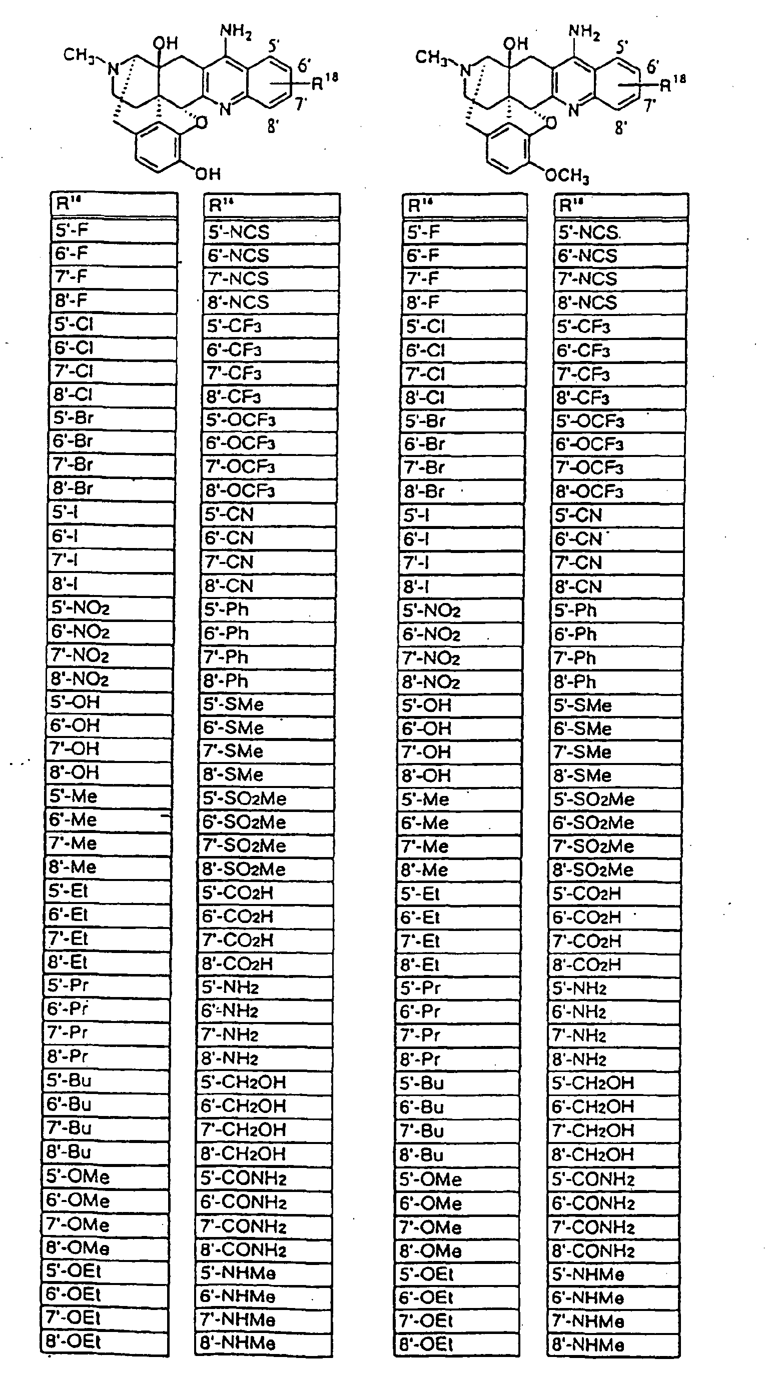

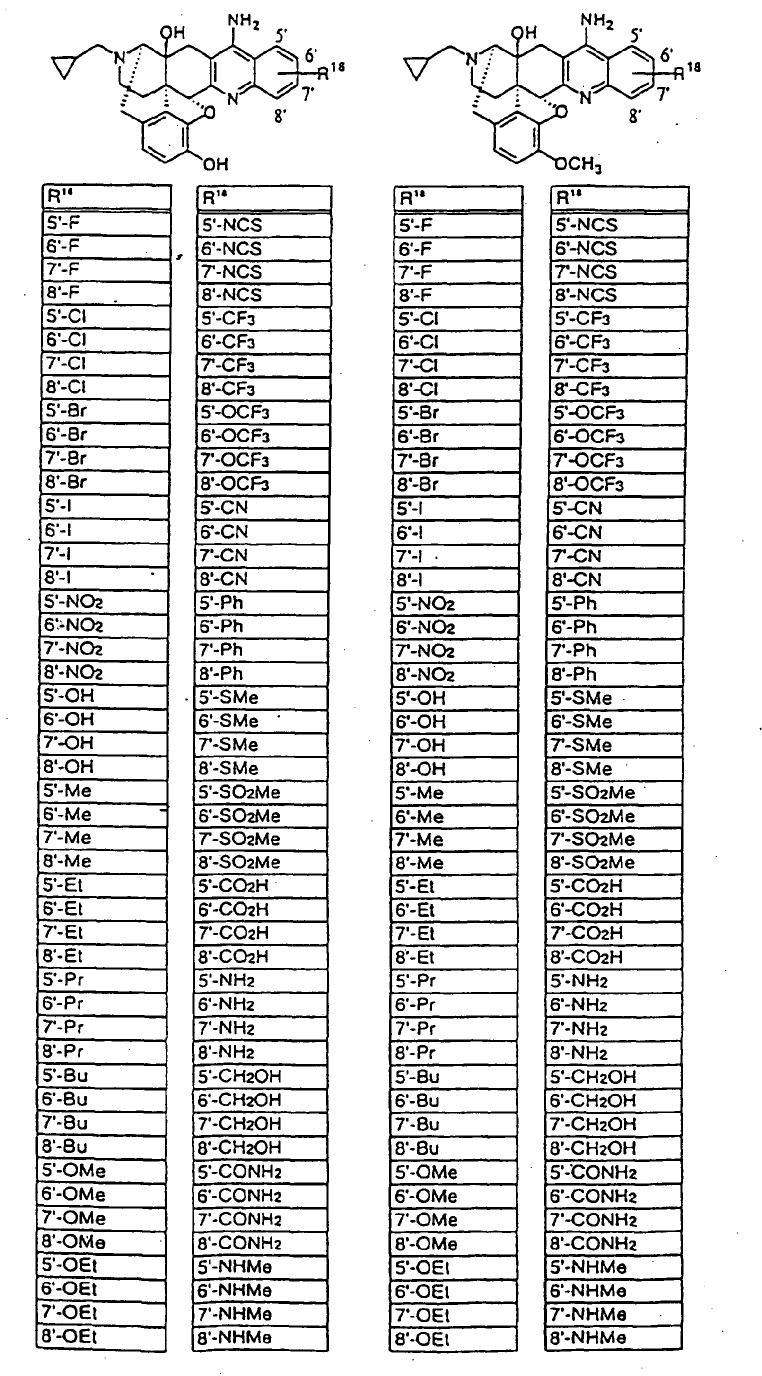

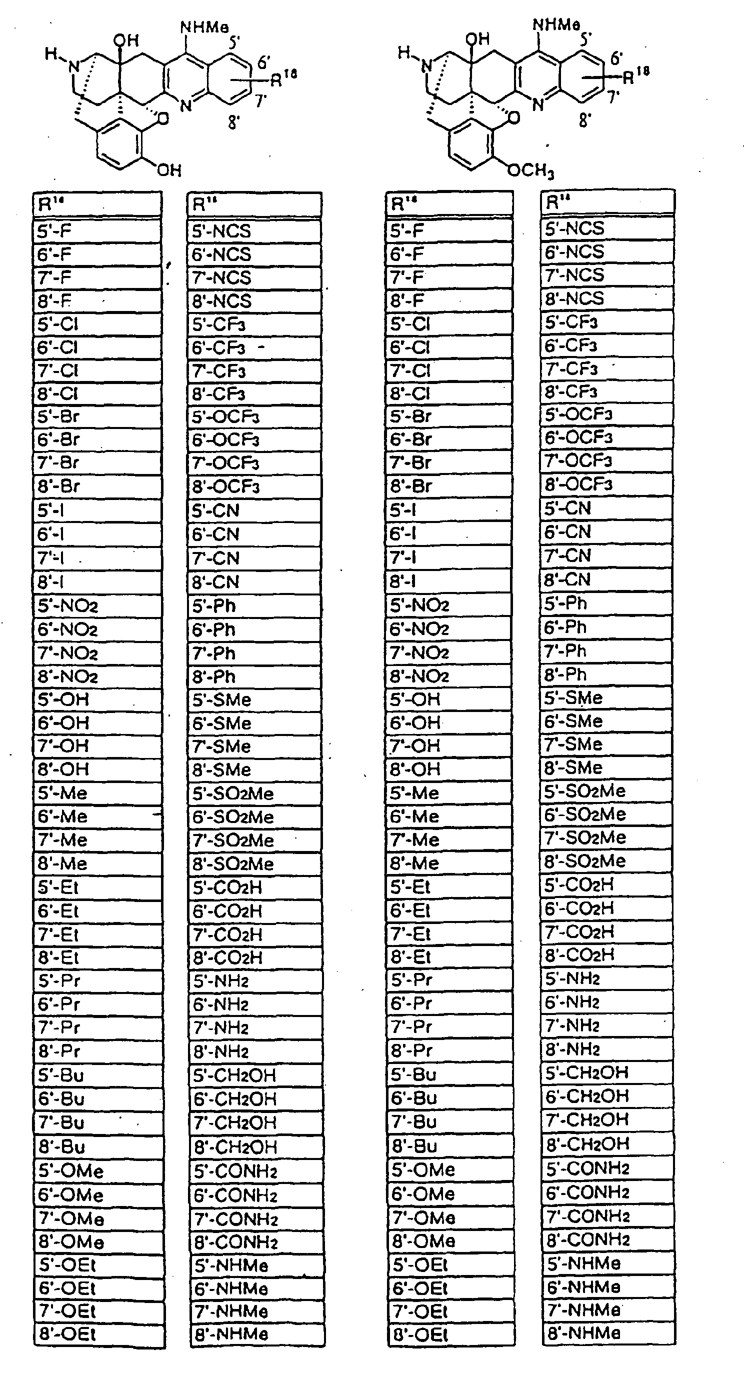

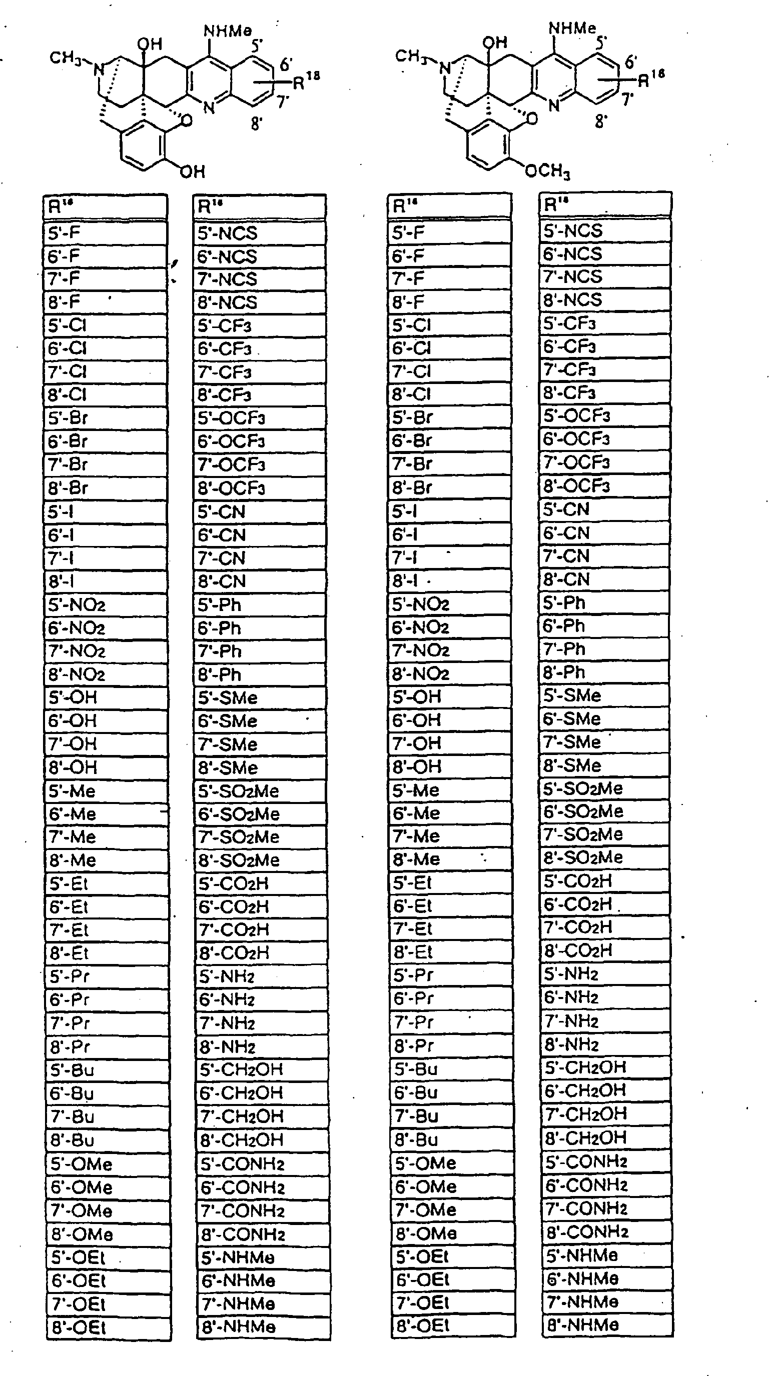

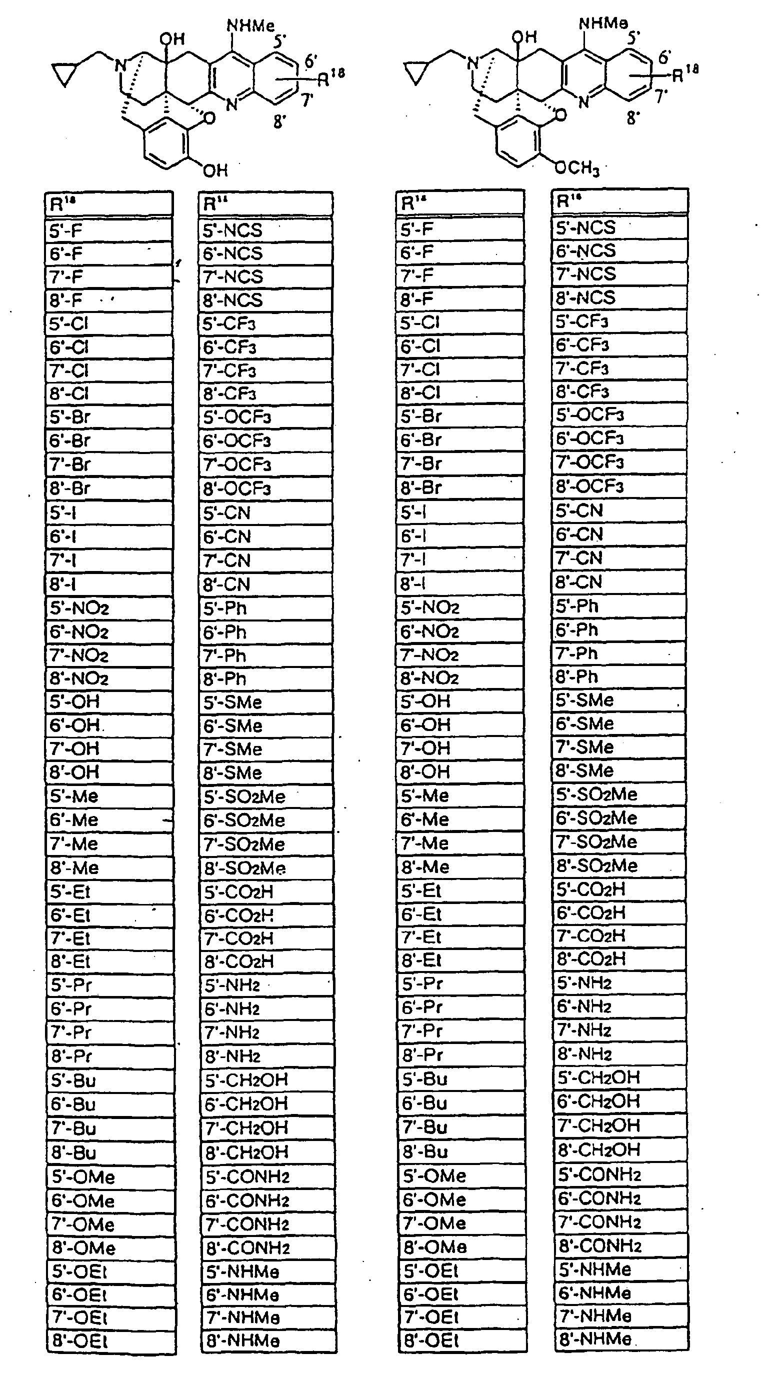

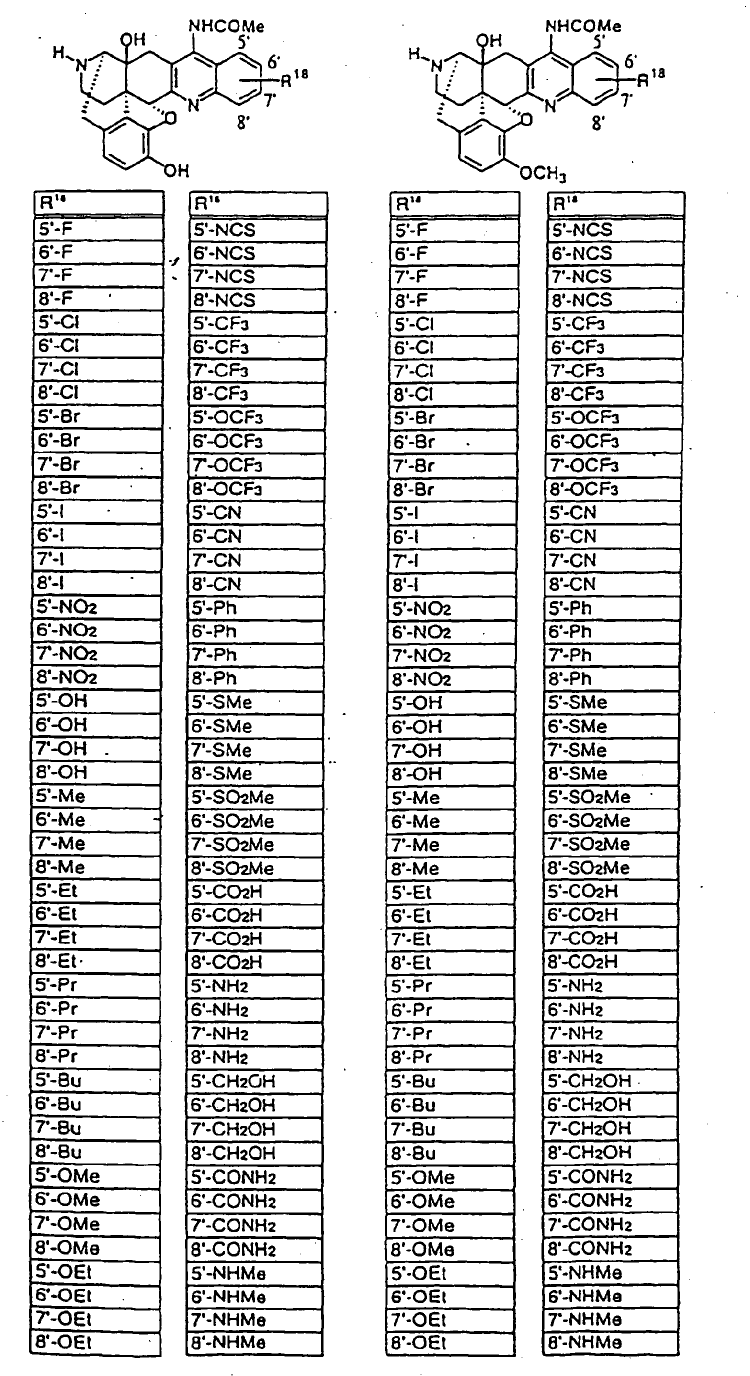

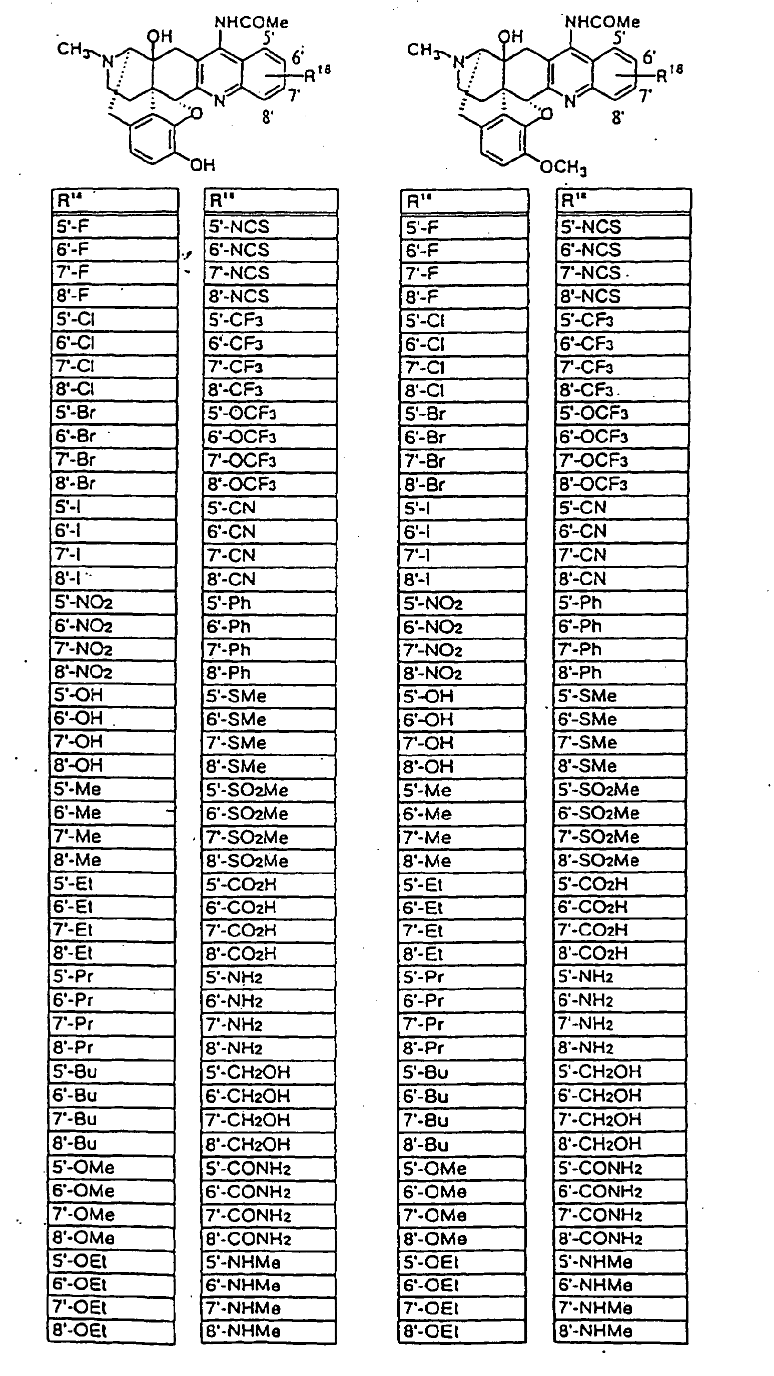

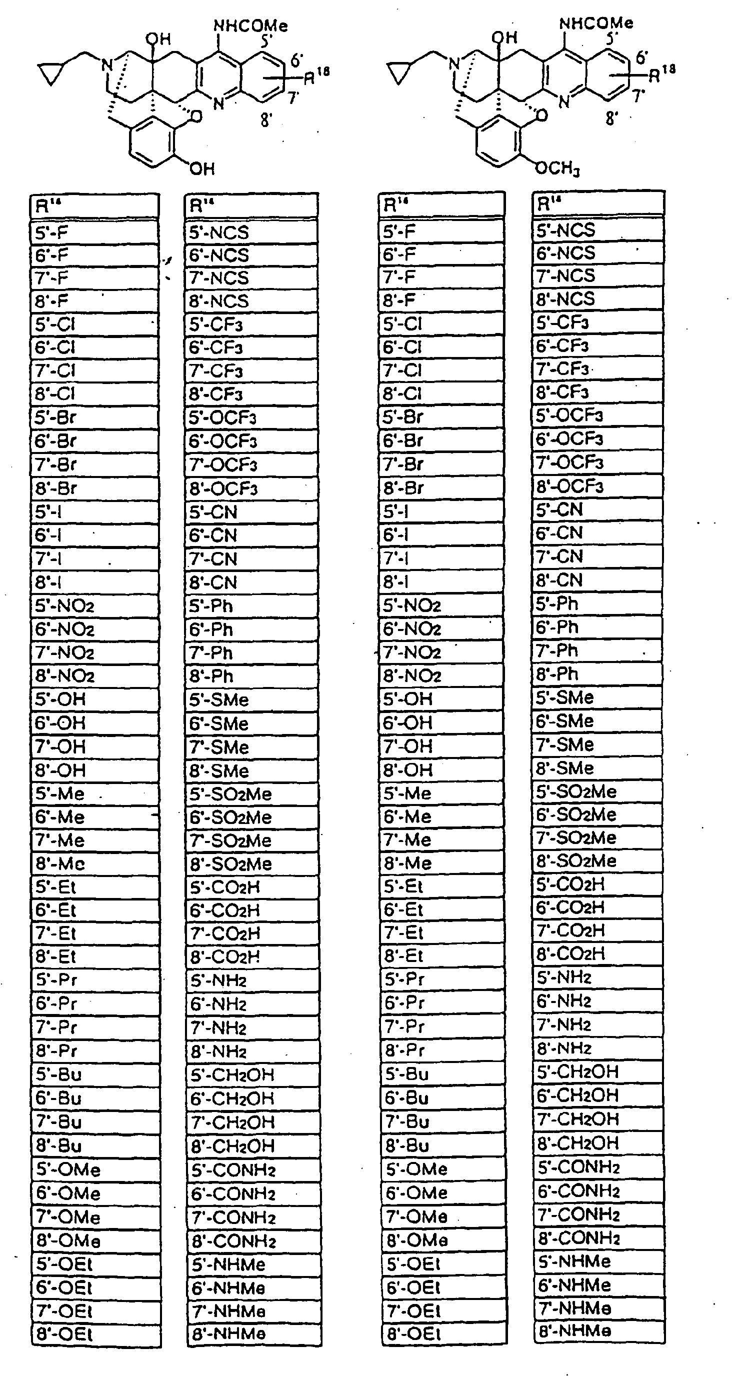

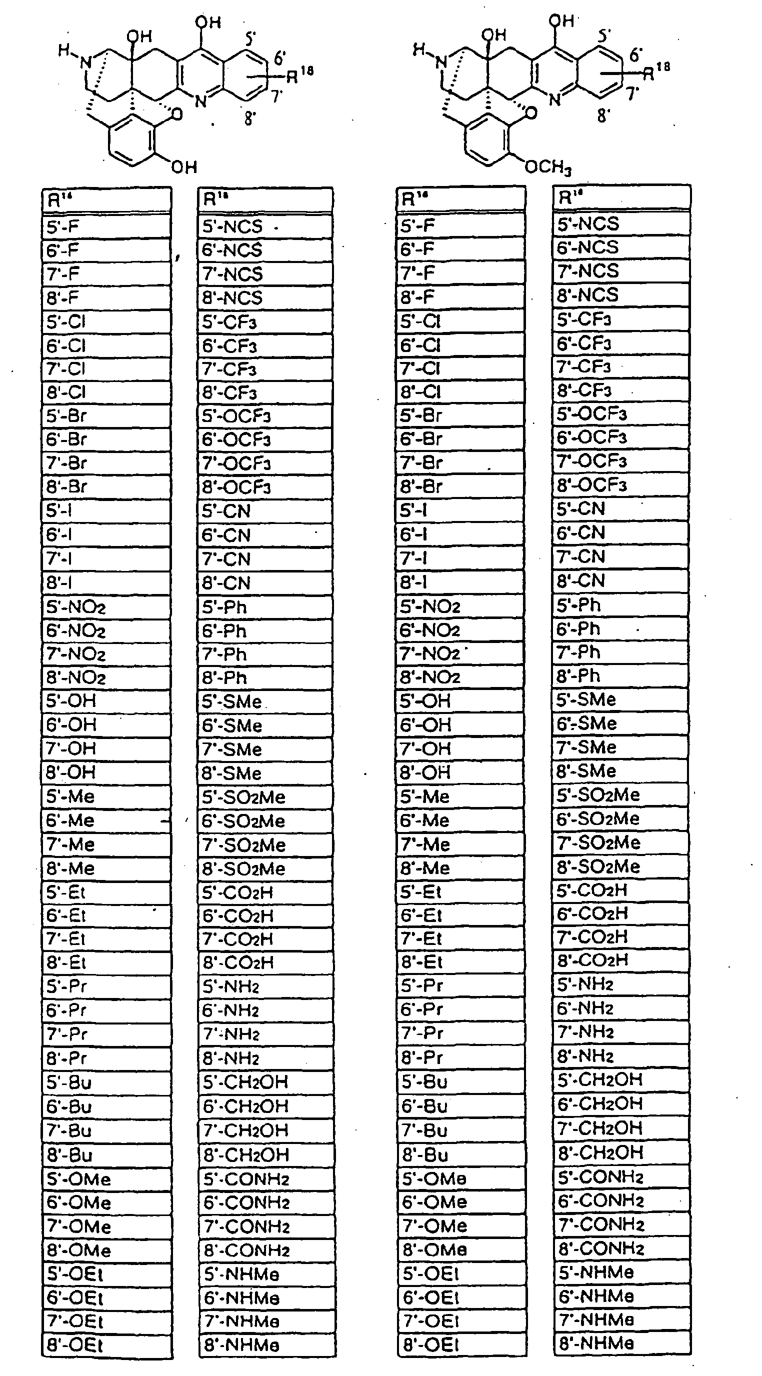

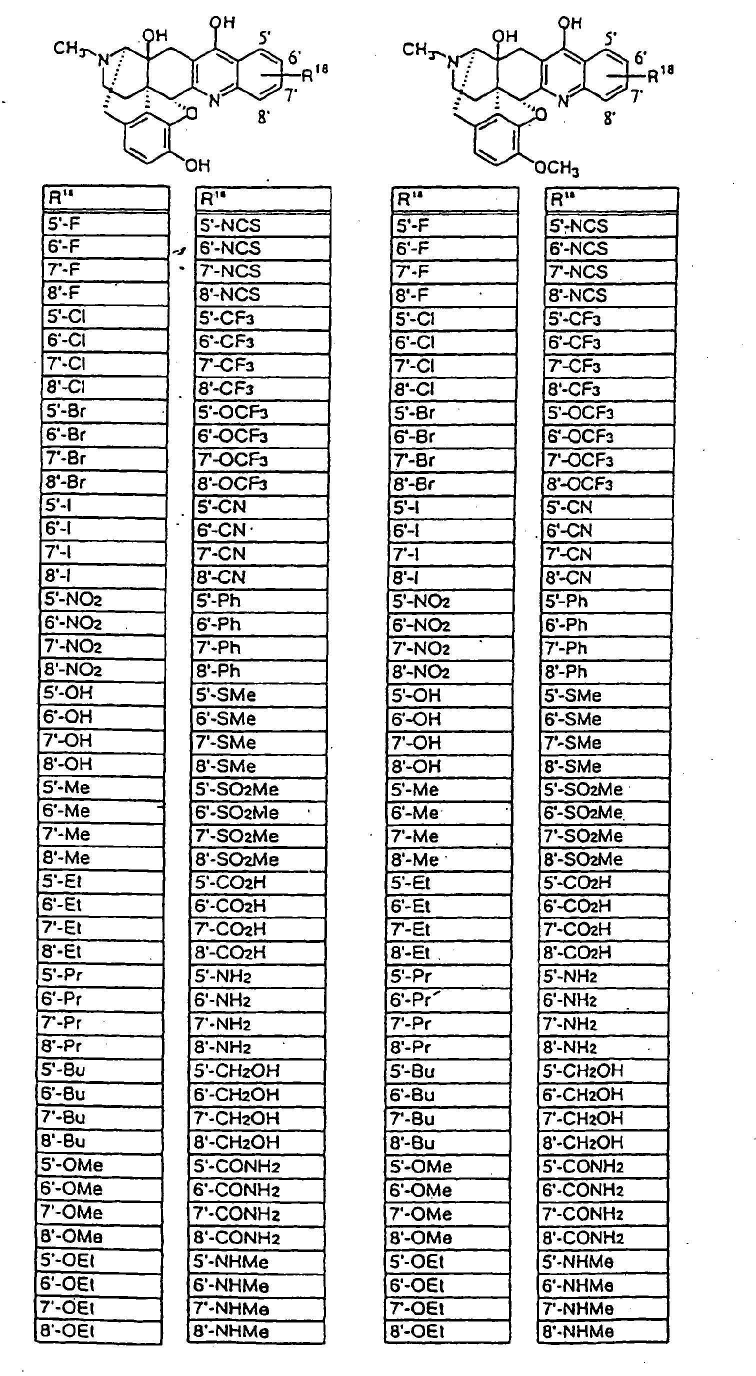

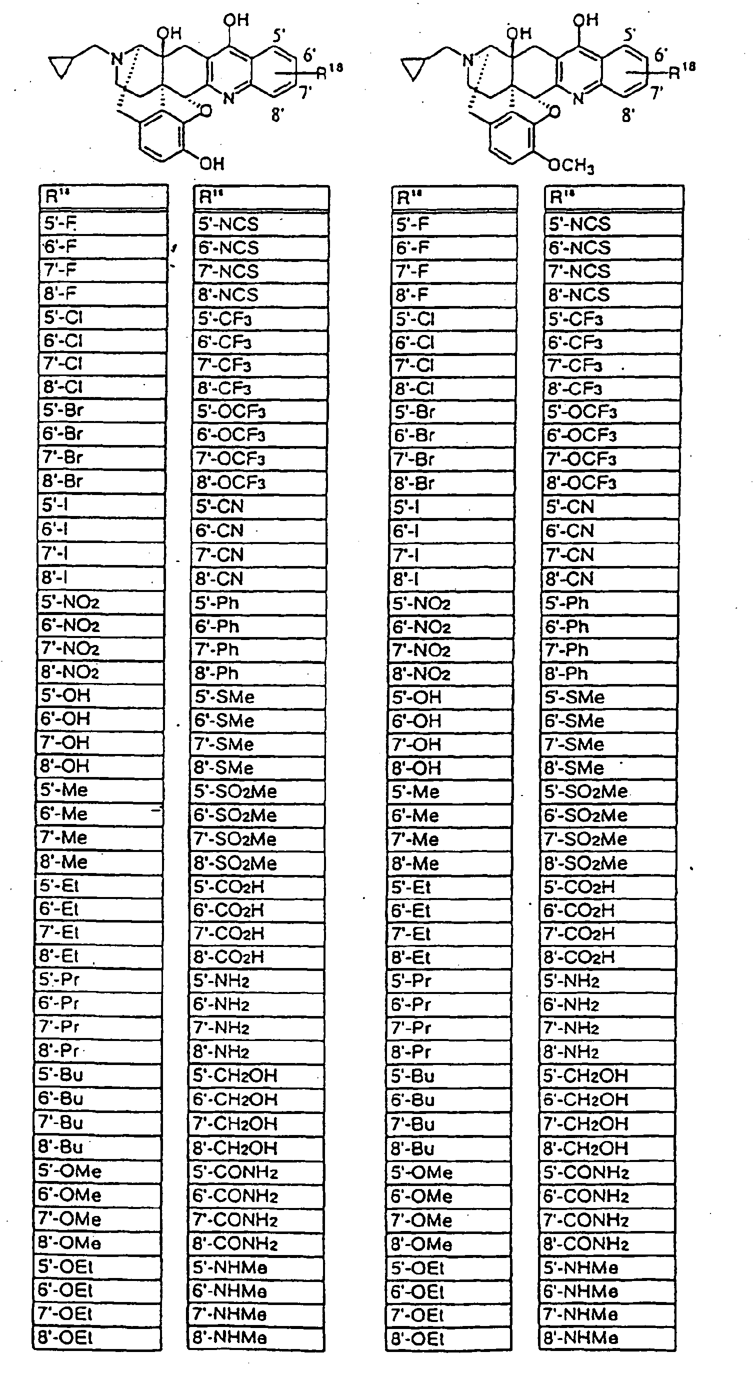

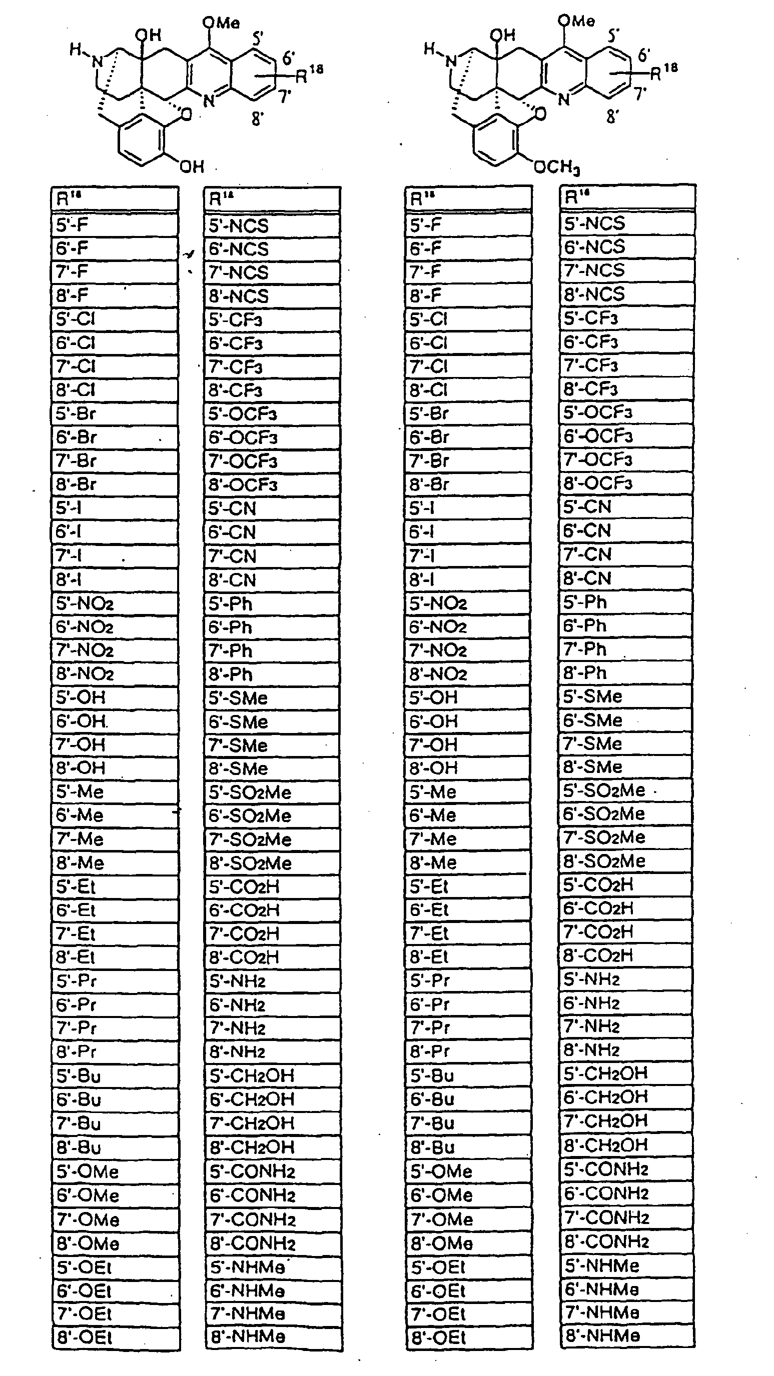

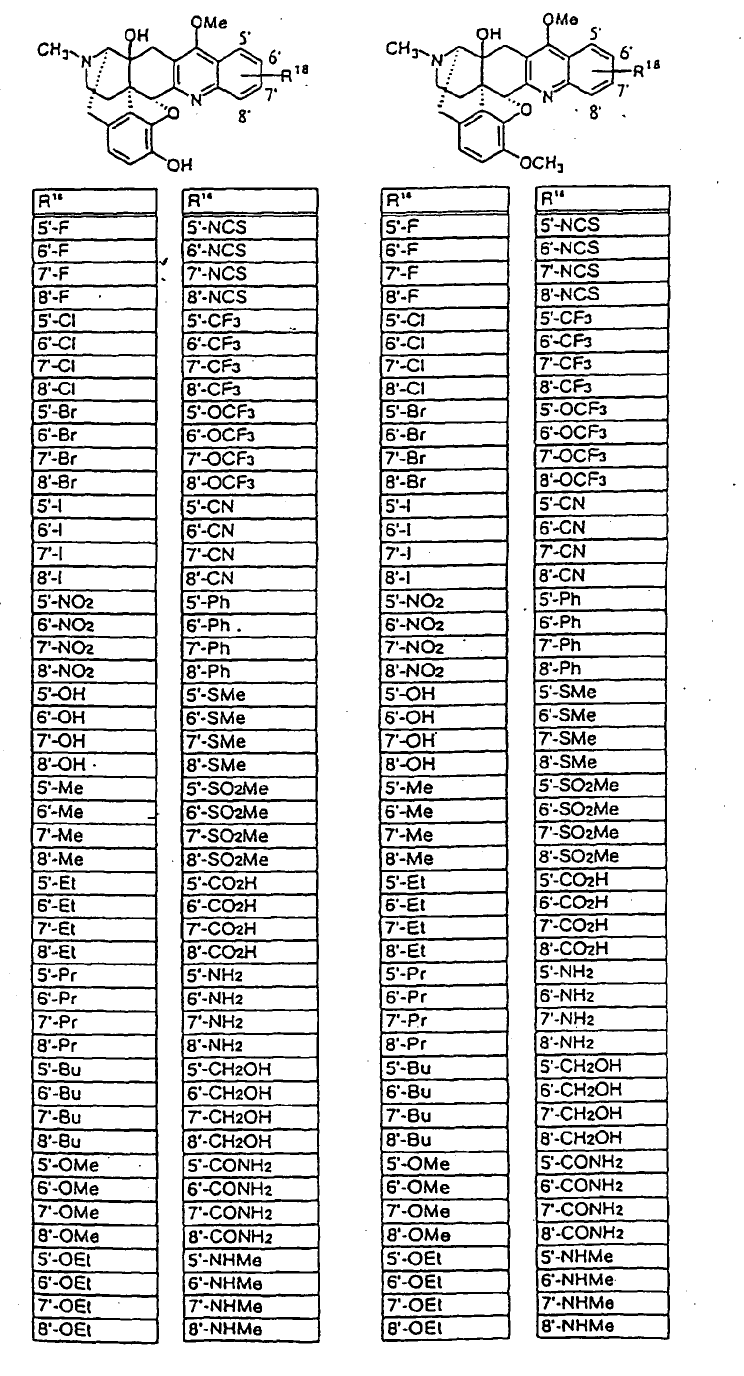

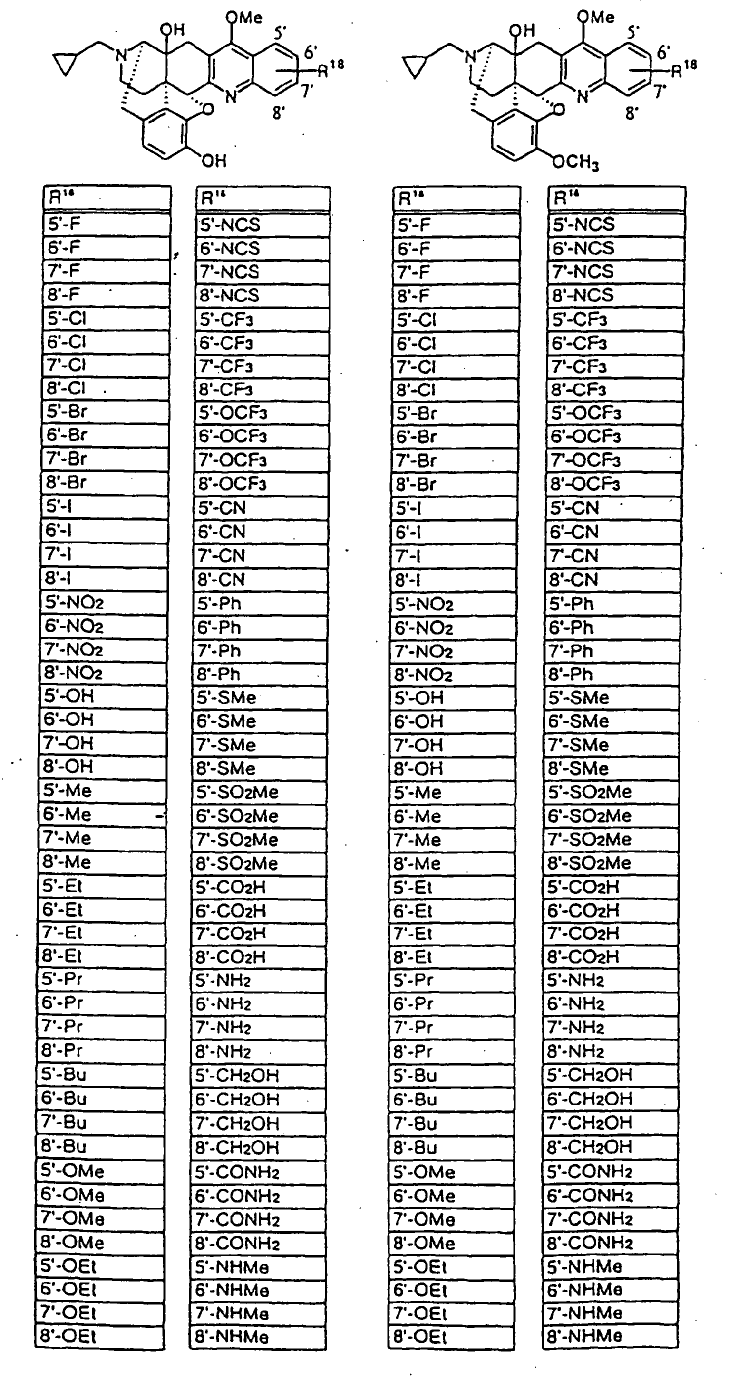

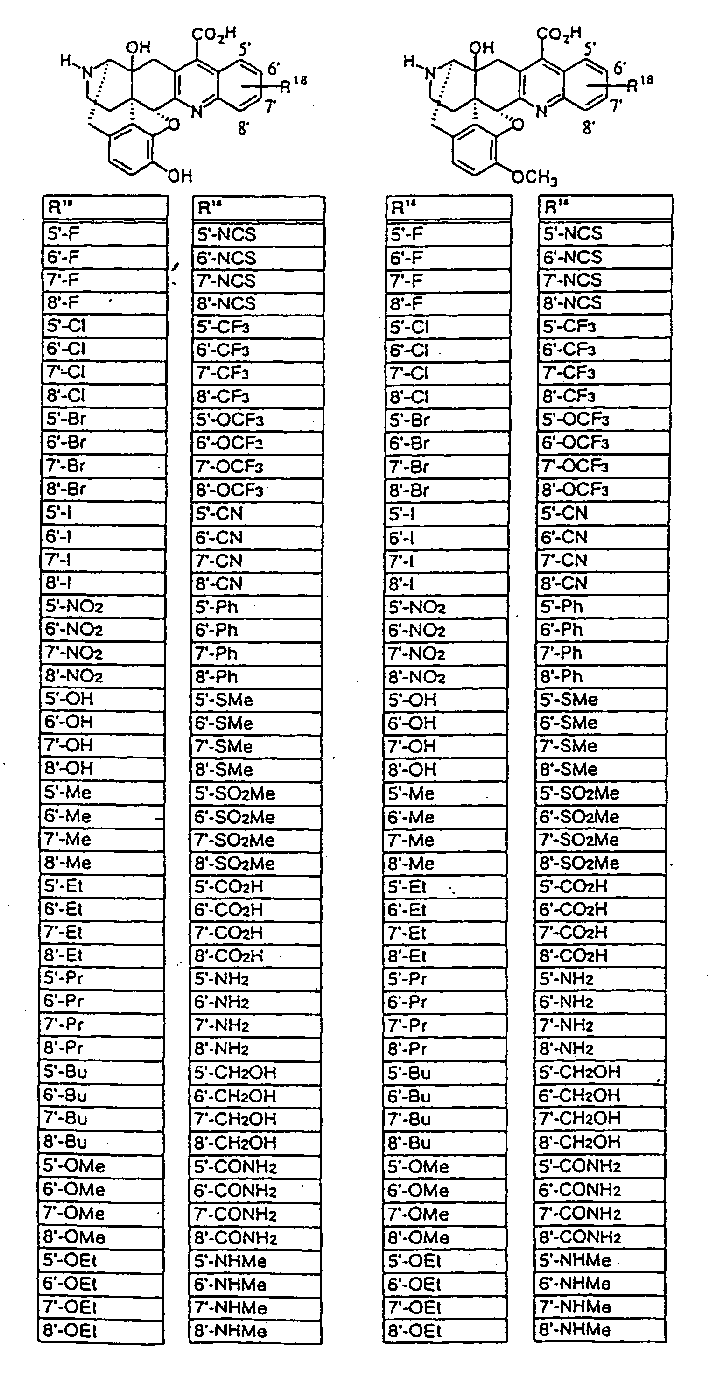

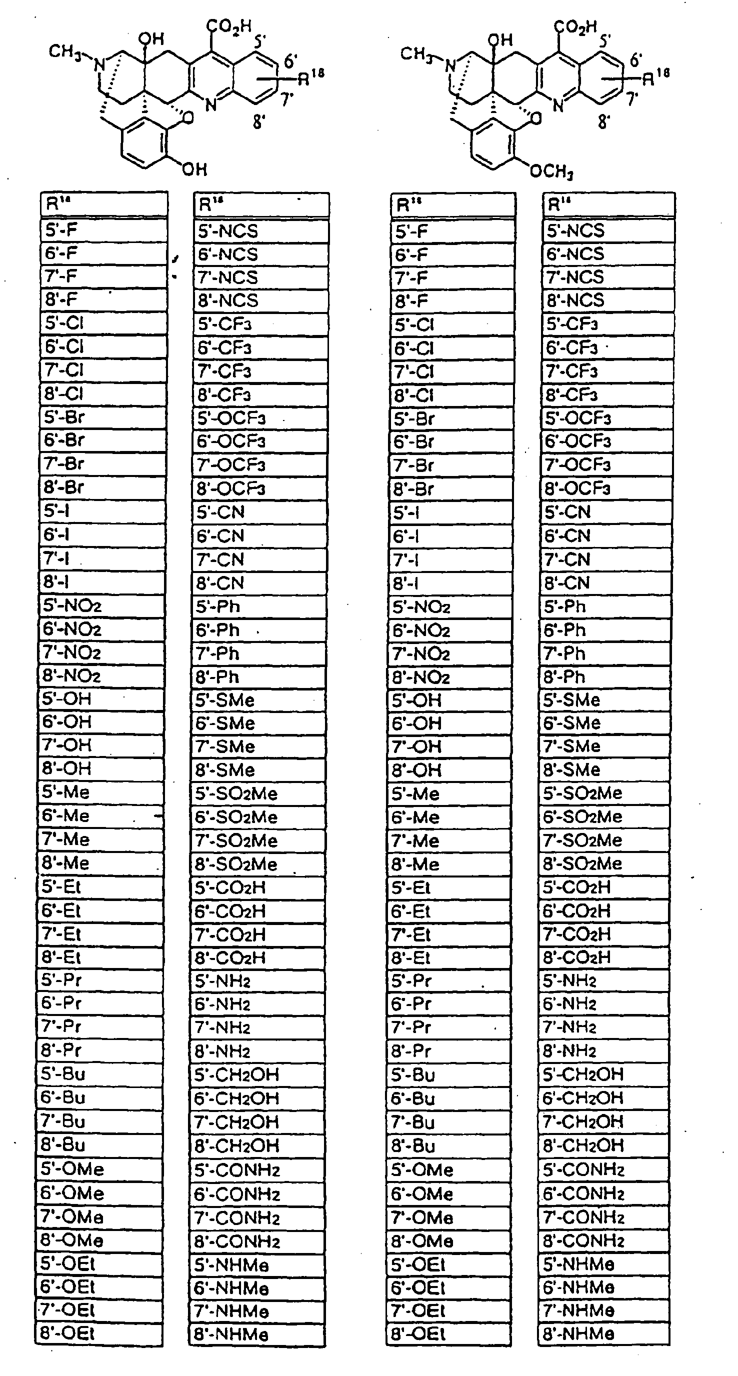

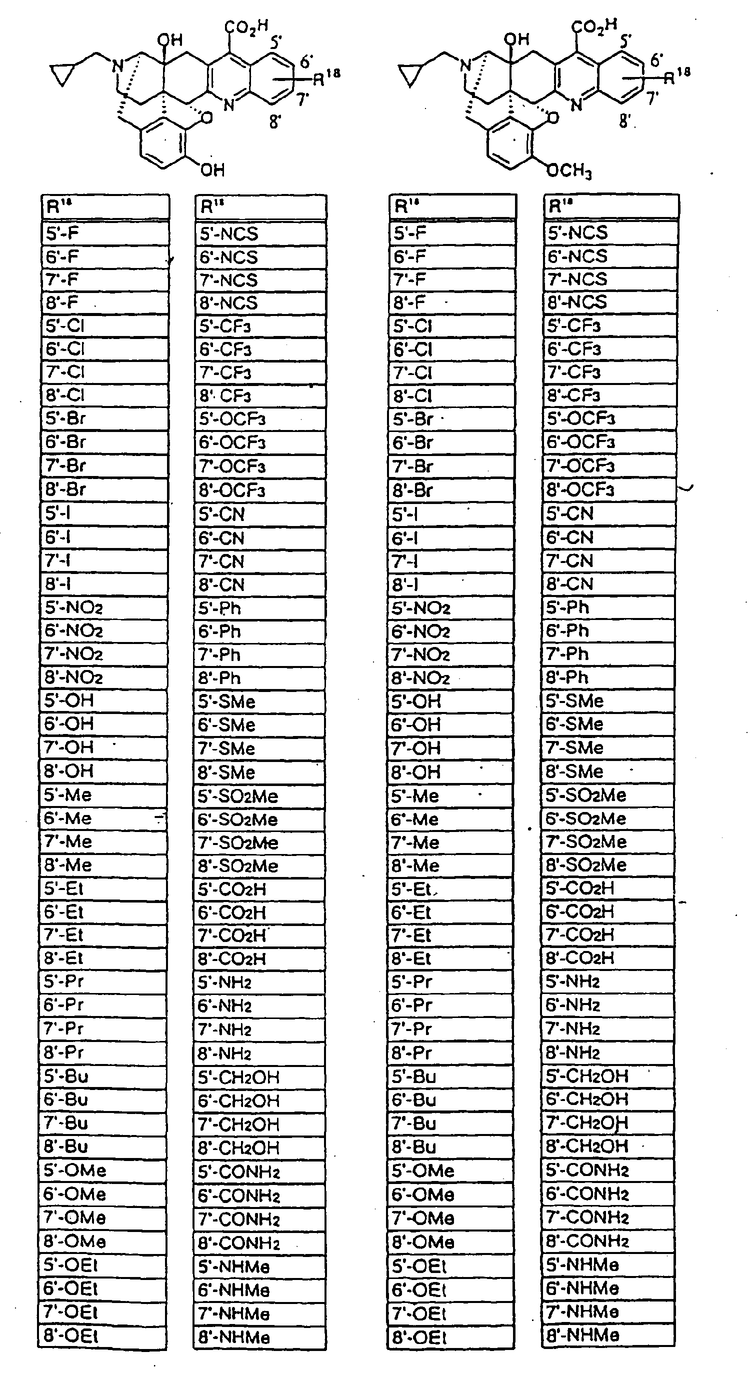

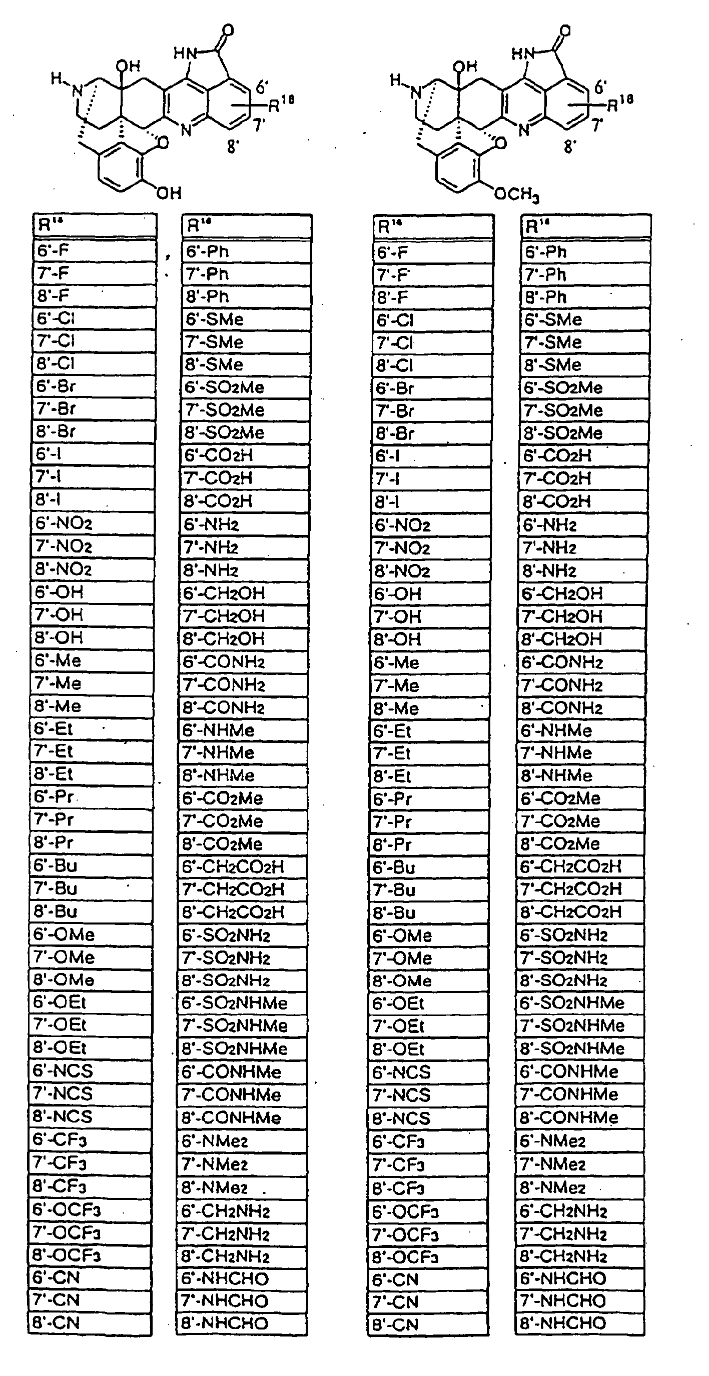

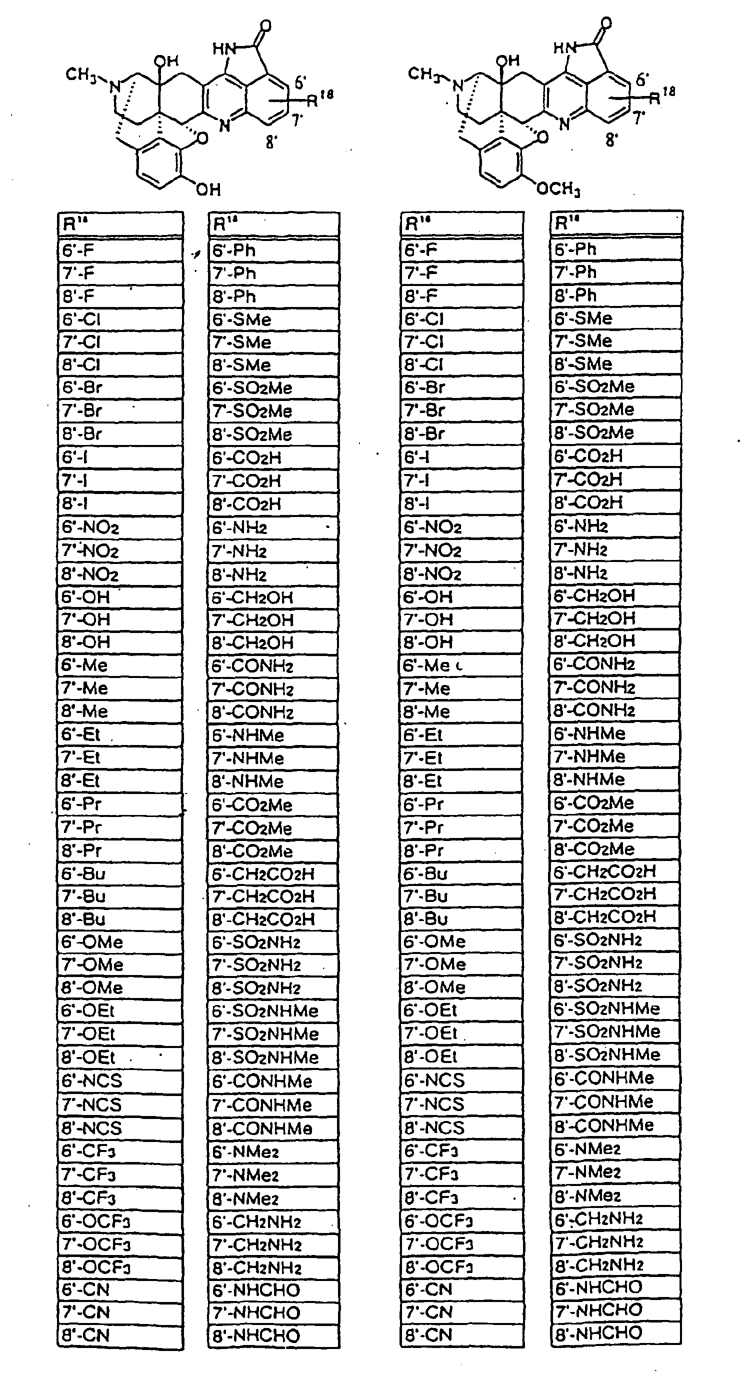

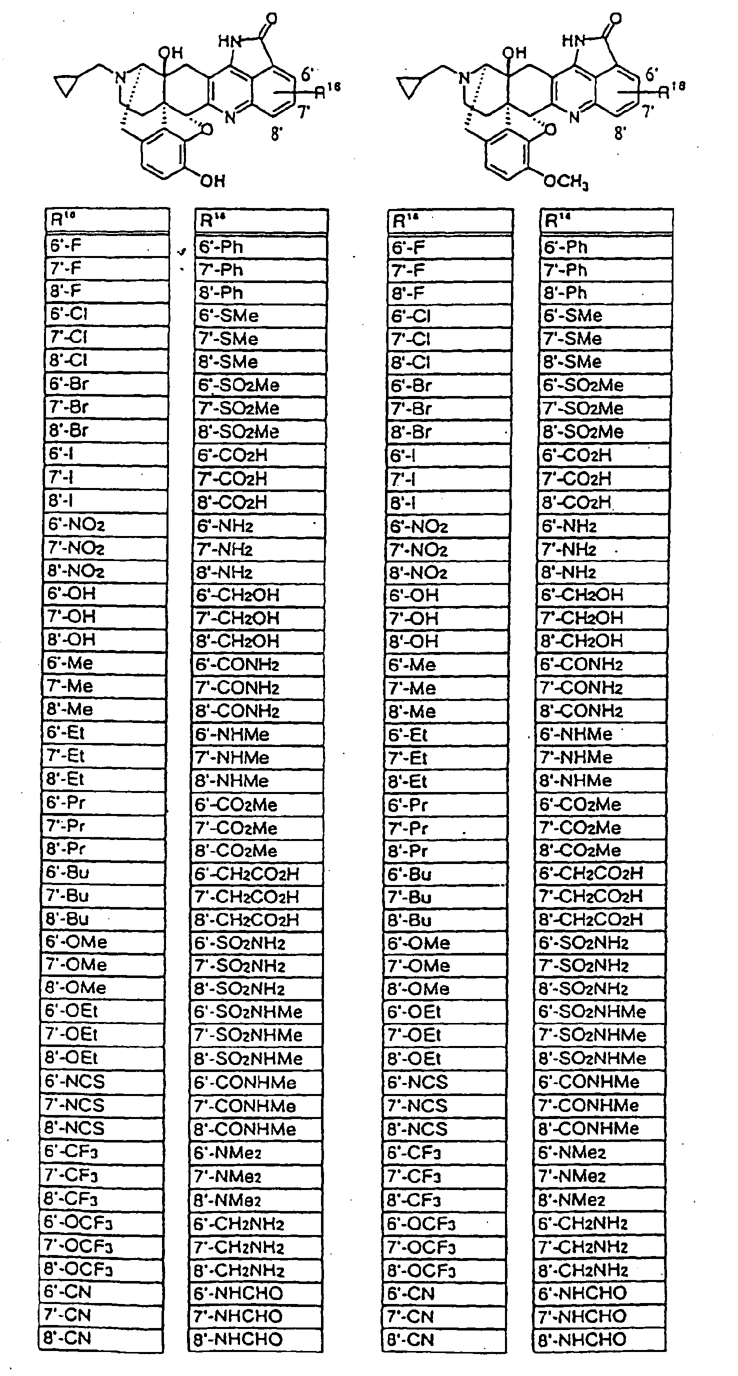

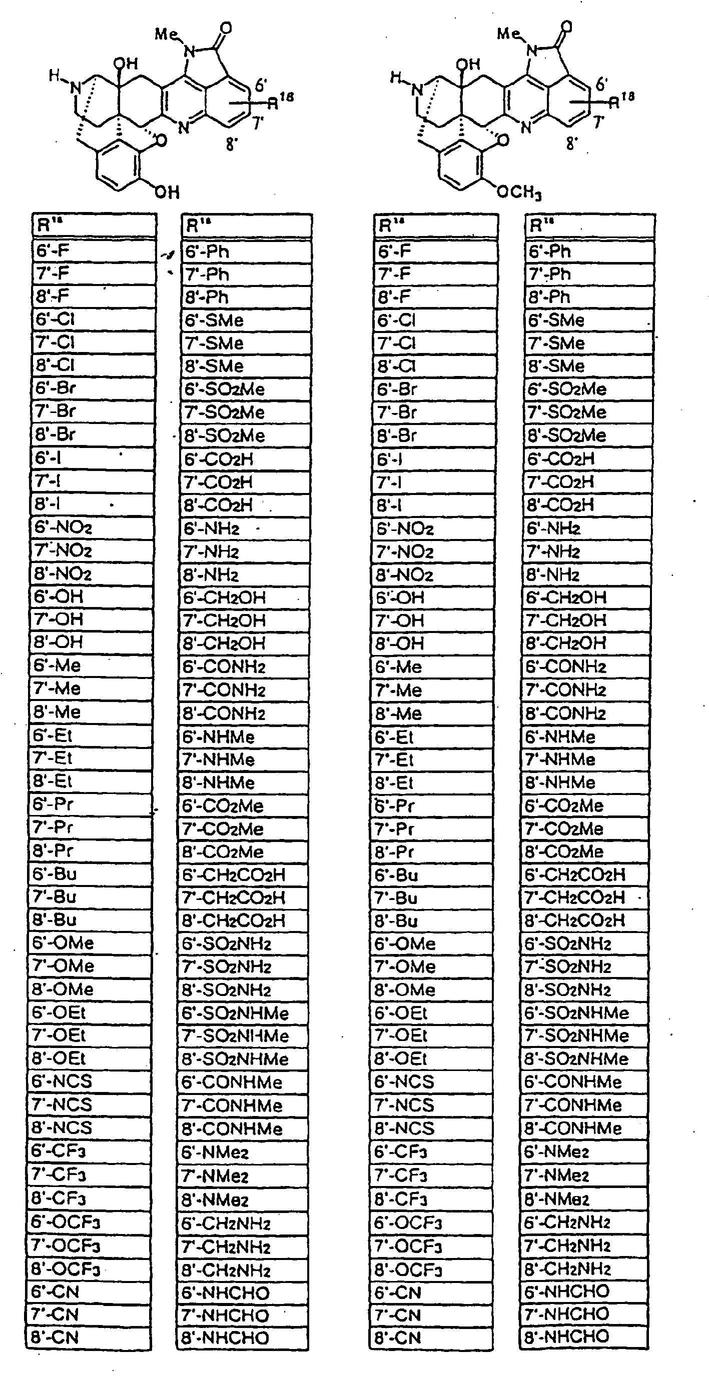

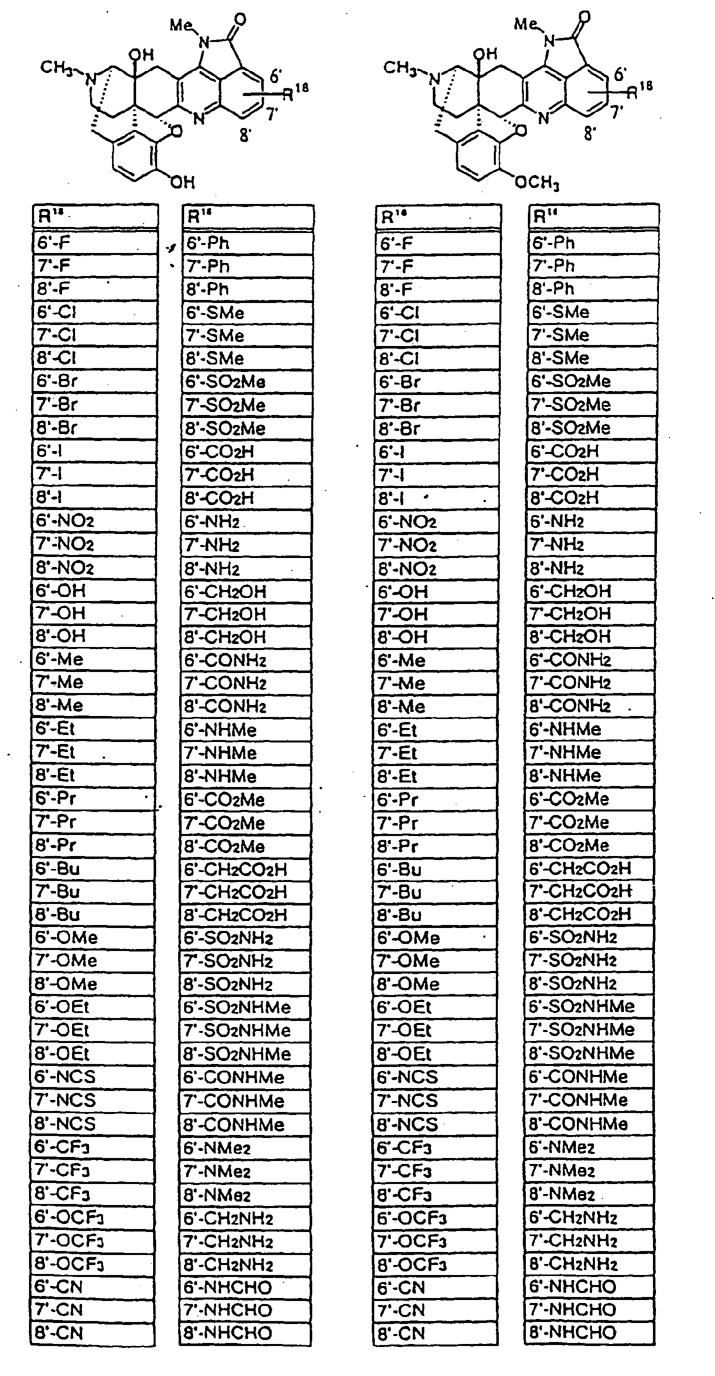

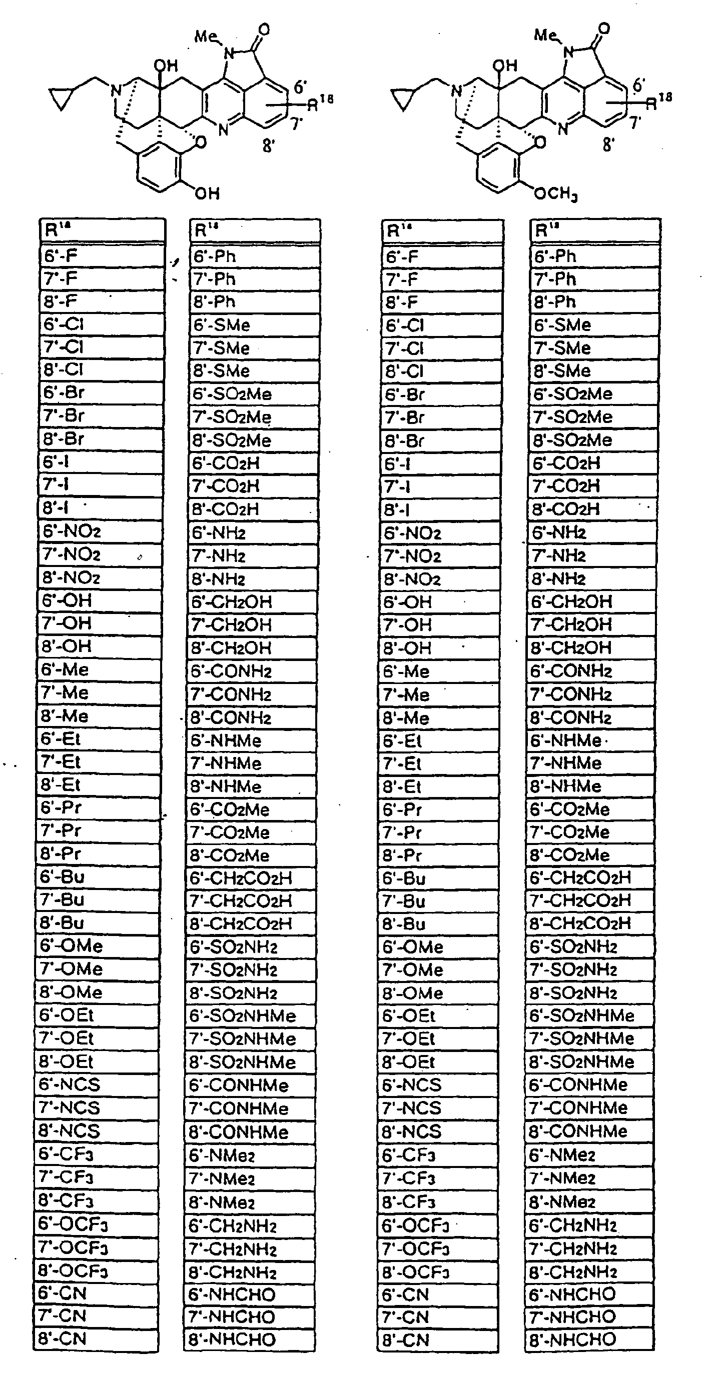

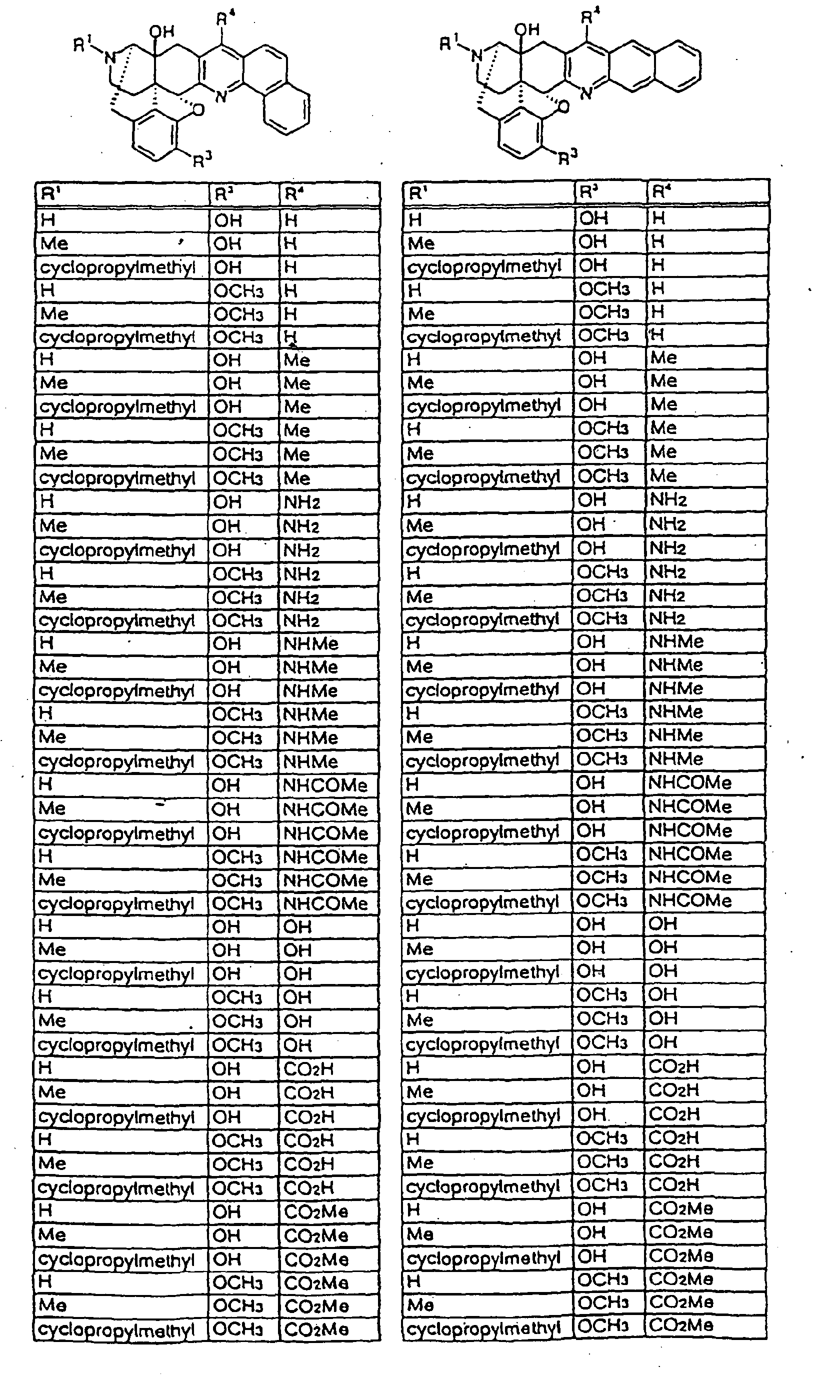

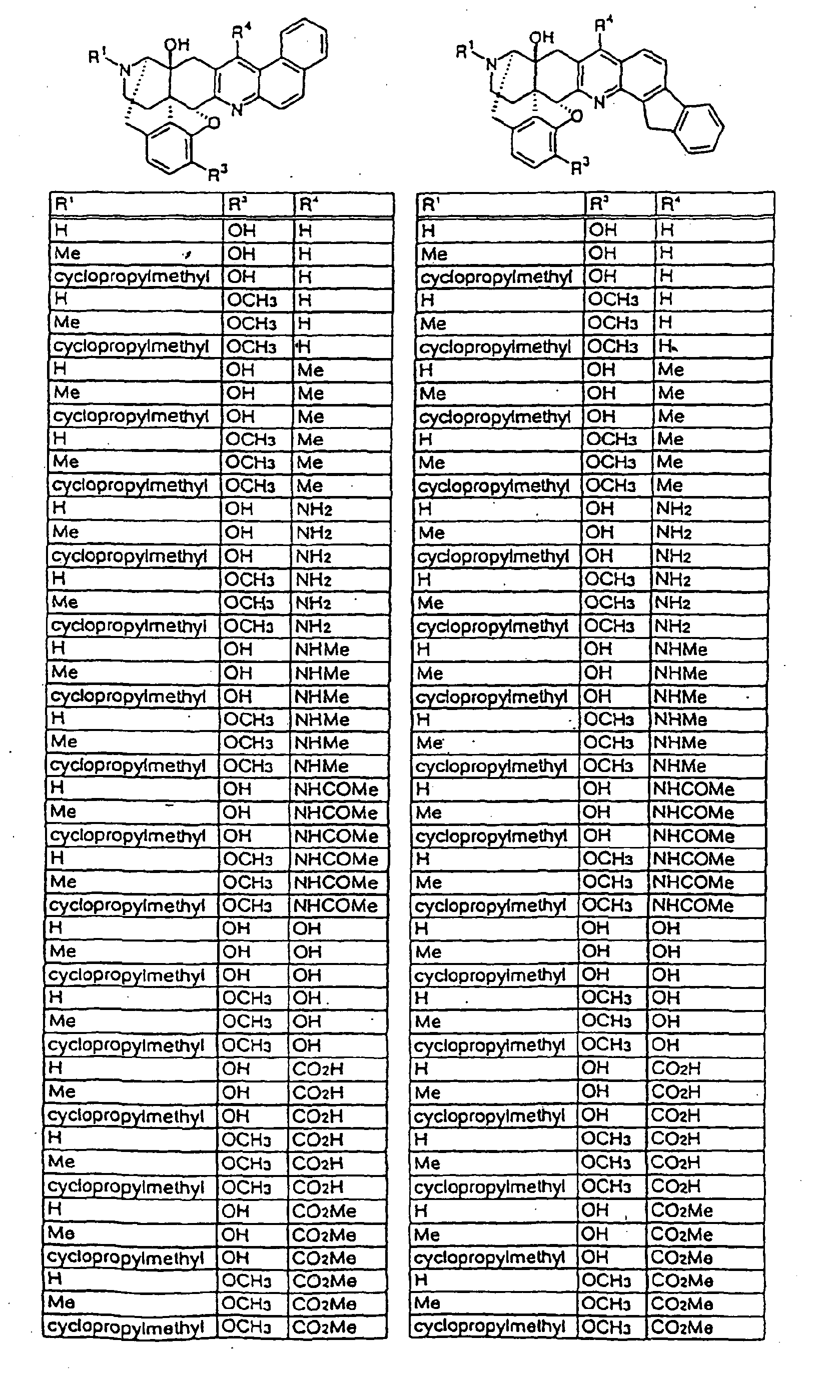

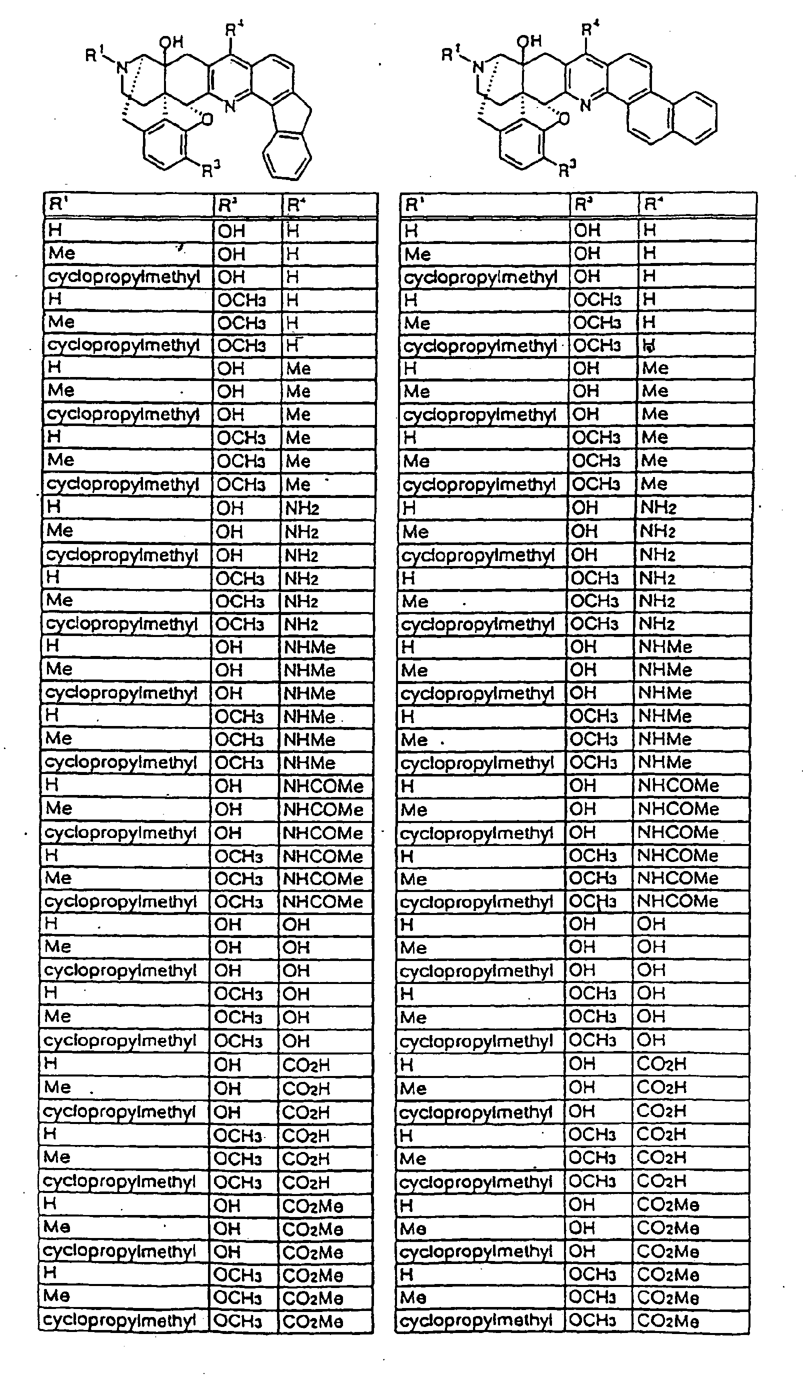

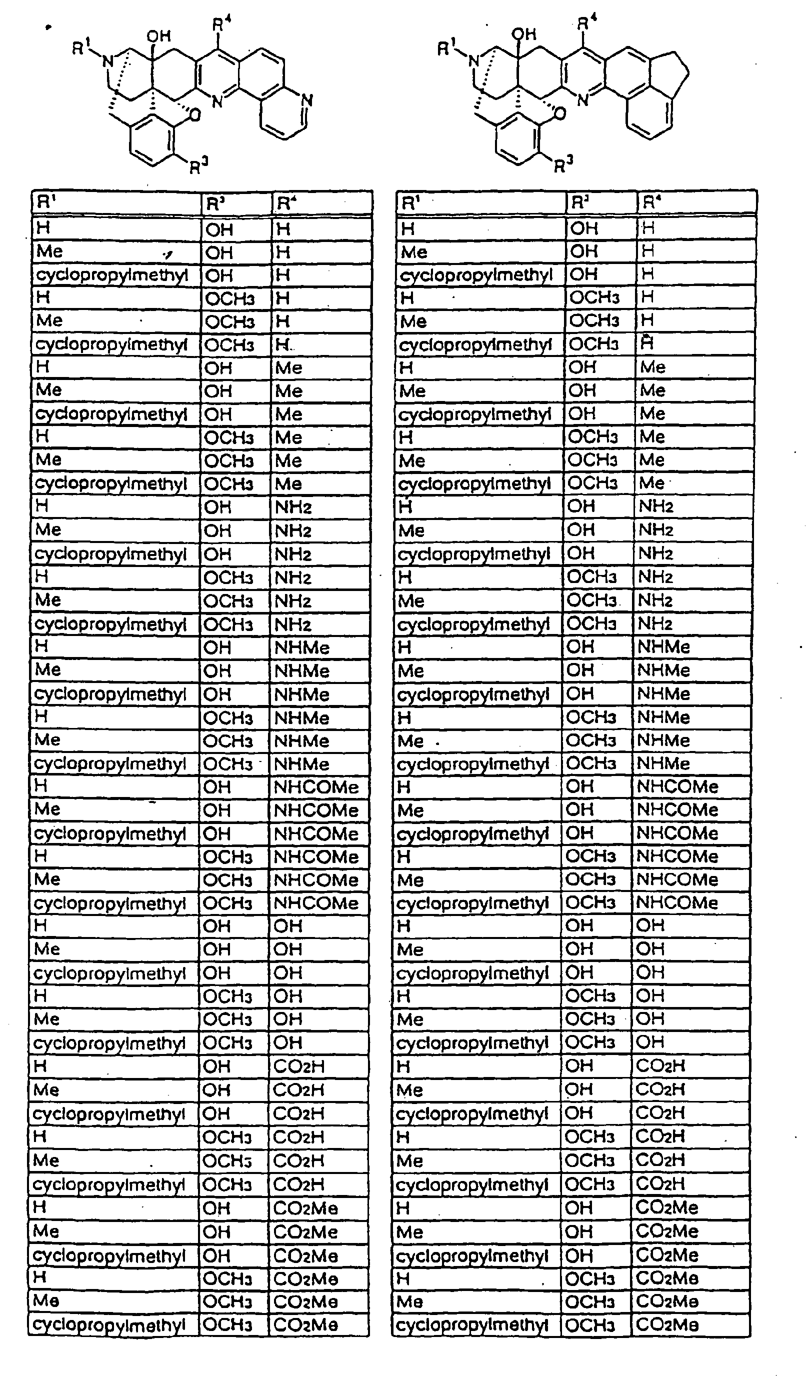

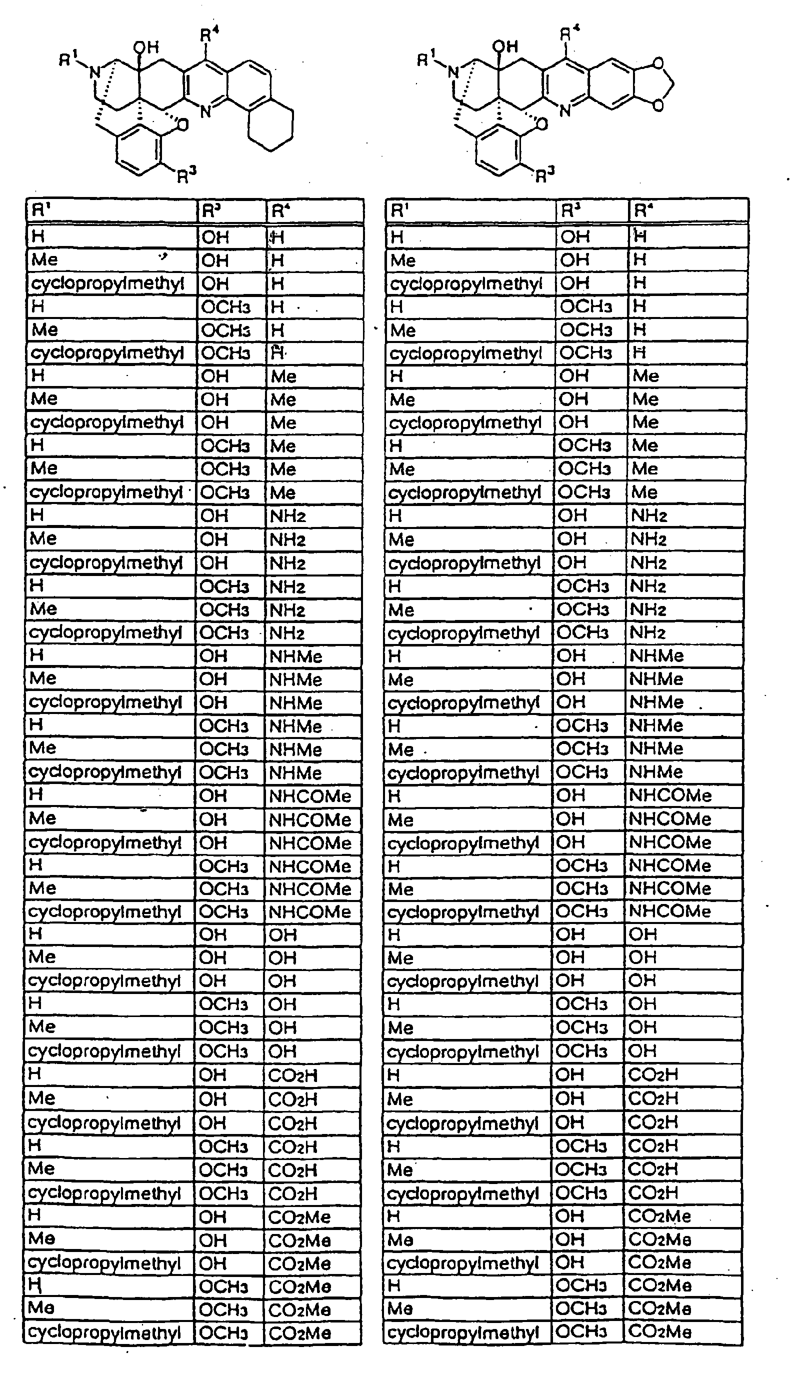

The compounds represented by formula (II) of the present invention (R¹, R², R³, R⁴ R⁵ and m are defined as the same as the above) can be generally produced by quinoline synthesis reaction using ketone compounds represented by formula (VII) (R¹, R¹ and R³ are defined as the same as the above) as raw materials, and aminocarbonyl derivatives presented by formula (VIII) (R⁴, R⁵ and m are defined as the same as the above) or aminobenzonitrile derivatives represented by formula (IX) (R⁵ and m are defined as the same as the above), as shown in Scheme 1. Of the compounds represented by formula (II), compounds wherein R⁴ is NR¹⁰R¹¹, and R¹⁰ and R¹¹ are each hydrogen can simply be produced by using aminobenzonitrile derivatives represented by formula (IX).

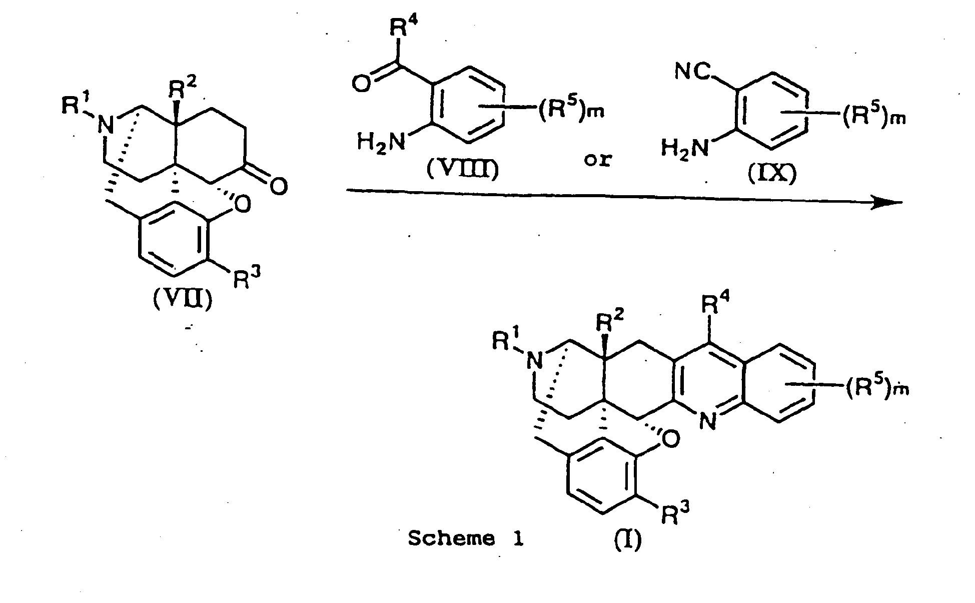

In the present invention, quinoline synthesis reaction can generally be effected in the presence of an appropriate acid according to demand in an appropriate solvent. Examples of the solvent include alcoholic solvents such as methanol, ethanol, and the like; organic carboxylic acid solvents such as formic acid, acetic acid, propionic acid, and the like; hydrocarbon solvents such as benzene, toluene, and the like; ether solvents such as diethyl ether, THF, and the like; halogenated hydrocarbon solvents such as chloroform, dichloromethane, dichloroethane, and the like: ester solvents such as ethyl acetate, and the like: aprotic polar solvents such as DMF, DMSO, and the like; water; and solvent mixtures thereof. Particularly, alcoholic solvents, organic acid solvents, and solvent mixtures of hydrocarbon solvents-aprotic polar solvents are preferably used. As reaction conditions, normal hearing conditions, azeotropic conditions or heating concentration conditions are preferably used. Examples of acids include any general acids such as inorganic acids such as hydrochloric acid, sulfuric acid, nitric acid, phosphoric acid, and the like; organic carboxylic acids such as formic acid, acetic acid, propionic acid, and the like: organic sulfonic acids such as methanesulfonic acid, p-toluenesulfonic acid, camphorsulfonic acid, and the like; Lewis acids such as zinc chloride, phosphorus trichloride, scandium triflate, and the like. Of these acids, hydrochloric acid, sulfuric acid, phosphoric acid, formic acid, acetic acid, propionic acid, methanesulfonic acid, p-toluenesulfonic acid, and scandium triflate are preferably used.

Of ketone compounds represented by formula (VII) and used as raw materials for quinoline synthesis reaction, compounds in which both of R² and R³ are hydroxy, and R¹ is hydrogen, methyl, allyl and cyclopropylmethyl are generally known as noroxymorphone, oxymorphone, naloxone, and nartrexone, respectively, and a compound in which R² is hydroxy, R³ is methoxy, and R¹ is hydrogen is generally known as noroxycodone. These compounds can be used without any change. Ketone compounds (VII) in which R¹ is a group other than the above can be prepared from noroxymorphone or noroxycodone in which R¹ is hydrogen by using the method disclosed in the document [J. Med. Chem., Vol. 35, 4329 (1992).] or the like. Specifically, by using ketone compounds (R² and R³ are defined as the same as the above) represented by formula (VII') in which R¹ is hydrogen, ketone compounds represented by formula (VII) can be prepared by (1) alkylation reaction using an alkyl halide R¹-X¹ (wherein X¹ represents chloro, bromo, iodo, or p-toluenesulfonyloxy) in the presence of an appropriate base, or (2) acylation reaction using an appropriate acid chloride R^{1a}-CO-Cl (wherein R^{1a} represents a group in which one terminal carbonyl is removed from R¹) according to a general method, as shown by the formula on the upper right of Scheme 2. The compounds of formula (II) can be produced by quinoline synthesis reaction using the thus-obtained ketone compounds represented by formula (VII) as raw materials.

Of compounds (II) of the present invention, compounds in which R⁴ is hydrogen, alkyl having 1 to 5 carbon atoms, aryl having 6 to 12 carbon atoms. NR¹⁰R¹¹, OR¹² , COOR¹³, or CONR¹⁴R¹⁵ (wherein R¹⁰, R¹¹ and R¹² independently represent alkyl having 1 to 5 carbon atoms, aralkyl having 7 to 13 carbon atoms, or alkanoyl having 1 to 5 carbon atoms; and R¹³, R¹⁴ and R¹⁵ independently represent alkyl having 1 to 5 carbon atoms, aryl having 6 to 12 carbon atoms, or aralkyl having 7 to 13 carbon atoms) can also be produced by the method shown by the formula on the under left of Scheme 2. In other words, such compounds can be produced by general alkylation or acylation reaction of an amino group by using compounds (I') (R², R³, R⁴, R⁵ and m are defined as the same as the above) produced by quinoline synthesis reaction using the ketone compounds (VII') (R² and R³ are defined as the same as the above) as raw materials. As a method for alkylation or acylation reaction of the amino group, any one of the methods (1), (2). (3) and (4) below can be used. Namely, by using the compounds (I') of the present invention, the compounds (II) can be produced by (1) alkylation reaction using an alkyl halide R¹-X¹ (wherein X¹ is defined as the same as the above) in the presence of an appropriate base, (2) reductive amination reaction using an appropriate aldehyde R^{1b}- CHO (wherein R^{1b} represents a group obtained by removing a methylene terminal from R¹) and a reducing agent such as sodium cyanoborohydride, sodium triacetoxyborohydride, or the like, or hydrogenation reaction, (3) acylation reaction using an appropriate acid chloride R^{1a}-CO-Cl (R^{1a} is defined as the same as the above) according to a general method, or (4) a method comprising acylation using an appropriate acid chloride R^{1b}-CO-Cl (R^{1b} is defined as the same as the above) and then reduction of amide by using a reducing agent such as lithium aluminum hydride, borane, or the like.

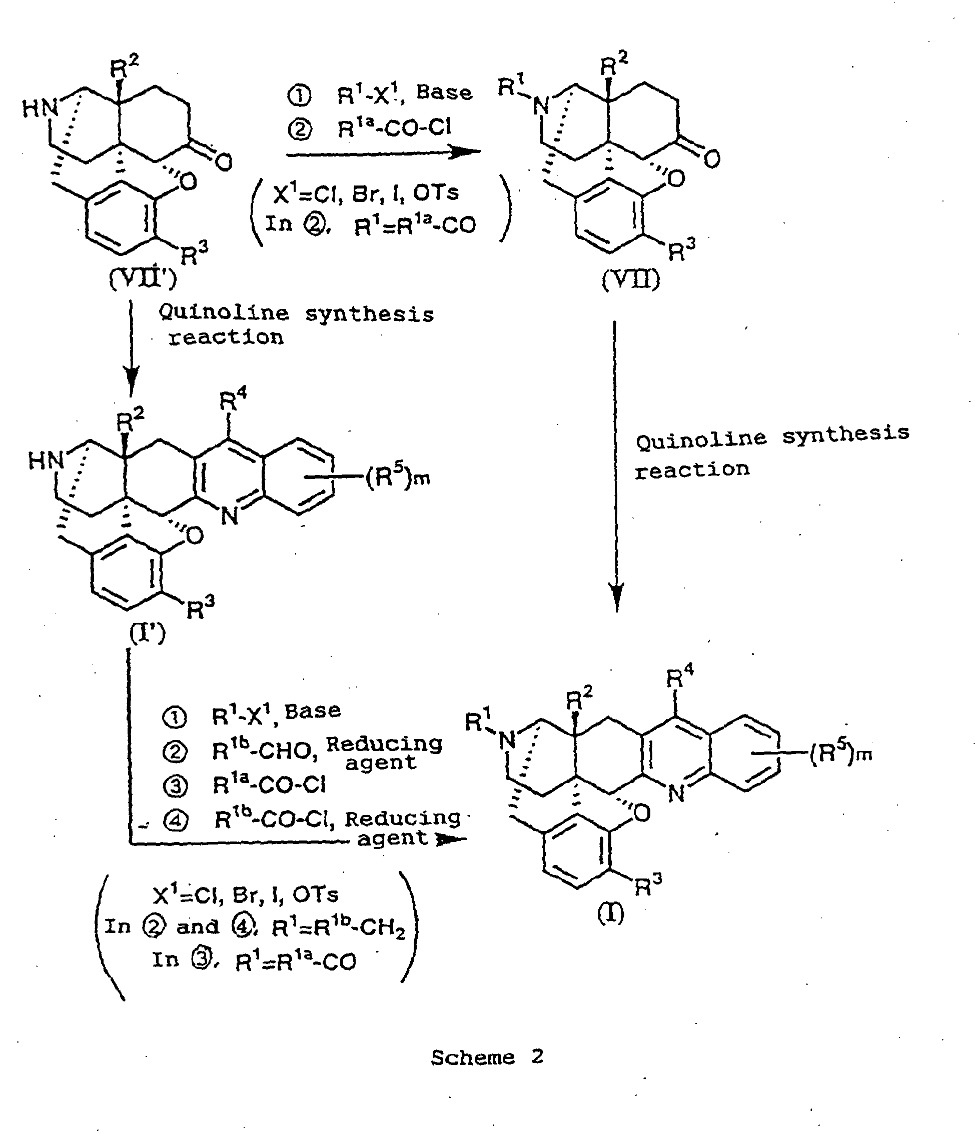

When an organic carboxylic acid solvent is used as a reaction solvent for quinoline synthesis reaction using the ketone compounds (VII') (wherein R² and R³ are defined as the same as the above) as raw materials, in some cases, compounds in which nitrogen at the 17-position is acylated are obtained. For example, the use of acetic acid sometimes produces compounds (I''), as shown in Scheme 2a. In this case, compounds (II'') can be converted into compounds (I') in which R¹ is hydrogen, by hydrolysis reaction.

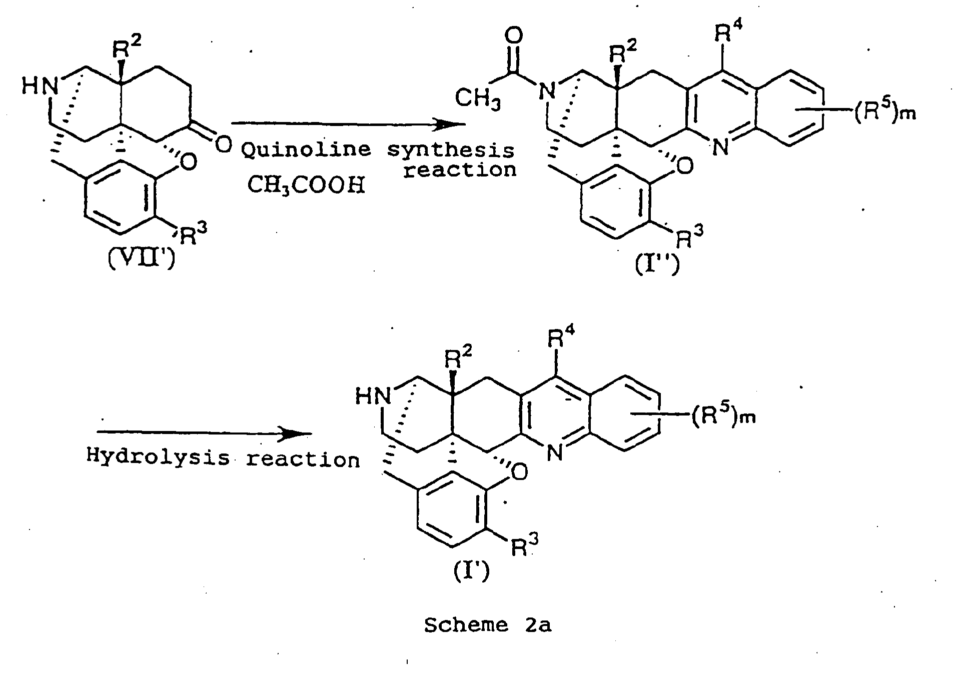

By applying the production method disclosed in U.S. Patent No. 4,816,586, of the compounds represented by formula (II) of the present invention, compounds represented by formula (Ia) in which R⁴ is hydrogen (R¹, R², R³ R⁵ and m are defined as the same as the above) can be produced by reacting the ketone compounds represented by formula (VII) and aminobenzaldehyde derivatives (VIIIa) (R⁵ is defined as the same as the above) in which R⁴ of aminocarbonyl derivatives of the formula (VIII) shown in Scheme 1 is hydrogen, as shown in Scheme 3.

Examples of the solvent used include alcoholic solvents such as methanol, ethanol, and the like; organic carboxylic acid solvents such as formic acid, acetic acid, propionic acid, and the like; hydrocarbon solvents such as benzene, toluene, and the like; and solvent mixtures thereof; particularly, ethanol, acetic acid, and toluene are preferable. Examples of the acid include inorganic acids such as hydrochloric acid, sulfuric acid, nitric acid, phosphoric acid, and the like; organic carboxylic acids such as formic acid, acetic acid, propionic acid, and the like; organic sulfonic acids such as methanesulfonic acid, p-toluenesulfonic acid, camphorsulfonic acid, and the like: Lewis acids such as zinc chloride, phosphorus trichloride, scandium triflate, and the like; particularly, hydrochloric acid and methanesulfonic acid are preferable.

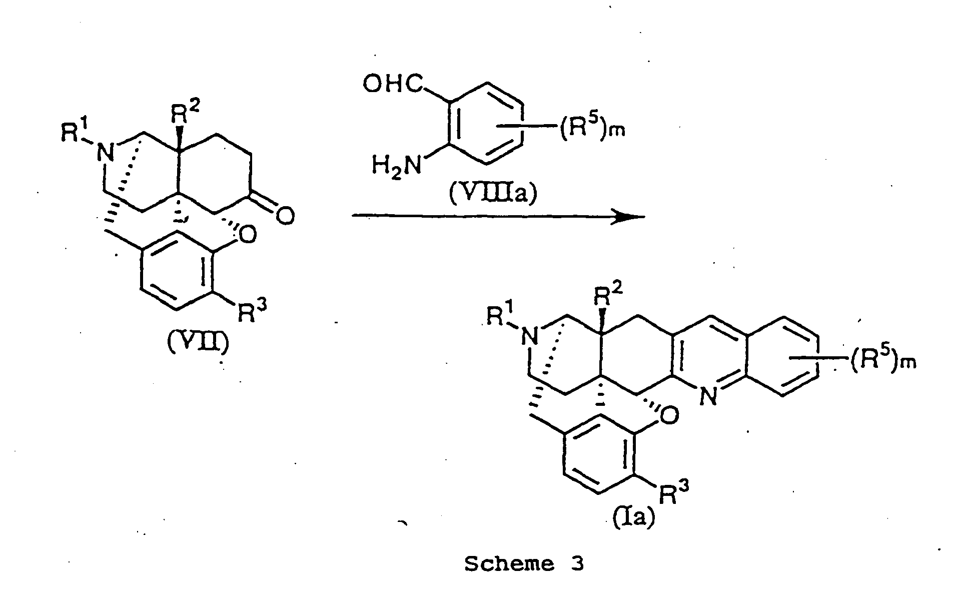

Compounds represented by the formula (Ia) can also be produced by reacting compounds represented by formula (X) (R¹, R² and R³ are defined as the same as the above) and acid addition salts or free compounds of aniline derivatives represented by formula (XI) (R⁵ is defined as the same as the above) in coexistence with an appropriate acid according to demand, as shown in Scheme 4.

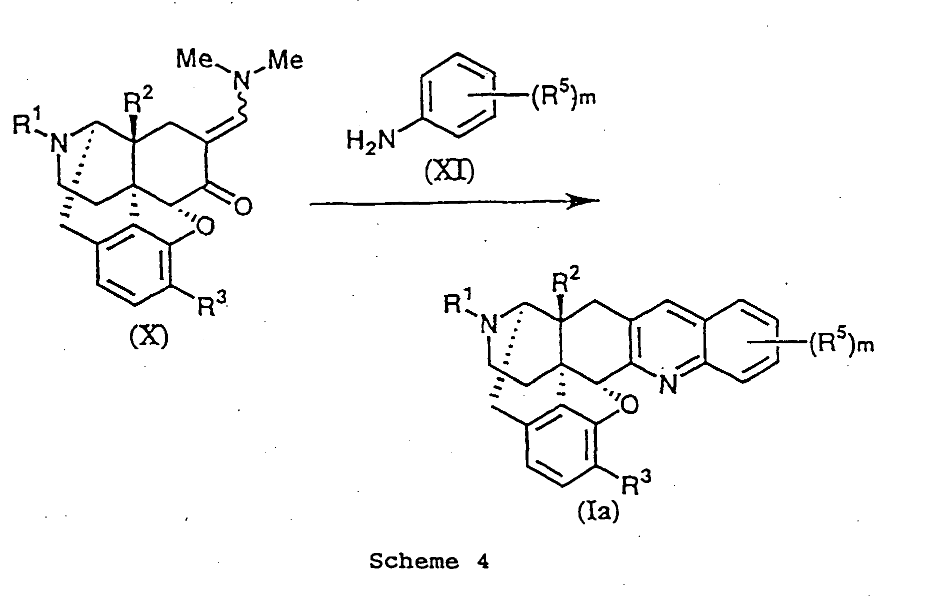

The compounds represented by formula (X) used as raw materials can be produced by reacting ketone compounds represented by formula (VII) and dimethylformamide dimethylacetal in a hydrocarbon solvent such as benzene, toluene, or the like, according to the method disclosed in the document [J. Med. Chem., 24, 1445 (1981)], as shown in Scheme 5. However, in the use of ketone compounds in which R³ of ketone compounds represented by formula (VII) is hydroxy, in some cases, the hydroxy group is methylated to compounds represented by formula (X) in which R³ is methoxy.

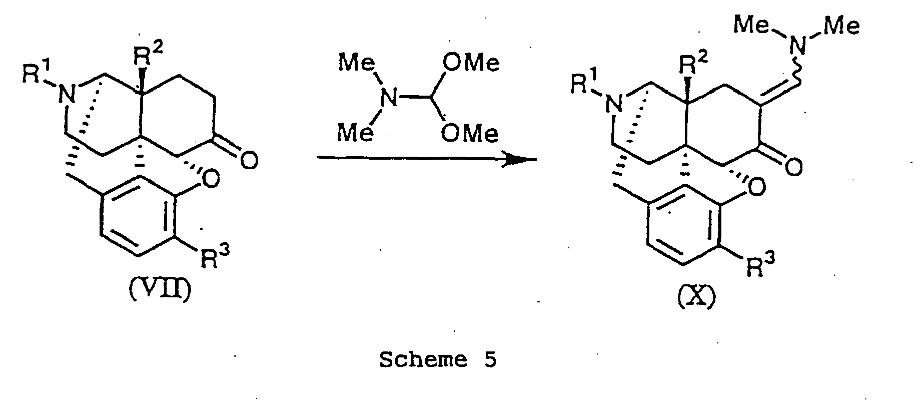

Examples of the solvent used in reaction of compounds represented by the formula (X) and compounds represented by the formula (XI) shown in Scheme 4 include alcoholic solvents such as methanol, ethanol, and the like; organic carboxylic acid solvents such as formic acid, acetic acid, propionic acid, lactic acid, and the like; hydrocarbon solvents such as benzene, toluene, and the like; and solvent mixtures thereof; particularly, acetic acid and lactic acid are preferable. Examples of the acid include inorganic acids such as hydrochloric acid, sulfuric acid, nitric acid, phosphoric acid, and the like; organic carboxylic acids such as formic acid, acetic acid, propionic acid, lactic acid, and the like; organic sulfonic acids such as methanesulfonic acid, p-toluenesulfonic acid, camphorsulfonic acid, and the like; Lewis acids such as zinc chloride, phosphorus trichloride, scandium triflate, and the like; generally, the use of acetic acid also used as the solvent produces sufficient effects. In some cases in which an intermediate is precipitated during reaction, the intermediate is filtered off, and dissolved in an appropriate solvent to effect reaction again. In purification of the compounds (Ia) of the present invention, the residual intermediate can easily be sometimes removed by hydrolysis reaction.

Of the compounds of formula (II) of the present invention, compounds represented by formula (Ib) (R¹, R¹, R³, R⁵ and m are defined as the same as the above) in which R⁴ is NR¹⁰R¹¹, and R¹⁰ and R¹¹ are each hydrogen can be produced by reacting ketone compounds represented by formula (VII) and aminobenzonitrile derivatives represented by formula (IX) (R⁵ and m are defined as the same as the above) in coexistence with an appropriate acid according to demand, as shown in Scheme 6.

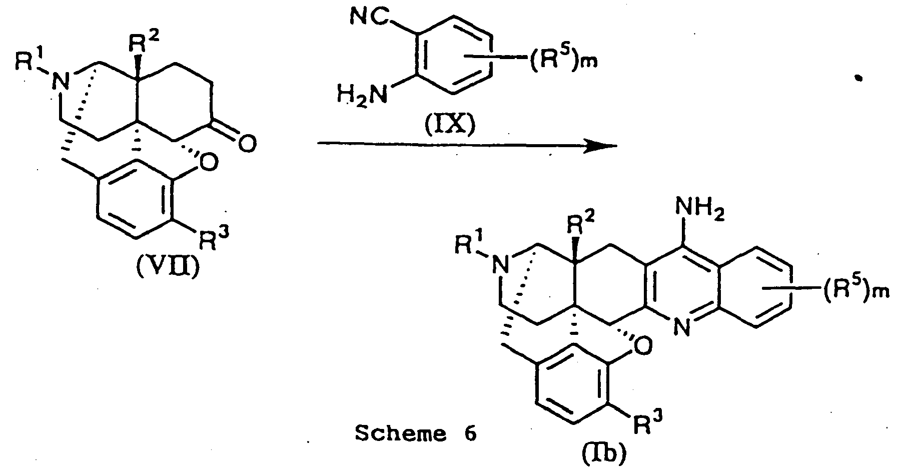

Examples of the solvent include alcoholic solvents such as methanol, ethanol, and the like; organic carboxylic acid solvents such as formic acid, acetic acid, propionic acid, and the like; hydrocarbon solvents such as benzene, toluene, and the like; water; and solvent mixtures thereof; particularly, hydrocarbon solvents such as benzene, toluene, and the like; organic carboxylic acid solvents such as acetic acid and propionic acid, and the like; water; and solvent mixtures thereof are preferable. Particularly, the use of toluene or acetic acid produces good results. Examples of the acid include inorganic acids such as hydrochloric acid, sulfuric acid, nitric acid, phosphoric acid, and the like; organic carboxylic acids such as formic acid, acetic acid, propionic acid, and the like; organic sulfonic acids such as methanesulfonic acid, p-toluenesulfonic acid, camphorsulfonic acid, and the like; Lewis acids such as zinc chloride, zinc trichloride, cuprous chloride, phosphorus trichloride, scandium triflate, and the like. Particularly, hydrochloric acid, sulfuric acid, acetic acid, propionic acid, methanesulfonic acid, zinc chloride, cuprous chloride, scandium triflate are preferable; the use of acetic acid, propionic acid or scandium triflate produces good results.

The aminobenzonitrile derivatives (IX) used in the reaction shown in Scheme 6 may be commercially available compounds or prepared from corresponding aniline derivatives (XI) (R⁵ and m are defined as the same as the above) by using the method disclosed in the document [Synth. Comm., 20(1), 71 (1990)], as shown in Scheme 7. The aminobenzonitrile derivatives (IX) used can also be prepared from corresponding nitrobenzonitrile derivatives (XII) (R⁵ and m are defined as the same as the above) by reduction of a nitro group using the method disclosed in the document [J. Med. Chem., 24, 742 (1981)], as shown in Scheme 8.

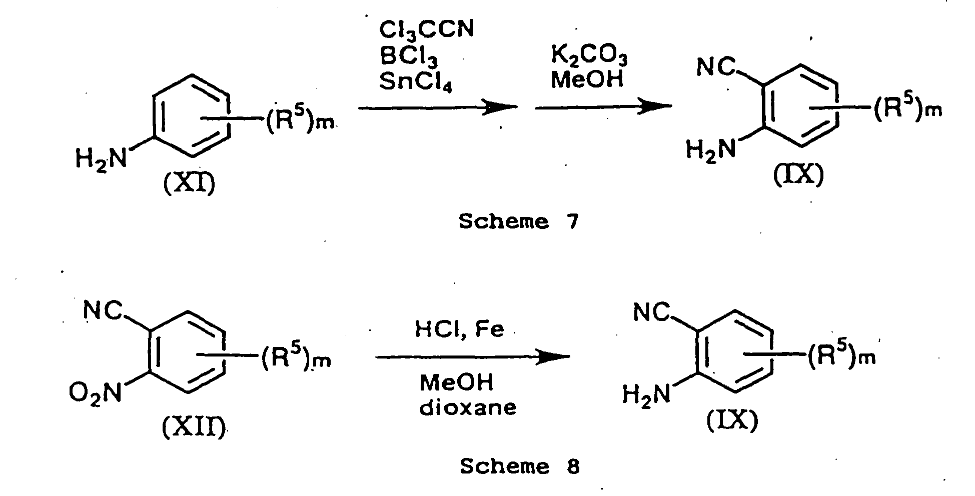

Of the compounds of formula (II) of the present invention, compounds represented by formula (Id) (R¹, R², R³, R⁵ and m are defined as the same as the above) in which R⁴ is NR¹⁰R¹¹, R¹⁰ is hydrogen, alkyl having 1 to 5 carbon atoms, aralkyl having 7 to 13 carbon atoms; or alkanoyl having 1 to 5 carbon atoms, and R¹¹ is independently alkyl having 1 to 5 carbon atoms, aralkyl having 7 to 13 carbon atoms, or alkanoyl having 1 to 5 carbon atoms can be produced by alkylation or acylation of amino groups in compounds represented by formula (Ic) (R¹, R², R³, R⁵, m and R¹⁰ are defined as the same as the above) according to a general method, as shown in Scheme 9.

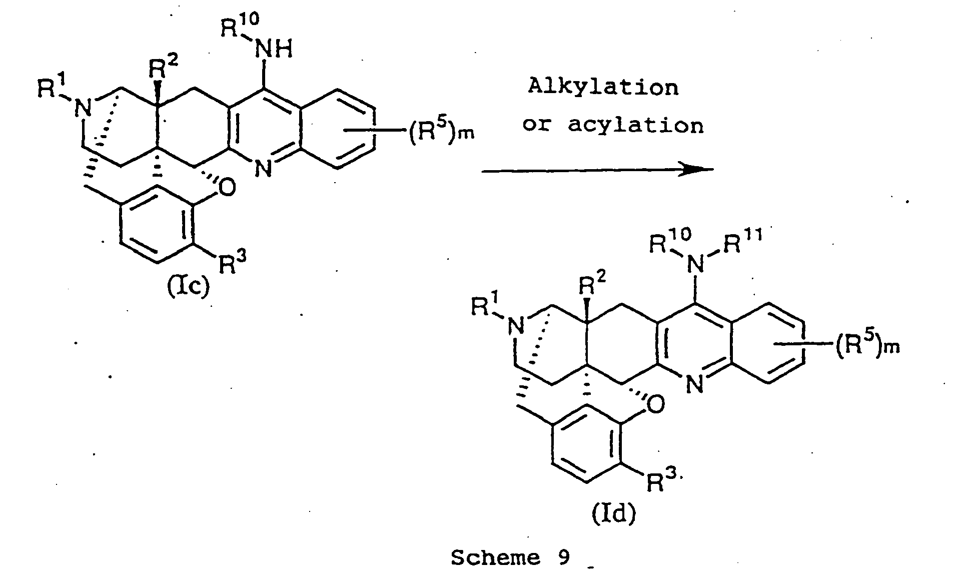

In the scheme 9, of compounds represented by formula (Ic) and used as raw materials, compounds represented by formula (Ic') (R¹, R², R³, R⁵ and m are defined as the same as the above) in which R¹⁰ is R^{10a} which is alkyl having 1 to 5 carbon atoms, aralkyl having 7 to 13 carbon atoms, or alkanoyl having 1 to 5 carbon atoms, can be produced by alkylation or acylation of the amino groups by a general method of converting the amino groups using compounds represented by formula (Ib) as raw materials, as shown in Scheme 10.

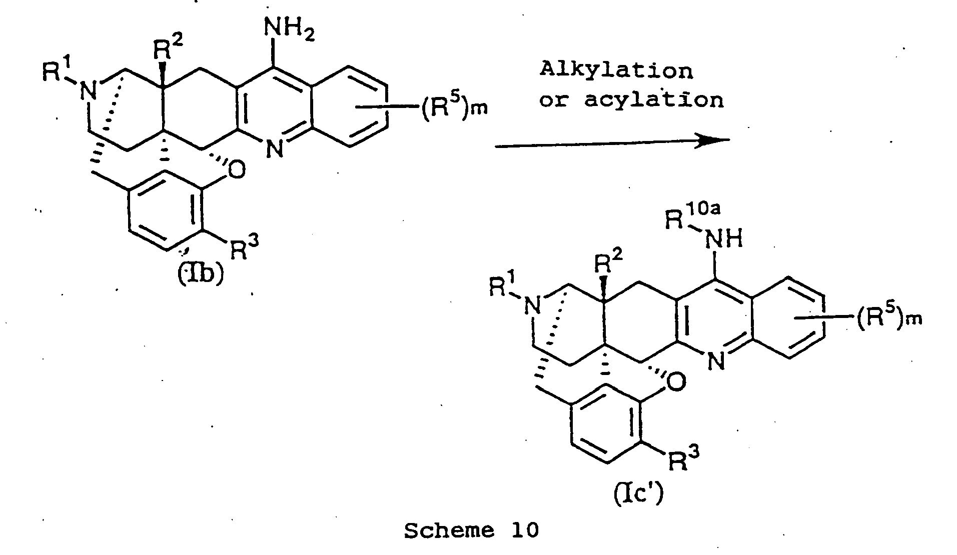

As described above, examples of the general method of converting the amino groups include (1) a method using a halide R¹¹-X¹ (wherein X¹ is defined as the same as the above) in the presence of an appropriate base, (2) reductive amination reaction using an appropriate aldehyde R^{11a}-CHO (wherein R^{11a} represents a group obtained by removing a methylene terminal from R¹¹) and a reducing agent such as sodium cyanoborohydride, sodium triacetoxyborohydride, or the like, or hydrogenation reaction, (3) acylation reaction using an appropriate acid chloride R^{11b}-CO-C1 (R^{11b} represents a group obtained by removing a carbonyl terminal from R¹¹) according to a normal method, and (4) an alkylation method comprising acylation using an appropriate acid chloride R^{11b}-CO-Cl (R^{11b} is defined as the same as the above) and then reduction of amide by using a reducing agent such as lithium aluminum hydride, borane, or the like. However, of compounds represented by formula (Id), in some cases, compounds (Id) in which R² and/or R³ is hydroxy are preferably produced by protecting the corresponding hydroxy groups of the compounds (Ic) as raw materials by appropriate protective groups, and then removing the protective groups by the above alkylation or acylation method.

The method shown in Scheme 9 is described in further detail below. Of the compounds of formula (Id) of the present invention, compounds in which R¹⁰ is hydrogen, alkyl having 1 to 5 carbon atoms, or aralkyl having 7 to 13 carbon atoms, and R¹¹ is alkyl having 1 to 5 carbon atoms, or aralkyl having 7 to 13 carbon atoms, can simply be produced by reacting compounds represented by formula (Ic) of the present invention and aldehyde represented by R^{11a}-CHO (R^{11a} represents a group obtained by removing terminal methylene from R¹¹) under acidic or basic conditions, and then reducing the resultant imine or iminium compounds. The reaction of converting to the imine or iminium compounds is generally effected by using an alcoholic solvent such as methanol, ethanol, or the like, an ether solvent such as THF or the like, or a hydrocarbon solvent such as benzene, toluene, or the like as a solvent, hydrochloric acid, sulfuric acid, acetic acid, p-toluenesulfonic acid, camphorsulfonic acid, or titanium tetrachloride as an acid, and piperidine or the like as a base; the solvent, the acid and the base are not limited to these compounds. General methods of reduction reaction include methods using sodium borohydride, sodium cyanoborohydride, sodium triacetoxyborohydride, lithium borohydride, or the like as a reducing agent in an alcoholic solvent such as methanol, ethanol, or the like; the reduction methods are not limited to these methods.

For example, of the compounds of formula (Id) of the present invention, compounds in which R¹⁰ is hydrogen, alkyl having 1 to 5 carbon atoms, or aralkyl having 7 to 13 carbon atoms, and R¹¹ is alkanoyl having 1 to 5 carbon atoms, can be produced by condensation of compounds represented by formula (Ic) of the present invention with a carboxylic acid chloride R^{11a}-CO-Cl, a carboxylic acid anhydride (R^{11a}CO)₂O or a carboxylic acid R^{11a}-COOH (R^{11a} is defined as the same as the above). Condensation with a carboxylic acid chloride R^{11a}-CO-Cl or a carboxylic acid anhydride (R^{11a}CO)₂O can be effected by using, as a base, a tertiary amine such as triethylamine, diisopropylethylamine, proton sponge, or the like, an organic base such as pyridine, dimethylaminopyridine; imidazole, or the like, or an inorganic base such as potassium carbonate, sodium carbonate, sodium hydrogencarbonate, sodium hydroxide, potassium hydroxide, or the like; as a solvent, a halogenated hydrocarbon solvent such as dichloromethane, chloroform, carbon tetrachloride, 1,2-dichloroethane, or the like, an ether solvent such as diethyl ether, THF, dioxane, or the like, pyridine, water or a solvent mixture thereof. Condensation with a carboxylic acid R^{11a}-COOH can be effected by using any one of generally known condensation agents. In condensation reaction with a carboxylic acid chloride, a carboxylic acid anhydride or a carboxylic acid, particularly in the case of compounds represented by formula (Ic) in which R² or R³, is hydroxy, the hydroxyl groups also react at the same time to obtain products. In this case, the products are hydrolyzed under basic conditions after the condensation reaction to obtain compounds represented by formula (Id) in which R² and R³ are hydroxy. The hydrolysis reaction can be effected by using an inorganic base such as potassium carbonate, sodium carbonate, sodium hydrogencarbonate, sodium hydroxide, potassium hydroxide, or the like, particularly potassium carbonate or sodium hydroxide, as a base in a solvent such as water, an alcoholic solvent such as methanol, ethanol, or the like, an ether solvent such as diethyl ether, DME, dioxane, or the like, a mixture thereof, or a solvent to which a halogenated hydrocarbon solvent such as dichloromethane, chloroform or the like is appropriately added in the case of low solubility. For example, of the compounds (Id) of the present invention, particularly compounds in which both R¹⁰ and R¹¹ are methyl can simply be produced by general methylation reaction using compounds represented by formula (Ib) as raw materials, and formaldehyde and formic acid.

Of the compounds represented by formula (II) of the present invention, compounds represented by formula (Ie) (R¹, R², R³, R⁵, m and R¹² are defined as the same as the above) in which R⁴ is OR¹² can be produced by diazotization of compounds represented by formula (Ib) of the present invention, and then solvolysis by reacting with water or an alcohol represented by R¹²OH (R¹² is defined as the same as the above), as shown in Scheme 11.

The diazotization reaction can generally be effected by using sodium nitrite in coexistence with an acid such as hydrochloric acid, sulfuric acid, phosphoric acid, or the like in a solvent such as water, acetic acid or a solvent mixture thereof, or using alkyl nitrite such as isoamyl nitrite in a solvent such as methanol, ethanol, or the like. The subsequent solvolysis can generally be carried out by heating in water or an alcoholic solvent represented by R¹²OH (R¹² is defined as the same as the above).

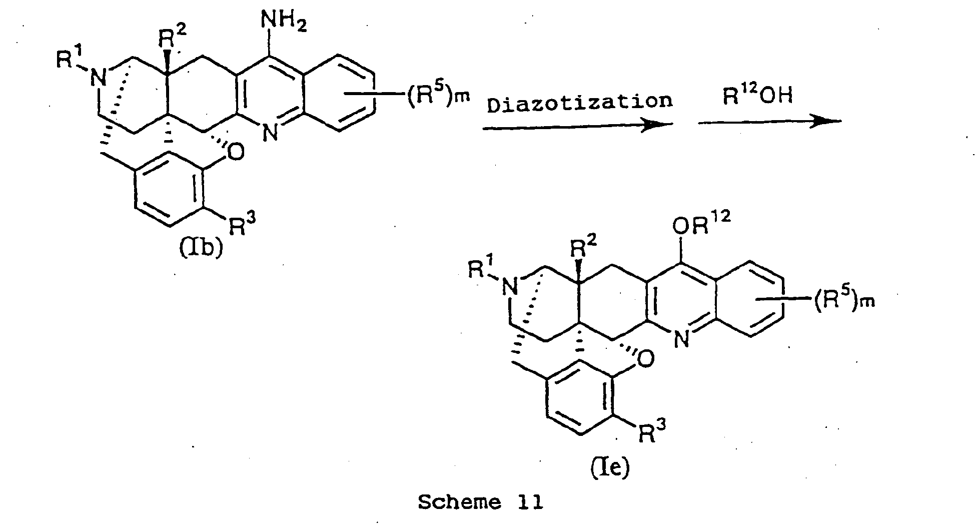

Of the compounds represented by formula (I) of the present invention, compounds represented by formula (If) (R¹, R², R³, R⁵, m and R¹⁷ are defined as the same as the above) in which R⁴ is substituent R^{4a}, comprising alkyl having 1 to 5 carbon atoms, hydroxyalkyl having 1 to 5 carbon atoms, or aryl having 6 to 12 carbon atoms (which may be substituted by at least one substituent R¹⁷) can be produced by reacting ketone compounds represented by formula (VII) and compounds represented by the formula (VIIIb) (R⁵, m and R^{4a} are defined as the same as the above) in which of the aminocarbonyl derivatives represented by the formula (VIII) shown in Scheme 1, R⁴ is R^{4a}, under acidic conditions, as shown in Scheme 12.

Examples of the solvent include alcoholic solvents such as methanol, ethanol, and the like; organic carboxylic acid solvents such as formic acid, acetic acid, propionic acid, and the like; aprotic polar solvents such as DMF, DMSO, or the like, hydrocarbon solvents such as benzene, toluene, and the like; the use of ethanol or acetic acid generally produces sufficiently satisfactory effects. Examples of the acid include a wide range of acids such as inorganic acids such as hydrochloric acid, sulfuric acid, nitric acid, phosphoric acid, and the like; organic carboxylic acids such as formic acid, acetic acid, propionic acid, and the like; organic sulfonic acids such as methanesulfonic acid, p-toluenesulfonic acid, camphorsulfonic acid, and the like, Lewis acids such as zinc chloride, phosphorus trichloride, scandium triflate, and the like. Particularly, hydrochloric acid, sulfuric acid, acetic acid, methanesulfonic acid, and scandium triflate are preferable; particularly, the use of acetic acid or methanesulfonic acid produces good results.

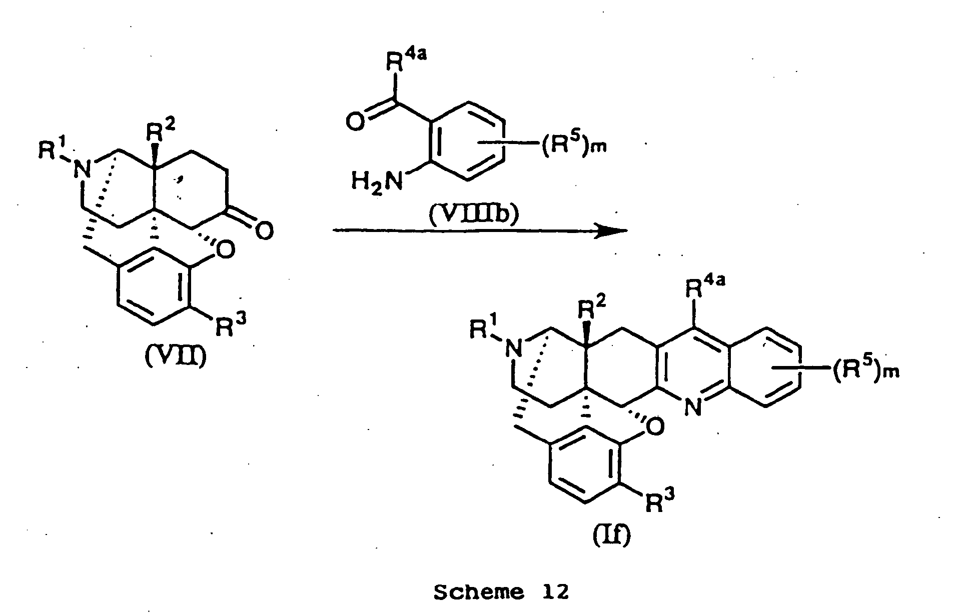

Of the compounds represented by formula (II) of the presnet invention, compounds represented by formula (Ig) (R¹, R², R³. R⁵, m and R¹³ are defined as the same as the above) in which R⁴ is COOR¹³ can be produced by quinoline synthesis reaction using ketone compounds represented by formula (VII) and aminoketo acid derivatives represented by formula (VIIIc) (R⁵, m and R¹¹ are defined as the same as the above) in which in the aminocarbonyl derivatives represented by formula (VIII) shown in Scheme 1, R⁴ is COOR¹³, as shown in Scheme 13.

Of the compounds represented by formula (Ig), compounds represented by formula (Ih) (R¹, R² , R³, R⁵ and m are defined as the same as the above) in which R¹³ is hydrogen can also, be produced by quinoline synthesis reaction using ketone compounds represented by formula (VII) and isatin derivatives represented by formula (VIIId) (R⁵ and m are defined as the same as the above), which are cyclic anhydrides of aminoketo acid derivatives (VIIIc), as shown in Scheme 14.

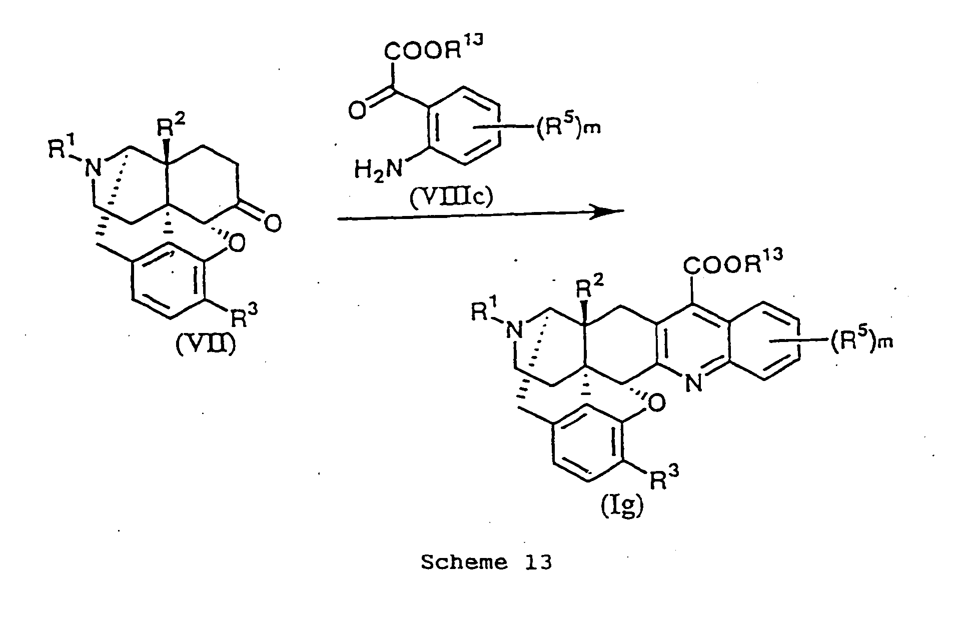

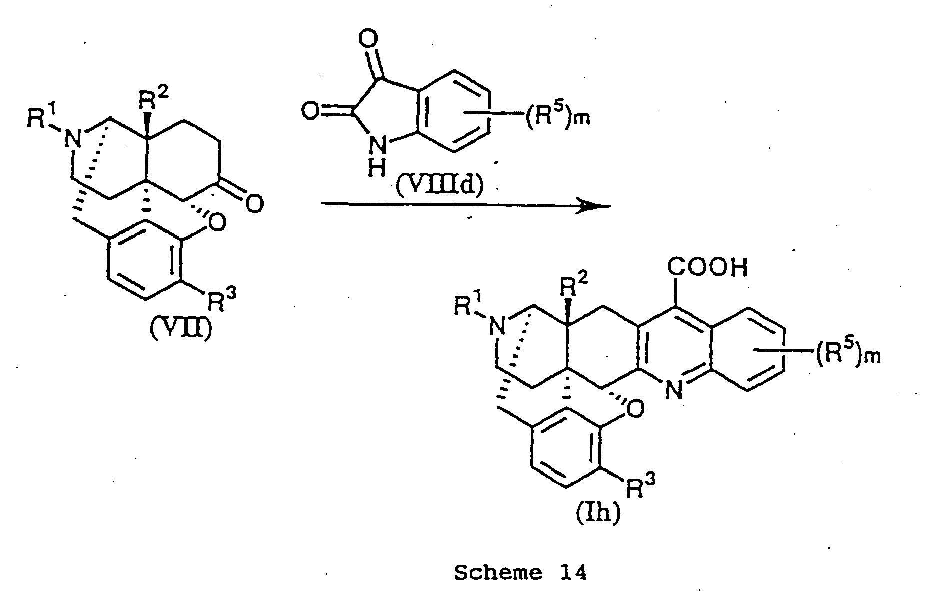

In Scheme 14, quinoline synthesis reaction can be effected in the presence of an appropriate acid or base according to demand. Examples of the base used include sodium hydroxide, potassium hydroxide, sodium alkoxide, and the like; and preferable examples of the solvent used in this case include alcoholic solvents such as methanol, ethanol, and the like; ether solvents such as diethyl ether, THF, and the like; water: and solvent mixtures thereof. Examples of the acid include inorganic acids such as hydrochloric acid, sulfuric acid, nitric acid, phosphoric acid, and the like; organic carboxylic acids such as formic acid, acetic acid, propionic acid, and the like; organic sulfonic acids such as methanesulfonic acid, p-toluenesulfonic acid, camphorsulfonic acid, and the like; Lewis acids such as zinc chloride, phosphorus trichloride, scandium triflate, and the like; particularly hydrochloric acid, sulfuric acid acetic acid are preferred. In this case, examples of the solvent used include alcoholic solvents such as methanol, ethanol, and the like; organic carboxylic acids such as formic acid, acetic acid, propionic acid, and the like; aprotic solvents such as DME and the like; water; and solvent mixtures thereof.

Of the compounds represented by formula (If) shown in Scheme 12, compounds in which R⁴ is hydroxyalkyl having 1 to 5 carbon atoms can be produced as described above: particularly, compounds represented by formula (Ii) (R¹, R², R³, R⁵ and m are defined as the same as the above) in which R⁴ is hydroxymethyl can also be produced by reduction of compounds of the present invention represented by formula (Ig), as shown in Scheme 15.

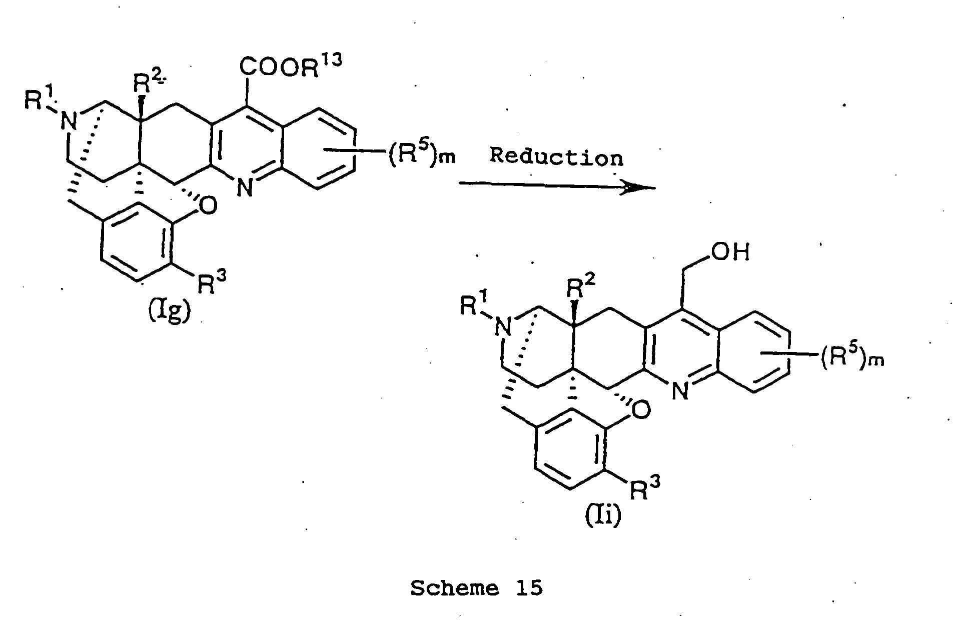

Examples of reducing agents include metal halide compounds having strong reducing power, such as lithium aluminum hydride, diisobutylaluminum hydride, lithium borohydride, diborane, a combination of an appropriate Lewis acid and sodium borohydride, and the like; particularly aluminum lithium hydride, lithium borohydride, and diborane are preferable. In the use of lithium aluminum hydride, lithium borohydride, or diborane, ether solvents such as THF, diethyl ether, DME, dioxane, and the like are preferably used as the solvent; particularly THF is preferably used. In the case of diisobutylaluminum hydride, hydrocarbon solvents such as benzene, toluene, and the like are preferably used. In the case of sodium borohydride, alcohollic solvents such as methaonl, ethanol, and the like are preferably used.

Of the compounds represented by formula (Ig) of the present invention shown in Scheme 13, compounds represented by formula (Ig') (R¹, R², R⁴, R⁵ and m are defined as the same as the above) in which R¹³ is R^{13a} which is alkyl having 1 to 5 carbon atoms, or aralkyl having 7 to 13 carbon atoms can be produced by the method shown in Scheme 13; such compounds can also be produced by another method comprising esterifying the compounds represented by formula (Ih) of the present invention, as shown in Scheme 16.

Examples of the esterification method using compounds represented by formula (Ih) of the present invention include (1) a method of reacting with an alcohol represented by R^{13a}OH (R^{13a} is defined as the same as the above) in the presence of an appropriate acid, (2) a method of reacting with thionyl chloride or oxalyl chloride to convert the compounds to acid chlorides represented by formula (XIII) (R¹, R², R³, R⁵ and m are defined as the same as the above), and then reacting with an alcohol represented by R^{13a}OH (R^{13a} is defined as the same as the above) in the presence of an appropriate base according to demand, and a method of reacting with diazomethane or trimethylsilyldiazomethane to obtain methyl esters, particularly when R^{13a} is methyl; the esterification methods are not limited to these methods.

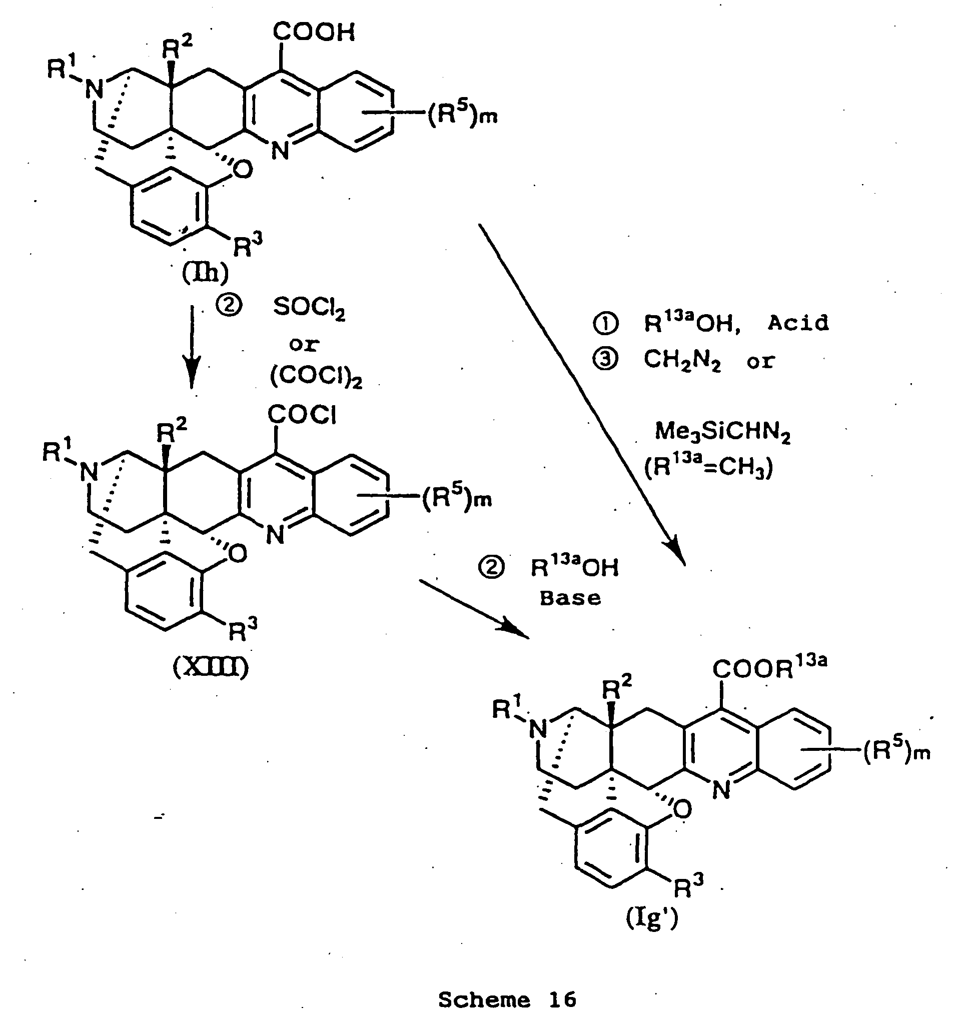

Of the compounds represented by formula (II) of the present invention, compounds represented by formula (II) (R¹, R², R³ , R⁵, m R¹⁴ and R¹⁵ are defined as the same as the above) in which R⁴ is CONR¹⁴R¹⁵ can be produced by amidation of compounds represented by formula (Ih) of the present invention, as shown in Scheme 17.

Examples of the amidation method using the compounds represented by formula (Ih) include (1) a method of condensation using an amine represented by R¹⁴R¹⁵NH (R¹⁴ and R¹⁵ are defined as the same as the above) and an appropriate condensation agent according to demand, and (2) a method of reacting with thionyl chloride or oxalyl chloride to convert to acid chlorides represented by formula (XIII), and then condensation with an amine represented by R¹⁴R¹⁵NH (R¹⁴ and R¹⁵ are defined as the same as the above) in the presence of an appropriate base according to demand; the amidation method is not limited to these methods.

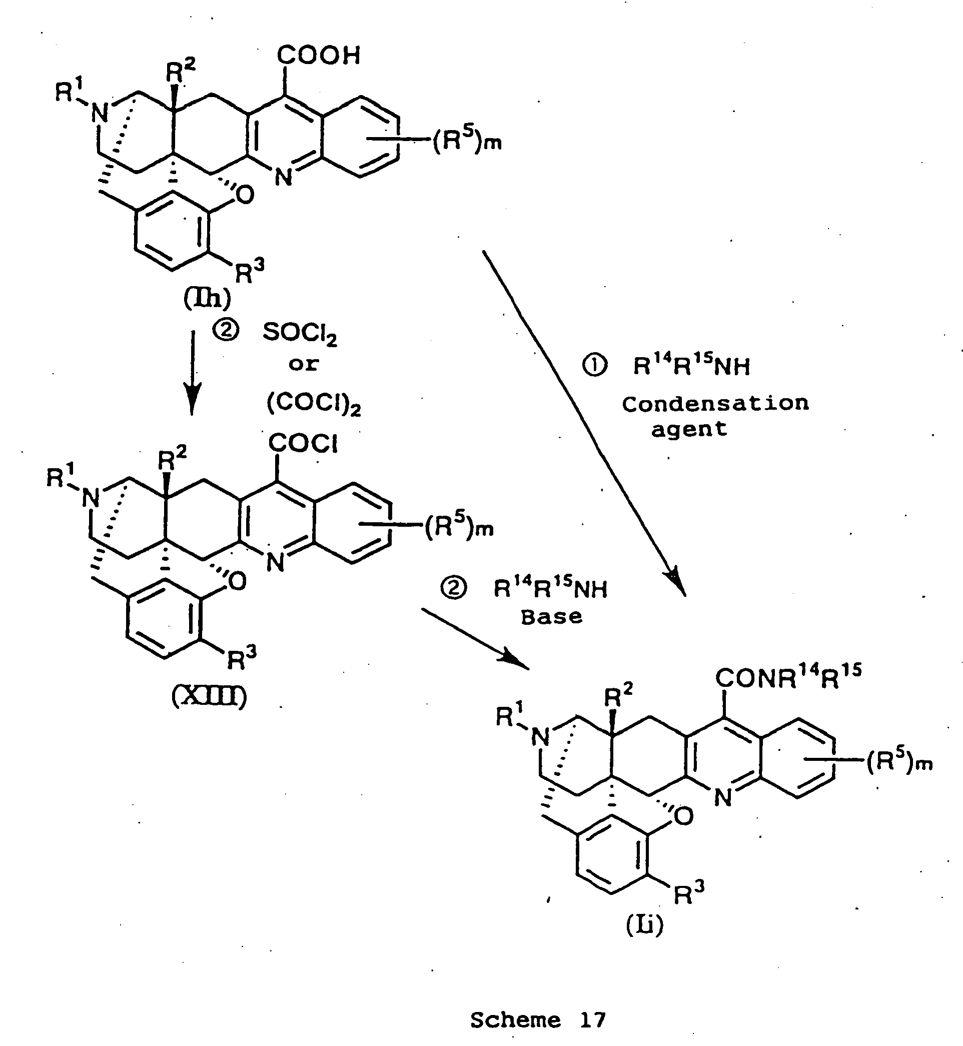

Of the compounds represented by formula (II) of the present invention, compounds represented by formula (Ij) (R¹. R², R³ and R⁵ are defined as the same as the above) in which R⁴ and R⁵ substituted at the peri-position form together R⁴-R⁵, NR¹⁶CO, wherein R¹⁶ is hydrogen, can be produced by quinoline synthesis reaction using ketone compounds represented by formula (VII) and aminophthalonitrile derivatives represented by formula (IXa) (R⁵ is defined as the same as the above) in the presence of an appropriate acid according to demand, as shown in Scheme 18.

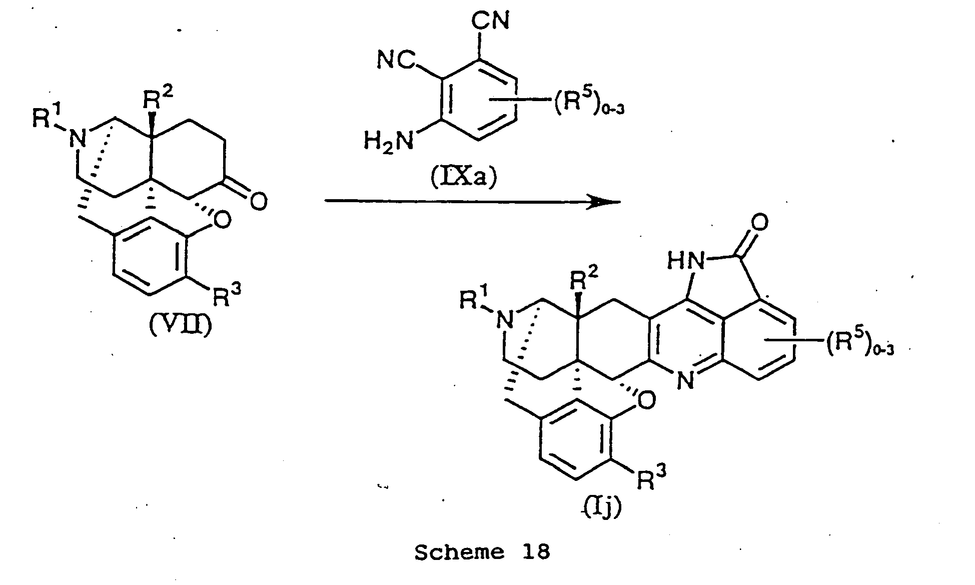

Examples of the solvent include alcoholic solvents such as methanol, ethanol, and the like; organic carboxylic acid solvents such as formic acid, acetic acid, propionic acid, and the like; hydrocarbon solvents such as benzene, toluene, and the like; aprotic polar solvents such as DMF, DMSO; and the like; and solvent mixtures thereof; particularly ethanol, acetic acid, toluene, and a DMF-benzene mixed solvent are preferable. As reaction conditions, normal heating conditions, azeotropic conditions, heating concentration conditions, and the like are preferably used. Examples of the acid include inorganic acids such as hydrochloric acid, sulfuric acid, nitric acid, phosphoric acid, and the like; organic carboxylic acids such as formic acid, acetic acid, propionic acid, and the like; organic sulfonic acids such as methanesulfonic acid, p-toluenesulfonic acid, camphorsulfonic acid, and the like; Lewis acids such as zinc chloride, zinc trichloride, phosphorus trichloride, scandium triflate, and the like. Particularly, acetic acid which is also used as a solvent, methanesulfonic acid, and scandium triflate are preferable.

At the same time, compounds represented by formula (Io) below are sometimes obtained. Particularly, when a DMF-benzene mixed solvent is used as the solvent, and methanesulfonic acid is used as the acid, the compounds represented by formula (Io) can be produced in high yield.

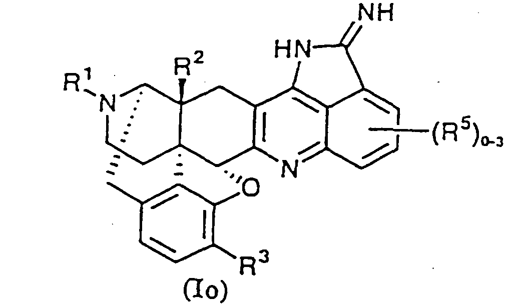

Of the compounds represented by formula (II) of the present invention, compounds represented by formula (Ip) (R¹, R², R³ and R⁵ are defined as the same as the above) in which R⁴ and R⁵ substituted at the peri-position form together R⁴-R⁵, NR¹⁶CO, wherein R¹⁶ is alkyl having 1 to 5 carbon atoms, cycloalkylalkyl having 4 to 7 carbon atoms, aralkyl having 7 to 13 carbon atoms, or alkanoyl having 1 to 5 carbon atoms, can be produced by alkylation or acylation of compounds represented by formula (Ij) according to a general method, as shown in Scheme 18-2.

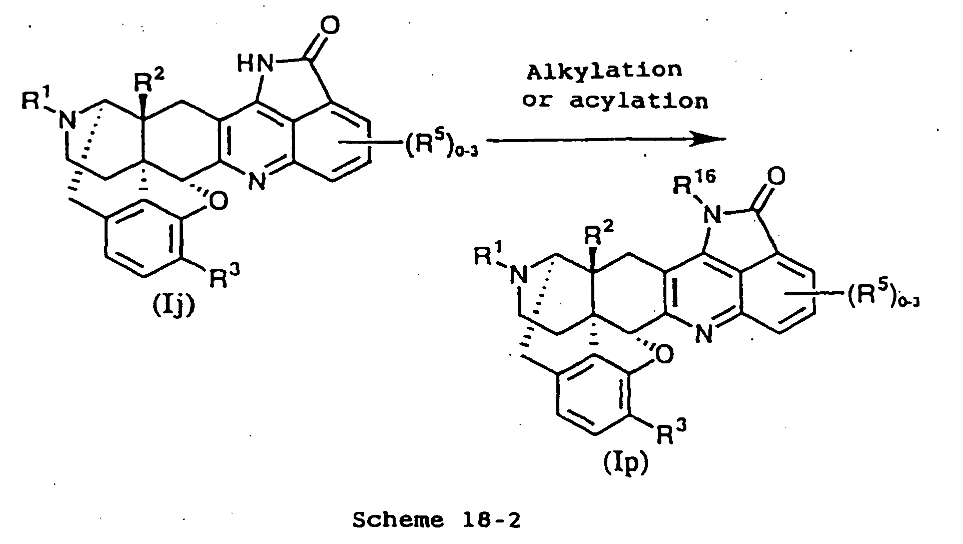

As described above, general methods of converting amino groups include (1) a method of alkylation using a halide R¹⁶-x¹ (X¹ is defined as the same as the above) in the presence of an appropriate base, and (2) a method of acylation using an appropriate acid chloride R^{16b}-CO-Cl (R^{16b} represents a group obtained by removing one terminal carbonyl from R¹⁶) according to a general method.

Of the compounds represented by formula (II) of the present invention, compounds represented by formula (Ik) (R¹, R², R³, R⁵ and R⁷ are defined as the same as the above) in which R⁴ is NR¹⁰R¹¹, both R¹⁰ and R¹¹ are hydrogen, R⁵ is substituted at the peri-position of R⁴ and is COOR⁷, can be produced by solvolysis of the compounds represented by formula (Ij) of the present invention under generally used conditions such as acidic or basic conditions in water or an alcohol represented by R⁷OH (R⁷ is defined as the same as the above), as shown in Scheme 19.

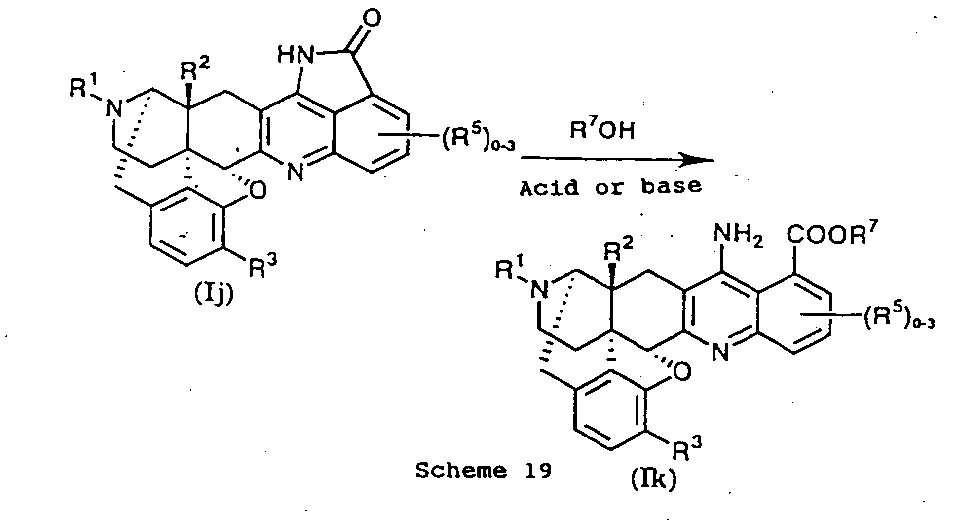

Examples of the acid include inorganic acids such as hydrochloric acid, sulfuric acid, nitric acid, phosphoric acid, and the like; the use of hydrochloric acid generally produces good results. Examples of the base include inorganic bases such as potassium carbonate, sodium carbonate, sodium hydrogencarbonate, sodium hydroxide, potassium hydroxide, and the like; the use of sodium hydroxide generally produces good results.

Of the compounds represented by formula (II) of the present invention, compounds represented by formula (Im) (R¹, R², R⁴, R⁵ and m are defined as the same as the above) in which R³ is methoxy can be converted to compounds represented by formula (In) (R¹, R², R⁴**,** R⁵ and m are defined as the same as the above) in which R³ is hydroxy, by general demethylation reaction of phenolic methyl ether, as shown in Scheme 20.

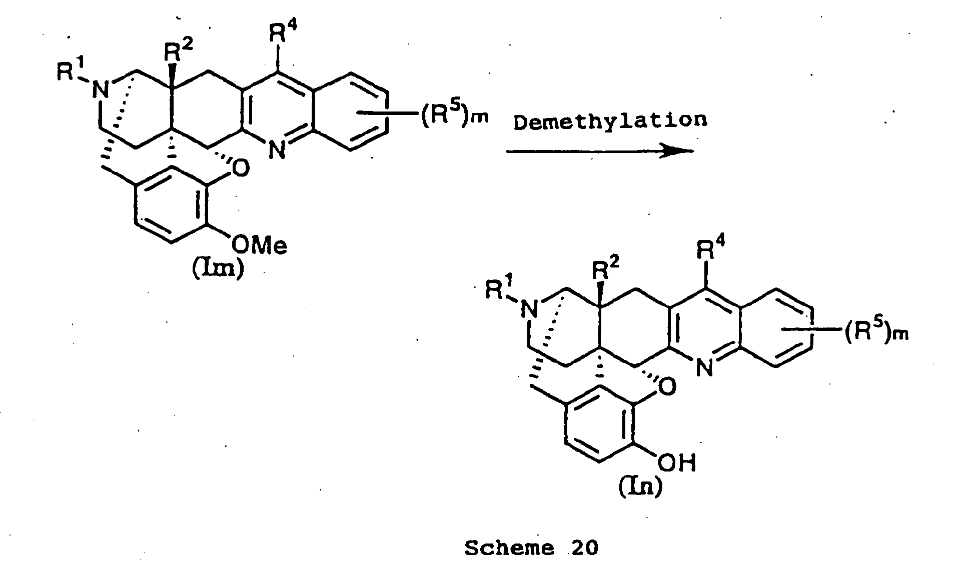

The demethylation reaction can be effected under generally known conditions: particularly the reaction can be effected by a method using dichloromethane. chloroform or 1,2-dichloroethane as a solvent, and boron tribromide [Document: Tetrahedron Vol. 24, 2289 (1968)] or boron trichloride, or a method using a thioalkoside, particularly sodium thioethoxide [Document: Tetrahedron Lett., 1327 (1970)] in DMF generally. The demethylation reaction also permits demethylation of a methoxy group which is present as a substituent other than R³ in the compounds represented by formula (II) of the present invention.

Conversely, of the compounds represented by formula (II) of the present invention, compounds represented by formula (Im) (R¹, R², R⁴ , R⁵ and m are defined as the same as the above) in which R³ is methoxy can also be produced by methylation of the phenolic hydroxyl group in compounds represented by formula (In) (R¹, R², R⁴, R⁵ and m are defined as the same.as the above) in which R³ is hydroxy, according to a general method, as shown in Scheme 21.

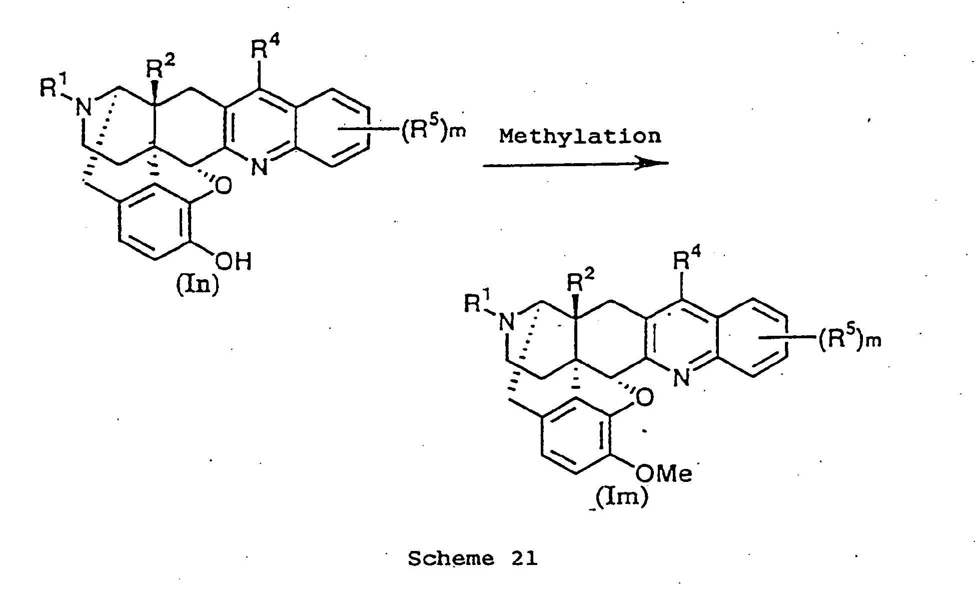

The methylation reaction can be effected under generally known conditions; particularly the reaction can preferably be effected by a method using methyl iodide in coexistence with an inorganic base such as sodium carbonate, potassium carbonate, lithium carbonate, sodium hydroxide, potassium hydroxide, or the like, in a solvent such as DMF, acetone, or the like, or in the presence of sodium hydride in an ether solvent such as THF or the like, or a method using diazomethane in coexistence with silica gel in a solvent such as diethyl ether or the like.

As a result of in vivo pharmacological evaluation, the quinolinomorphinan derivatives of the present invention represented by formula (I) exhibit excellent effects on disorders of the cerebral nerve cells, as described in the examples below. Therefore, the compounds of the present invention can be used as agents for curing·preventing cerebral disorders, i.e., medicines useful for ameliorating various cerebral diseases and aftereffects thereof, and preventing the recurrence thereof. Specifically, it was made apparent that the compounds of the present invention can be used as therapeutic agents for cerebral stroke, therapeutic agents for traumatic cerebral diseases, therapeutic agents for cerebral edema, therapeutic agents for ischemic diseases, therapeutic agents for cerebral neurodegenerative diseases, and therapeutic agents for aftereffects of cerebral diseases. The compounds of the present invention exhibited the excellent neuroprotetive action on damages of the cerebral nerve cells, and it was thus found that the compounds of the present invention are useful as cerebral neuroprotective agents which inhibit ischemic or hemorrhagic cerebrovascular diseases, traumatic cerebral diseases and various cerebral neurodegenerative diseases by the protecting action on the cerebral nerve cells.

The therapeutic agents for cerebral stroke are medicines used for curing, ameliorating or preventing ischemic or hemorrhagic cerebral stroke, specifically, cerebral infarction (cerebral embolism, cerebral thrombosis), cerebral hemorrhage, subarachnoid hemorrhage, transient ischemic attack (TIA), hypertensive encephalophathy, etc. The therapeutic agents for traumatic cerebral diseases are medicines used for ameliorating cerebral disorder caused by trauma and functional disorder of the brain accompanied thereby, and ameliorating aftereffects. The therapeutic agents for cerebral edema are medicines used for ameliorating, curing or preventing cerebral edema caused by a lesion of hemorrhage, infarction, tumor, trauma, or the like which occurs in the brain, or an increase in intracranial pressure to ameliorate disorders of the cerebral nerve cells due to cerebral edema. The therapeutic agents for ischemic diseases are medicines used for curing, ameliorating or preventing the cerebral disorders caused by insufficient supply of oxygen and glucose to the cerebral nerve cells on the basis of ischemia due to hypoxia, hypoglycemia, drug poisoning, or the like. The therapeutic agents for cerebral neurodegenerative diseases are medicines used for curing, ameliorating or preventing cerebral diseases such as Alzheimer's disease, Parkinson's disease, Huntington's chorea, diffuse Lewy's bodies, Creutzfeldt-Jakob's disease, and the like, which cause disorders of the cerebral nerve cells accompanied with degeneration of the nerve cells. The therapeutic agents for aftereffects of cerebral diseases are medicines used for curing, ameliorating or preventing aftereffects caused by the above cerebral disorders, such as cerebrovascular dementia, amnesia, disorder of consciousness, motor paralysis, allophasis, sensory disorder, mental disorder, memory disorder, and the like.

In the clinical use of the agent for curing·preventing cerebral disorder of the present invention, a free base or salt thereof may be used, and additives such as an excipient, a stabilizer, a preservative, a buffer, a solubilizer, an emulsifier, a diluent, an isotonizing agent, etc. may be appropriately mixed. As an administration form, either parenteral administration or oral administration produces sufficient effects. Administration formulations include an injection, a tablet, a liquid, a capsule, granules, a powder, and the like, and these formulations can be produced by known formulation techniques. Although the dosage is appropriately selected in accordance with the symptoms, age and body weight of a patient, the administration method, etc., the amount of the effective component per adult is 0.0001 mg to 10 g per day, preferably 0.001 mg to 1 g per day, and the agent can be administered once or divided into several doses.

### [Examples]

Although the present invention is described in detail below with reference to reference examples and examples, the present invention is not limited to these example.

### [Reference Example 1]

### 17-cyclopropylmethyl- 6,7-dehydro-4,5α-epoxy-3,14β-dihydroxy-6,7,2',3'-quinolinomorphinan 1 methanesulfonate

This compound was synthesized in accordance with the method disclosed in U.S. Patent No. 4,816,586. 4.69 g (12.4 mmol) of 17-cyclopropylmethyl-6-oxo-4,5α-epoxy-3,14β-dihydroxymorphinan hydrochloride, and 3.0 g of o-aminobenzaldehyde were added to 100 ml of ethanol, and 0.81 ml (12.4 mmol) of methanesulfonic acid was added to the resultant mixture, followed by heating under reflux for 2.5 hours. The reaction solution was concentrated under reduced pressure, and an aqueous saturated sodium hydrogencarbonate solution to the resultant residue, followed by extraction with ethyl acetate. The organic layers were together dried over anhydrous sodium sulfate, and then concentrated. Methanol was added to the residue to obtain 4.12 g (yield 78 %) of free title compound as crystals. The thus-obtained compound was dissolved in methanol, and methanesulfonic acid was added to the resultant solution to isolate a salt of the compound.

### [Reference Example 2]

### 17-cyclopropylmethyl-6,7-dehydro-4,5α-epoxy-14p-hydroxy-3-methoxy-6,7,2',3'-(7',8'-benzoquinolino)morphinan 2 • methanesulfonate

3.18 g (7.75 mmol) of 17-cyclopropylmethyl-6-oxo-4,5α-epoxy-14β-hydroxy-3-methoxy-7-[(dimethylamino)methylene] morphinan produced in accordance with the method disclosed in the document [J. Med. Chem., 24, 1445 (1981)] was dissolved in 15 ml of trifluoroacetic acid, and 5.57 g (31. 0 mmol) of α-naphthylamine hydrochloride was added to the resultant solution, followed by stirring under heating at 120°C. After 3 ml of trifluoroacetic acid was distilled off, the residue was heated under reflux at 120°C for 23 hours. After the reaction solution was concentrated, an aqueous saturated sodium hydrogencarbonate solution was added to the residue, followed by extraction with chloroform. The organic layers were together dried over anhydrous sodium sulfate, and then concentrated. The obtained crude product was purified by medium pressure silica gel column chromatography, and then recrystallized from chloroform-methanol to obtain 3.03 g (80 %) of salt-free title compound. The thus-obtained compound was dissolved in methanol-THF, and methanesulfonic acid was added to the resultant solution to isolate a salt of the compound.

### [Reference Examples 3 - 7]

In accordance with the method of Reference Example 2, 4-methoxyaniline, 2-phenylaniline, 1-amino-5,6,7,8-tetrahydronaphthalene, 4-methylaniline, and 4-(dimethylamino)aniline hydrochloride were used in place of α-naphthylamine hydrochloride to obtain 17-cyclopropylmethyl-6,7-dehydro-4,5a-epoxy-14p-hydroxy-3-methoxy-6,7,2',3'-(6'-methoxyquinolino)morphinan 3, 17-cyclopropylmethyl-6,7-dehydro-4,5α-epoxy-14β-hydroxy-3-methoxy-6,7,2',3'-(8'-phenylquinolino)morphinan 4, 17-cyclopropylmethyl-6,7-dehydro-4,5a-epoxy-14β-hydroxy-3-methoxy-6,7,2',3'-(7',8'-cyclohexenoquinolino)morphinan 5, 17-cyclopropylmethyl-6,7-dehydro-4,5α-epoxy-14β-hydroxy-3-methoxy-6,7,2',3'-(6'-methylquinolino)morphinan 6, and 17-cyclopropylmethyl-6,7-dehydro-4,5α-epoxy-14β-hydroxy-3-methoxyy-6,7,2',3'-[6'-(dimethylamino)quinolino]morphinan 7, respectively.

### [Examples 1 - 2]

In accordance with the method of Reference Example 2, 5-aminoacenaphthene hydrochloride and 1-aminofluorene were used in place of α-naphthylamine hydrochloride to obtain 17-cyclopropylmethyl-6,7-dehydro-4,5α-epoxy-14β-hydroxy-3-methoxy-6,7,2',3'-(6',6"-ethano-7',8'-benzoquinolino)morphinan 8 and 17-cyclopropylmethyl-6,7-dehydro-4,5α-epoxy-14β-hydroxy-3-methoxy-6,7,2',3'-(7',8',3",2"-indenoquinolino)morphinan 9, respectively.

### [Example 3]

### 17-cyclopropylmethyl-6,7-dehydro-4,5a-epoxy-3,14p-dihydroxy-6,7,2',3'-(4'-aminoquinolino)morphinan 10 hydrochloride

1.25 g (3.66 mmol) of 17-cyclopropylmethyl-6-oxo-4,5a-epoxy-3,14β-dihydroxymorphinan, and 0.50 g (4.39 mmol) of o-aminobenzonitrile were added to 10 ml of acetic acid, and the resultant mixture was heated under reflux for 25 hours. The reaction solution was concentrated under reduced pressure, and an aqueous saturated sodium hydrogencarbonate solution was added the resultant residue, followed by extraction with ethyl acetate. The organic layers were together dried over anhydrous sodium sulfate and then concentrated, and the resultant crude product was purified by medium pressure silica gel column chromatography (chloroform : methanol = 100:1 → 20:1) to obtain 0.59 g (37 %) of free title compound. The thus-obtained compound was suspended in methanol, and hydrochloric acid was added to the suspension to isolate a salt of the compound.

### [Examples 4 - 14]

In accordance with the method of Example 3, 2-amino-4-chlorobenzonitrile, 2-amino-4-methylbenzonitrile, 2-amino-3-chlorobenzonitrile, 2-amino-4,5-dimethoxybenzonitrile, 2-amino-3-fluorobenzonitrile, 2-amino-5-nitrobenzonitrile, 2-amino-5-chlorobenzonitrile (prepared from 2-nitro-5-chlorobenzonitrile), 2-amino-4-trifluoromethylbenzonitrile (prepared from 2-nitro-4-trifluoromethylbenzonitrile), 1-amino-2-naphthonitrile (prepared from α-naphthylamine), 2-amino-5-methylbenzonitrile (prepared from 4-methylaniline), and 2-amino-5-methoxybenzonitrile (prepared from 4-methoxyaniline) were used in place of o-aminobenzonitrile to obtain 17-cyclopropylmethyl-6,7-dehydro-4,5α-epoxy-3,14β-dihydroxy-6,7,2',3'-(4'-amino-7'-chloroquinolino)morphinan 11, 17-cyclopropylmethyl-6,7-dehydro-4,5α-epoxy-3,14β-dihydroxy-6,7,2',3'-(4'-amino-7'-methylquinolino)morphinan 12, 17-cyclopropylmethyl-6,7-dehydro-4,5α-epoxy-3,14β-dihydroxy-6,7,2',3'-(4'-amino-8'-chloroquinolino)morphinan 13, 17-cyclopropylmethyl-6,7-dehydro-4,5α-epoxy-3,14β dihydroxy-6,7,2',3'-(4'-amino-6',7'-dimethoxyquinolino)morphinan 14, 17-cyclopropylmethyl-6,7-dehydro-4,5α-epoxy-3,14β-dihydroxy-6,7,2',3'-(4'-amino-8'-fluoroquinolino)morphinan 15, 17-cyclopropylmethyl-6,7-dehydro-4,5α-epoxy-3,14β-dihydroxy-6,7,2',3'-(4'-amino-6'-nitroquinolino)morphinan 16, 17-cyclopropylmethyl-6,7-dehydro-4,5α-epoxy-3,14β-dihydroxy-6,7,2',3'-(4'-amino-6'-chloroquinolino)morphinan 17, 17-cyclopropylmethyl-6,7-dehydro-4,5α-epoxy-3,14β-dihydroxy-6,7,2',3'-(4'-amino-7'-trifluoromethylquinolino)morphinan 18, 17-cyclopropylmethyl-6,7-dehydro-4,5α-epoxy-3,14β-dihydroxy-6,7,2',3'-(4'-amino-7',8'-benzoquinolino)morphinan 19, 17-cyclopropylmethyl-6,7-dehydro-4,5α-epoxy-3,14β-dihydroxy-6,7,2',3'(4-amino-6'-methylquinolino)morphinan 20, and 17-cyclopropylmethyl-6,7-dehydro-4,5α-epoxy-3,14β-dihydroxy-6,7,2',3'-(4'-amino-6'-methoxyquinolino)morphinan 21, respectively.

### [Examples 15 - 17]

In accordance with the method of Example 3, 17-cyclopropylmethyl-6-oxo-4,5α-epoxy-14β-hydroxy-3-methoxymorphinan, 17-methyl-6-oxo-4,5α-epoxy-14β-hydroxy-3-methoxymorphinan, and 6-oxo-4,5α-epoxy-14β-hydroxy-3-methoxymorphinan were used in place of 17-cyclopropylmethyl-6-oxo-4,5α-epoxy-3,14β-dihydroxymorphinan to obtain 17-cyclopropylmethyl-6,7-dehydro-4,5α-epoxy-14β-hydroxy-3-methoxy-6,7,2',3'-(4.'-aminoquinolino)morphinan 22, 17 methyl-6,7-dehydro-4,5n-epoxy-14p-hydroxy-3-methoxy-6,7,2',3'-(4'-aminoquinolino)morphinan 23, and 17-acetyl-6,7-dehydro-4,5α-epoxy-14β-hydroxy-3-methoxy-6,7,2',3'-(4'-aminoquinolino)morphinan 24, respectively.

### [Example 18]

### 17-cyclopropylmethyl-6,7-dehydro-4,5α-epoxy-3,14β-dihydroxy-6,7,2',3'-[4'-(acetylamino)quinolino]morphinan 25 hydrochloride

232 mg (0.53 mmol) of 17-cyclopropylmethyl-6,7-dehydro-4,5α-epoxy-3,14β-dihydroxy-6,7,2',3'-(4'-aminoquinolino)morphinan 10 was added to 5 ml of acetic anhydride, followed by heating under reflux for 20 hours. The reaction solution was concentrated, and then 5 ml of methanol and 2 ml of 3N aqueous sodium hydroxide solution were added to the residue, followed by stirring for 20 hours. The reaction solution was concentrated, and then 50 ml of aqueous saturated sodium hydrogencarbonate solution was added to the residue, followed by extraction with ethyl acetate (50 ml x 3). The organic layers were together dried over anhydrous sodium sulfate, and then concentrated, and the resultant crude product was purified by medium pressure silica gel column chromatography (chloroform - chloroform : methanol = 20 : 1) to obtain 136 mg (54 %) of free title compound. The thus-obtained compound was isolated as a hydrochloride.

### [Example 19]

### 17-cyclopropylmethyl-6,7-dehydro-4,5α-epoxy-3,14β-dihydroxy-6,7,2',3'-(4'-methylquinolino)morphinan 26 methanesulfonate

391 mg (1.04 mmol) of 17-cyclopropylmethyl-6-oxo-4,5α-epoxy-3,14β-d-ihydroxymorphinan was suspended in 6 ml of acetic acid, and 0.138 ml (1.14 mmol) of 2'-aminoacetophenone was added to the resultant suspension, followed by heating to form a solution. After heating under reflux for 4 hours, the reaction solution was allowed to cool to room temperature, and then concentrated, and 8 ml of a 2N aqueous sodium hydroxide solution was added to the residue to make the solution basic, followed by extraction with chloroform. The organic layers were together dried over anhydrous sodium sulfate, and then concentrated, and the resultant crude product was purified by silica gel column chromatography (chloroform : methanol = 50:1 → 30: 1) to obtain 470 mg of free title compound. The thus-obtained compound was dissolved in methanol, and methanesulfonic acid was added to the solution to isolate 499 mg (90 %) of title compound as a salt.

### [Examples 20 - 22]

In accordance with the method of Example 19, 2-aminobenzophenone, 2'-amino-4',5'-dimethoxyacetophenone, and 6'-amino-3',4'-methylenedioxyacetophenone were used in place, of 2'-aminoacetophenone to obtain 17-cyclopropylmethyl-6,7-dehydro-4,5α-epoxy-3,14β-dihydroxy-6,7,2',3'-(4'-phenylquinolino)morphinan 27, 17-cyclopropylmethyl-6,7-dehydro-4,5α-epoxy-3,14β-dihydroxy-6,7,2',3'-(4'-methyl-6',7'-dimethoxyquinolino)morphinan 28, and 17-cyclopropylmethyl-6,7-dehydro-4,5α-epoxy-3,14β-dihydroxy-6,'7,2',3'-(4'-methy-6',7'-dioxolenoquinolino)morphinan 29, respectively.

### [Example 23]

### 17-cyclopropylmethyl-6,7-dehydro-4,5α-epoxy-3,140-dihydroxy-6,7,2',3'-(4'-carboxyquinolino)morphinan 30 hydrochloride

511 mg (1.50 mmol) of 17-cyclopropylmethyl-6-oxo-4,5α-epoxy-3,14β-dihydroxymorphinan and 220 mg (1.50 mmol) of isatin were added to 5 ml of acetic acid, and the resultant mixture was stirred at 80°C for 1.5 hours. To the mixture was added 1 ml of conc. hydrochloric acid, and the mixture was stirred at 110°C for hours. To the mixture was further added 1 ml of conc. hydrochloric acid, followed by heating under reflux at 130°C for 24 hours. The reaction solution was concentrated under reduced pressure, and chloroform was added to the resultant residue, followed by extraction with water. The aqueous layers were combined and then concentrated, and the resultant crude product was purified by a Sephadex column (methanol) to obtain 494 mg (65 %) of title compound.

### [Example 24]

In accordance with the method of Example 23, 17-cyclopropylmethyl-6-oxo-4, 5α-epoxy-14β-hydroxy-3-methoxymorphinan was used in place of 17-cyclopropylmethyl-6-oxo-4,5α-epoxy-3,14β-dihydroxymorphinan to obtain 17-cyclopropylmethyl-6,7-dehydro-4,5α-epoxy-14β-hydroxy-3-methoxy-6,7,2',3'-(4'-carboxyquinolino)morphinan 31.

### [Example 25]

### 17-cyclopropylmethyl-6,7-dehydro-4,5α-epoxy-3,14β-dihydroxy-6,7,2',3'-(4'-methoxycarbonylquinolino)morphinan 32 • methanesulfonate

203 mg (0.43 mmol) of 17-cyclopropylmethyl-6,7-dehydro-4,5α-epoxy-3,14β-dihydroxy-6,7,2',3'-(4'-carboxyquinolino)morphinan 30 hydrochloride was dissolved in a mixed solvent containing 1 ml of methanol and 4 ml of benzene, and 2.0 ml (1.4 mmol) of trimethylsilyldiazomethane (10% hexane solution) was added dropwise to the resultant solution, followed by stirring at room temperature for 1 hour. To the reaction solution was added water, and the solution was extracted with chloroform. The organic layers were together dried over anhydrous sodium sulfate, and the concentrated, and the resultant crude product was purified by medium pressure silica gel column chromatography (chloroform → chloroform : methanol = 100:1) to obtain 169 mg (81 %) of free title compound. The thus-obtained compound was dissolved in methanol, and methanesulfonic acid was added to the solution to isolate a salt of the compound.

### [Example 26]

### 17-cyclopropylmethyl-6,7-dehydro-4,5α-epoxy-3,14β-dihydroxy-6,7,2',3'-[4'-(hydroxymethyl)quinolino]morphinan 33 • methanesulfonate

100 mg (0.21 mmol) of 17-cyclopropylmethyl-6,7-dehydro-4,5α-epoxy-3,14β-dihydroxy-6,7,2',3'-(4'-methoxycarbonylquinolino)morphinan 32 was suspended in 5 ml of THF, and 28 mg (1.29 mmol) of lithium borohydride was added to the resultant suspension, followed by heating under reflux for 22 hours. To the reaction solution was added 10 ml of a 0.1N aqueous hydrochloric acid solution, and the resultant mixture was stirred for 10 minutes. To the mixture was added an aqueous saturated sodium hydrogencarbonate solution, and the mixture was extracted with chloroform. The organic layers were together dried over anhydrous sodium sulfate, and then concentrated, and the resultant crude product was purified by medium pressure silica gel column chromatography (chloroform → chloroform : methanol = 20:1) to obtain 45 mg (48 %) of free title compound. The thus-obtained compound was dissolved in methanol, and methanesulfonic acid was added to the resulatnat solution to isolate a salt of the compound.

### [Example 27]

### 17-cyclopropylmethyl-6,7-dehydro-4,5α-epoxy-3,14β-dihydroxy-6,7,2',3'-[4',5'-[imino(oxomethano)quinolino]morphinan 34 hydrochloride

2.4 g of 17-cyclopropylmethyl-6-oxo-4,5α-epoxy-3,14β-dihydroxymorphinan was dissolved in acetic acid, and 1.2 g of 3-aminophthalonitrile (prepared by 3-nitrophthalonitrile) and 350 mg of scandium triflate were added to the resultant solution, followed by heating under reflux for 90 hours. After the reaction solution was cooled, the solvent was distilled off under reduced pressure, and 150 ml of an aqueous saturated sodium hydrogencarbonate solution was added to the residue, followed by extraction with ethyl acetate. The organic layers were together dried over anhydrous sodium sulfate and then concentrated, and the resultant crude product was purified by medium pressure silica gel column chromatography (chloroform : methanol = 50:1 → 20:1) to obtain 650 mg (20 %) of free title compound. The thus-obtained compound was isolated as a hydrochloride.

### [Example 28]

### 17-cyclopropylmethyl-6,7-dehydro-4,5α-epoxy-3,14β-dihydroxy-6,7,2',3'-(4'-amino-5'-carboxyquinolino)morphinan 35 hydrochloride

240 mg (0.48 mmol) of 17-cyclopropylmethyl-6,7-dehydro-**4,5α-epoxy-3,14β-dihydroxy-6,7,2',3'-[4',5'-**[imino(oxomethano)]quinolino]morphinan 34 hydrochloride was dissolved in 60 ml of water, and the resultant solution was sealed in an ampoule and then allowed to stand in a constant-temperature bath at 60°C for 5 days. After the ampoule was cooled, the reaction solution was taken out, and washed with 50 ml of ammonia-saturated chloroform and then with chloroform (50 ml x 2), followed by freeze drying to obtain 220 mg of crude product. The thus-obtained crude product was purified by a Sephadex column (methanol), freeze-dried to obtain 53 mg (21 %) of title compound.

### [Reference Example 8]

### 17-cyclopropylmethyl-6,7-dehydro-4,5α-epoxy-3,14β-dihydroxy-6,7,2',3'-(7',8'-benzoquinolino)morphinan 36 methanesulfonate

3.03 g (6.18 mmol) of 17-cyclopropylmethyl-6,7-dehydro-4,5α-epoxy-14β-hydroxy-3-methoxy-6,7,2',31-(7',8'-benzoquinolino)morphinan 2 was dissolved in 60 ml of anhydrous methylene chloride, and the resultant solution was cooled to 00°C. To the solution was added dropwise 18.5 ml (18.5 mmol) of a 1.0M solution of boron tribromide in methylene chloride, followed by stirring at 0°C for 0.5 hour. To the reaction solution were added 60 ml of water and 40 ml of aqueous ammonia solution, and the resultant mixture was stirred at room temperature for 1 hour and then extracted with chloroform (100 ml x 2). The organic layers were together dried over anhydrous sodium sulfate and then concentrated, and the resultant crude product was purified by medium silica gel column chromatography (chloroform) and then recrystallized from chloroform (2 ml)-methanol (10 ml) to obtain 2.04 g (69 %) of free title compound. The thus-obtained compound was isolated as a methanesulfonate.

### [Reference Examples 9 - 13]

In accordance with the method of Reference Example 8, 17-cyclopropylmethyl-6,7-dehydro-4,5a-epoxy-14p-hydroxy-3-methoxy-6,7,2',3'-(6'-methoxyquinolino)morphinan 3, 17-cyclopropylmethyl-6,7-dehydro-4,5α-epoxy-14β-hydroxy-3-methoxy-6,7,2',3'-(8'-phenylquinolino)morphinan 4, 17-cyclopropylmethyl-6,7-dehydro-4,5α-epoxy-14β-hydroxy-3-methoxy-6,7,2',3'-(7',8'-cyclohexenoquinolino)morphinan 5, 17-cyclopropylmethyl-6,7-dehydro-4,5α-epoxy-14β-hydroxy-3-methoxy-6,7,2',3'-(6'-methylquinolino)morphinan 6, and 17-cyclopropylmethyl-6,7-dehydro-4,5α-epoxy-14β-hydroxy-3-methoxy-6,7,2',3'-[6'-(dimethylamino)quinolino]morphinan 7 were used in place of 17-cyclopropylmethyl-6,7-dehydro-4,5a-epoxy-14p-hydroxy-3-methoxy-6,7,2',3'-(7',8'-benzoquinolino)morphinan 2 to obtain 17-cyclopropylmethyl-6,7-dehydro-4,5α-epoxy-3,14β-dihydroxy-6,7,2',3'-(6'-methoxyquinolino)morphinan 37, 17-cyclopropylmethyl-6,7-dehydro-4,5α-epoxy-3,14β-dihydroxy-6,7,2',3'-(8'-phenylquinolino)morphinan 38, 17-cyclopropylmethyl-6,7-dehydro-4,5α-epoxy-3,14β-dihydroxy-6,7,2',3'-(7',8'-cyclohexenoquinolino)morphinan 39, 17-cyclopropylmethyl-6,7-dehydro-4,5α-epoxy-3,14β-dihydroxy-6,7,2',3'-(6'-methylquinolino)morphinan 40, and 17-cyclopropylmethyl-6,7-dehydro-4,5α-epoxy-3,14β-dihydroxy-6,7,2',3'-[6'-(dimethylamino)quinolino]morphinan 41, respectively.

### [Examples 29 - 33]

In accordance with the method of Reference Example 8, 17-acetyl-6,7-dehydro-4,5α-epoxy-14β-hydroxy-3-methoxy-6,7,2',3'-(4'-aminoquinolino)morphinan 24, 17-methyl-6,7-dehydro-4,5α-epoxy-14β-hydroxy-3-methoxy-6,7,2',3'-(4'-aminoquinolino)morphinan 23, 17-cyclopropylmethyl-6,7-dehydro-4,5α-epoxy-14β-hydroxy-3-methoxy-6,7,2',3'-(6',6"-ethano-7',8'-benzoquinolino)morphinan 8, 17-cyclopropylmethyl-6,7-dehydro-4,5α-epoxy-14β-hydroxy-3-methoxy-6,7,2',3'-(7',8',3",2"-indenoquinolino)morphinan 9, and 17-cyclopropylmethyl-6,7-dehydro-4,5α-epoxy-14β-hydroxy-3-methoxy-6,7,2',3'-(6'-methoxyquinolino)morphinan 3 were used in place of 17-cyclopropylmethyl-6,7-dehydro-4,5a-epoxy-14β-hydroxy-3-methoxy-6,7,2',3'-(7',8'-benzoquinolino)morphinan 2 to obtain 17-acetyl-6,7-dehydro-4,5α-epoxy-3,14β-dihydroxy-6,7,2',3'-(4'-aminoquinolino)morphinan 42, 17-methyl-6,7-dehydro-4,5α-epoxy-3,14β-dihydroxy-6,7,2',3'-(4'-aminoquinolino)morphinan 43, 17-cyclopropylmethyl-6,7-dehydro-4,5α-epoxy-3,14β-dihydroxy-6,7,2',3'-(6',6"-ethano-7'-8'-benzoquinolino)morphinan 44, 17-cyclopropylmethyl-6,7-dehydro-4,5α-epoxy-3,14β-dihydroxy-6,7,2',3'-(7',8',3",2"-indenoquinolino)morphinan 45, and 17-cyclopropylmethyl-6,7-dehydro-4,5α-epoxy-3,14β-dihydroxy-6,7,2',3'-(6'-hydroxyquinolino)morphinan 46, respectively.

### [Example 34]

### 17-cyclopropylmethyl-6,7-dehydro-4,5α-epoxy-3,14β-dihydroxy-6,7,2',3'-[4',5'-[N-methylimino(oxomethano)]quinolino]morphinan 47° methanesulfonate

400 mg of 17-cyclopropylmethyl-6,7-dehydro-4,5α-epoxy-3,14β-dihydroxy-6,7,2',3'-[4',5'-[imino(oxomethano)]quinolino]morphinan 34 - hydrochloride was dissolved in 10 ml of DMF, and 255 mg (3.75 mmol) of imidazole and 346 mg (2.25 mmol) of t-butyltrimethylsilyl chloride were added to the resultant solution, followed by reaction at room temperature for 2 hours. After the solvent was distilled off under reduced pressure, water was added to the residue, followed by extraction with ether. The organic layers were washed with saturated salt water, dried over anhydrous sodium sulfate, and then concentrated to obtain 427 mg of 3-t-butyltrimethylsiloxy-17-cyclopropylmethyl-6,7-dehydro-4,5α-epoxy-14β-hydroxy-6,7,2',3'-[4',5'-[imino(oxomethano)]quinolino]morphinan.

427 mg of 3-t-butyltrimethylsiloxy-17-cyclopropylmethyl-6,7-dehydro-4,5α-epoxy-14β-hydroxy-6,7,2',3'-[4.',5'-[imino(oxomethano)]quinolino]morphinan was dissolved in 15 ml of DMF, and 400 mg (2.9 mmol) of potassium carbonate and 180 µl (2.9 mmol) of methyl iodide were added to the resultant solution, followed by reaction at room temperature for 15 hours. Potassium carbonate was filtered off, and the filtrate was concentrated under reduced pressure. To the residue was added water, and the mixture was extracted with ethyl acetate. The organic layers were washed with saturated brine, dried over anhydrous sodium sulfate, and then concentrated. The thus-obtained crude product was purified by silica gel column chromatography (chloroform : methanol = 45:1 - 30:1) to obtain 348 mg (80 % total yield of two steps) of 3-t-butyltrimethylsiloxy-17-cyclopropylmethyl-6,7-dehydro-4,5α-epoxy-14β-hydroxy-6,7,2',3'-[4',5'-[N-methylimino(oxomethano)]quinolino]morphinan.

348 mg (0.57 mmol) of 3-t-butyltrimethylsiloxy-17-cyclopropylmethyl-6,7-dehydro-4,5α-epoxy-14β-hydroxy-6,7,2',3'-[4'.5'-[N-methylimino(oxomethano)]quinolino]morphinan was dissolved in 10 ml of THF, and 0.7 ml (0.7 mmol) of tetrabutylammonium fluoride (1M in THF) was added to the resultant solution, followed by reaction at room temperature for 5 minutes. The reaction solution was purified by silica gel column chromatography (chloroform : methanol = 200:1 → 100:1) to obtain 266 mg (yield 96 %) of title compound. The thus-obtained compound was isolated as a methanesulfonate.

### [Example 35]

### 17-cyclopropylmethyl-6,7-dehydro-4,5α-epoxy-14β-hydroxy-3-methoxy-6,7,2',3'-(4'-hydroxyquinolino)morphinan 48

710.8 mg (1.56 mmol) of 17-cyclopropylmethyl-6,7-dehydro-4,5α-epoxy-3,14β-dihydroxy-6,7,2',3'-(4'-aminoquinolino)morphinan 10 was dissolved in methanol, and a hydrogen chloride/methanol solution was added to acidify the resultant solution, followed by concentration to form a hydrochloride. The hydrochloride was dissolved in a mixed solvent of 1.5 ml of acetic acid and 10 ml of 20% aqueous sulfuric acid solution, and the resultant solution was then cooled to 0°C. To the solution was added dropwise 1 ml of an aqueous solution of 140.3 mg (2.03 mmol) of sodium nitrite, and the resultant mixture was stirred at 0°C for 1.5 hours as it was, and then heated under reflux for 1.5 hours. The reaction solution was allowed to cool, concentrated to a volume of about 1/2, and then made basic by adding about 42 ml of a 2N aqueous sodium hydroxide solution. After extraction with chloroform-ammonia-saturated chloroform-methanol (2:2:1) (30 ml + 10 ml x2), the organic layers were together dried over anhydrous sodium sulfate, and then concentrated. 611 mg of the thus-obtained crude product was purified by silica gel column chromatography (chloroform : methanol = 30:1 → 15:1) to obtain 359.8 mg (yield 51%) of title compound.

### [Examples 36 - 37]

In accordance with the method of Reference Example 8, 17-cyclopropylmethyl-6,7-dehydro-4,5α-epoxy-14β-hydroxy-3-methoxy-6,7,2',3',-(4'-hydroxyquinolino)morphinan 48 and 17-cyclopropylmethyl-6,7-dehydro-4,5α-epoxy-3,14β-dihydroxy-6,7,2',3'-(4'-amino-6'-methoxyquinolino)morphinan 21 were used in place of 17-cyclopropylmethyl-6,7-dehydro-4,5α-epoxy-14β-hydroxy-3-methoxy-6,7,2',3'-(7',8'-benzoquinolino)morphinan 2 to obtain 17-cyclopropylmethyl-6,7-dehydro-4,5α-epoxy-3,14β-dihydroxy-6,7,2',3'-(4'-hydroxyquinolino)morphinan 49 and 17-cyclopropylmethyl-6,7-dehydro-4,5α-epoxy-3,14β-dihydroxy-6,7,2',3'-(4'-amino-6'-hydroxyquinolino)morphinan 50, respectively.

### [Examples 38 - 40]

In accordance with the method of Example 19, 2'-amino-3'-hydroxyacetophenone, 1-(2'-aminophenyl)-2-methyl-1-propanone, and 1-(2'-aminophenyl)-1-propanone were used in place of 2'-aminoacetophenone to obtain 17-cyclopropylmethyl-6,7-dehydro-4,5α-epoxy-3,14β-dihydroxy-6,7,2',3'-(4'-methyl-8'-hydroxyquinolino)morphinan 51, 17-cyclopropylmethyl-6,7-dehydro-4,5α-epoxy-3,14β-dihydroxy-6,7,2',3'-(4'-isopropylquinolino)morphinan 52, and 17-cyclopropylmethyl-6,7-dehydro-4,5α-epoxy-3,14β-dihydroxy-6,7,2',3'-(4'-ethylquinolino)morphinan 53, respectively.

### [Example 41]

### 17-cyclopropylmethyl-6,7-dehydro-4,5α-epoxy-3,14β-dihydroxy-6,7,2',3'-(4'-aminocarbonylquinolino)morphinan 54 methanesulfonate

Oxalyl chloride (0.12 ml, 1.38 mmol), DMF (2 droplets) and 210 mg (0.45 mmol) of 17-cyclopropylmethyl-6,7-dehydro-4,5α-epoxy-3',14β-dihydroxy-6.7.2',3'-(4'-carboxyquinolino)morphinan 30 were added to methylene chloride (10 ml), followed by stirring at room temperature in a nitrogen atmosphere for 22 hours. After concentration, water (5 ml), 28% ammonia water (10 ml) and chloroform (10 ml) were added to the residue, followed by stirring for 16 hours. To the solution was added water (50 ml), and the resultant mixture was extracted with chloroform (50 ml x 2). The organic layers were together dried over anhydrous sodium sulfate, and then concentrated. The thus-obtained product was purified by medium pressure silica gel column chromatography (chloroform : methanol = 40:1 → 20:1), and methanesulfonic acid was added to the product, followed by purification by a Sephadex column to obtain 88 mg (42%) of title compound as a methanesulfonate.

### [Example 42]

### 17-cyclopropylmethyl-6,7-dehydro-4,5α-epoxy-3,14β-dihydroxy-6,7,2',3'-[4'-(benzylamino)quinolino]morphinan 55. methanesulfonate

203 mg (0.46 mmol) of 17-cyclopropylmethyl-6,7-dehydro-4,5α-epoxy-3,14β-dihydroxy-6,7,2'.3'-(4'-aminoquinolino)morphinan 10 was dissolved in THF, and benzaldehyde (94 µl. 0.92 mmol), piperidine (91 µl, 0.92 mmol) and toluene (10 ml) were added to the resultant solution, followed by heating under reflux for 5 hours, with the water separator provided. After the reaction-solution was cooled, the solution was concentrated, and an aqueous saturated sodium hydrogencarbonate solution (50 ml) was added to the residue, followed by extraction with chloroform (50 ml x 2). The organic layers were together dried over anhydrous sodium sulfate, and then concentrated. The resultant residue was purified by medium pressure silica gel column chromatography (chloroform : methanol = 100:1 → 20:1) to obtain 93 mg of imine intermediate. The thus-obtained imine intermediate (93 mg, 0.18 mmol) was dissolved in THF, and lithium borohydride (19 mg, 0.88 mmol) was added to the resultant solution, followed by heating under reflux in a nitrogen atmosphere for 3 hours. To the solution was further added lithium borohydride (20 mg, 0.91 mmol), and the resultant mixture was heated under reflux for 5 hours. The reaction solution was cooled, and a saturated aqueous sodium hydrogencarbonate solution (50 ml) was added to the solution, followed by extraction with chloroform (50 ml x 2). The organic layers were together dried over anhydrous sodium sulfate and then concentrated. The residue was purified by medium pressure silica gel column chromatography (chloroform → chloroform : methanol = 50:1) to obtain 57 mg (23%) of title compound. The compound was isolated as a methanesulfonate.

### [Example 43]

In accordance with the method of Example 42, cyclohexanecarboaldehyde was used in place of benzaldehyde to obtain 17-cyclopropylmethyl-6,7-dehydro-4,5α-epoxy-3,140-dihydroxy-6,7,2',3'-[4'-(cyclohexylamino)quinolino]morphinan 56.

### [Example 44]

### 6',7-dehydro-4,5a-epoxy-3,14p-dihydroxy-6,7,2',3'-(4'-aminoquinolino)morphinan 57 · hydrochloride

To 0.93 g (2.16 mmol) of 17-acetyl-6,7-dehydro-4,5α-epoxy-3,14β-dihydroxy-6,7,2',3'-(4'-aminoquinolino)morphinan 41 was added 80 ml of 6N hydrochloric acid in an argon atmosphere, and 80 ml of methanol was further added to the resultant mixture to form a solution, followed by heating under reflux for 15.5 hours. The solution was allowed to cool and then concentrated. The resultant crude product was purified by a Sephadex column (methanol) to obtain 529 mg (53%) of title compound.

### [Example 45]

In accordance with the method of Example 27, a DMF-benzene mixed solvent was used in place of acetic acid, and methanesulfonic acid was used in place of scandium triflate to obtain 11-cyclopropylmethyl-6,7-dehydro-4,5α-epoxy-3,14β-dihydroxy-6,7,2',3'-[4',5'-[imino(iminomethano)]quinolino]morphinan 58.

The structural formulae, acid addition salts, production yields, and various spectral data of the compounds of the above reference examples and examples of the present invention are shown in the table below.

| | | |
|---|---|---|
| 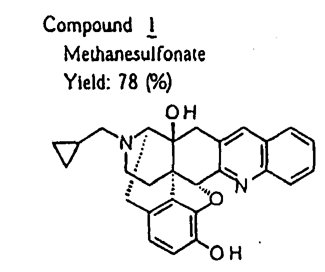 | NMR (ppm) (400 MHz. CDCl₃) 0.14-0.20 (2H, m), 0.53-0.62 (2H, m), 0.90 (1H, m), 1.83-1.91 (1H, m). 1.94 (2H, br s), 2.36-2.50 (4H, m). 2.68-2.77 (2H, m), 2.80 (1H, d,1=16.1 Hz), 2.87-2.91 (1H, m), 3.19 (1H, d, J=18.6 Hz). 3.34 (1H, d, J=6.8 Hz), 5.68 (1H, s), 6.59 (1 H, d, J=8.1 Hz), 6.67 (1H, d, J=8.1 Hz), 7.45-7.50 (1H, m), 1.60.1.64 (1H, m), 7.69 (1H, d, J=7.8 Hz), 7.82 (1H, s), 8.08 (1H. d. J=8.8 Hz). | Melling Point 209 (dec) (°C). Elemental Analysis as C₂₁H₂₆N₂O₂·2.OCH₃SO₃H· 1.7H₂O Calculated: C, 53.64. H, 5.81; N. 4.31; S. 9.88. Found: C, 53.51; H. 5.84: N. 4.12; S. 10.22. IR (cm⁻¹) (not measured) Mass (EI 426 (M⁺) (data of salt-free compound) |
| 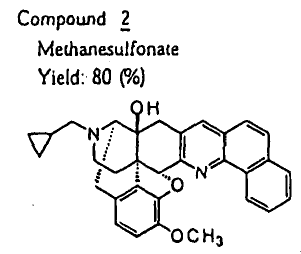 | NMR (ppm) (300 MHz. DMSO-d₆) 0.42-0.57 (2H, m), 0.61-0.80 (2H, m), 1.12 (1H, m). 1.94 m). 2.30 (3H, s). 2.64-2.89 (3H, m). 2.96 (1H. m), 3.08 (1H, d, J=17.0 Hz), 3.14-3.32 (2H, m). 3.36-3.43 (1H, m). 3.54-3.62 (1H, m). 3.66 (3H, s). 9.14 (1H, d, J=6.0 Hz), 5.87 (1H, s), 6.51 (1H, s), 6.79 (1H, d, J=8.3 Hz), 6.86 (1H, d, J=8.3 Hz), 7.75-7.85 (3H. m), 7.96 (1H, d, J=8.8 Hz), 8.05 (1H, m). 8.16 (1H, s), 9.05 (1H, brs), 9.21 (1H, m). | Melting Point 207-213 (dec). (°C). (1H, Elemental Analysis as C₃₂H₃₀N₂O₃·CH₃SO₃H·0.4H₂O Calculated:C, 66.74; H. 5.9 1: N. 4.72, S, 5.40. Found: C. 66.52: H, 6.07; N. 4.80; S. 5.47. 1R (cm⁻¹) (KBr) 3900, 1638. 1508, 1959, 1408. 1288, 1265, 1209, 1125, 1048. 905. 814. 775, 553, 526. Mass (FAB) 491 ((M+H)⁺). |
| 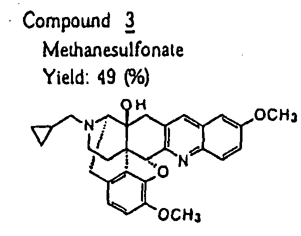 | NMR (ppm) (300 MHz, DMSO-d₆) 0.41-0.55 (2H. m). 0.60-0.79 (2H, m), 1.10 (1H, m), 1.88 (1H. d, J=11.8 Hz), 2.30 (3.3H, s), 2.56-2.86 (2H. m), 2.77 (1H, d, J= 16.5 Hz). 2.92.3.20 (1H, m). 3.01 (1H, d, J=16.8Hz), 3.11-3,30 (2H, m), 3.36-3.49 (1H, m), 3.50 (1H, d, J=20.0 Hz), 3.67 (3H, s). 3.88 (3H, s). 4.11 (1H, d, J=6.0 Hz), 5.68 (1H, s), 6.45 (1H, br s), 6.77 (1H, d, J=8.3 Hz), 6.85 (1H, d, J=8.3 Hz), 7.32 (1H, d, J-2.7 Hz), 7.40-7.44. (1H, m), 7.96 (1H, d, J=9.3 Hz), 7.98 (1H, s), 9.01 (1H, br s). | Melting Point 195-207 (dec). (°C). Elemental Analysis as C₂₉H₃₀N₂O₄· 1.1CH₃SO₃H·0.5H₂O Calculated: C. 61.77: H, 6.10: N, 4.79: S, 6.03. Found: C, 61.65; H, 6.29; N. 4.79; S, 6.11. IR (cm⁻¹) (KBr) 3400, 1626, 1499, 1458. 1338, 1210, 1195. 1052, 899, 785. 561, 536. Mass (EI) 470 (M⁺) (data of salt-free compound) |
| 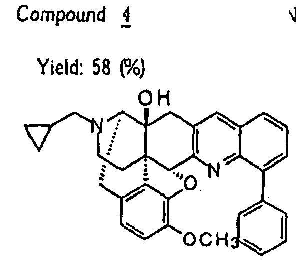 | NMR (ppm) (300 MHz, CDCl₃) 0.13-0.20 (2H, m), 0.50-0.60 (2H, m). 0.82-0.98 (1H, m), 1.80-1.85 (1H, m). 2.35-2.50 (4H, m). 2.67-2.91 (4H, m), 3.21 (1H, d, J=18.0 Hz), 3.30-3.40 (1H, m). 3.81 (3H, s), 5.67 (1H. s), 6.61 (1H, d, J-8.7 Hz). 6.67 (1H, d, J=8.7 Hz), 7.38.7.43 (1H, m), 7.48-7.55 (3H, m). 7.66-7.74 (2H, m), 7.82-7.90 (3H, m). | Mass (EI) 516 (M⁺) |
| 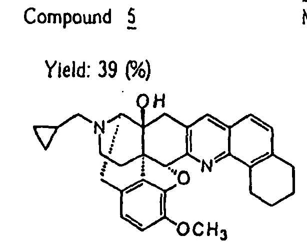 | NMR (ppm) (300 MHz, CDCl₃) 0.13-0.20 (2H, m). 0.53-0.60 (2H, m), 0.90 (1H, m). 1.82-1.98 (5H, m), 2.38-2.51 (4H, m), 2.67.2.76 (3H, m), 2.85-2.92 (3H, m). 3.20 (1H, d, J=18.7 Hz), 3.26-3.38 (2H. m), 3.42-3.55 (1H, m), 3.78 (3H, s), 4.91 (1H, br s), 5.72 (1H, s), 6.61 (1H, d, J=8.1 Hz), 6.66 (1H, d, J=8.1 Hz), 7.17 (1H, d, J=8.5 Hz), 7.41 (1H, d, J=8.2 Hz), 7.70 (1H, s). | Mass (EI) 494 (M⁺) |
| 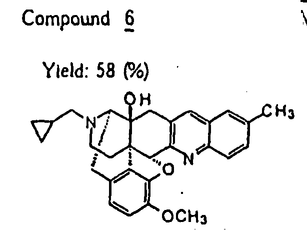 | NMR (ppm) (300 MHz, CDCl₃) 0.13-0.20 (2H, m). 0.53-0.61 (2H, m), 0.82-0.94 (1H, m), 1.80-1.90 (1H, m), 2.37-2.50 (4H, m), 2.49 (3H, s), 2.66-2.90 (4H, m), 3.20 (1H, d, J=18.6 Hz), 3.33 (1H, d, J=6.5 Hz), 3.78 (3H, s), 5.69 (1H, s), 6.61 (1H, d, J=8.1 Hz), 6.66 (1H, d, J=8.1 Hz), 7.43 (1H, s). 7.45 (1H, d, J=8.7 Hz), 7.70 (1H, s), 8.03 (1H, d, J=8.7 Hz). | Mass (EI) 454 (M⁺) |
| 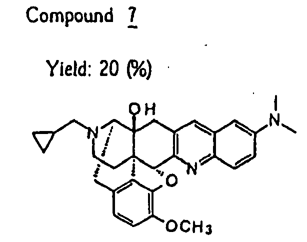 | NMR (ppm) (300 MHz, CDCl₃) 0.16-0.20 (2H, m), 0.55-0.60 (2H, m), 0.80-0.95 (1H, m). 1.82-1.86 (1H, m), 2.39.2.48 (4H, m), 2.65.2.90 (4H, m), 3.05 (6H, s). 3..19 (1H, d, J=18.9 Hz), 3.30-3.38 (1H, m), 3.79 (3H, s), 5.67 (1H, s), 6.46-6.68 (3H.m). 7.30 (1H, dd, J=9.3, 2.7 Hz), 7.58 (1H, s), 7.99 (1H, d, J=9.3 Hz). | Mass (EI) 483 (M⁺) |
| 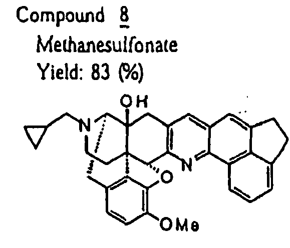 | NMR (ppm) (300 MHz. DMSO-d₆) 0.42-0.57 (2H, m), 0.59-0.79 (2H, m). 1.12 (1H, m), 1.92 (1H, d, J=12.1 Hz), 2.30 (3H, s), 2.62-2.90 (2H, m), 2.85 (1H, d, J=17.0 Hz), 2.92.3.05 (1H, m), 3.04 (1H, d, J=16.8 Hz), 3.13-3.35 (2H, m), 3.35-3.51 (5H, m), 3.57 (1H, d, J=20.3 Hz). 3.66 (3H, s). 4.12 (1H, d, J=6.3 Hz), 5.85 (1H, s), 6.50 (1H, br s), 6.78 (1H, d, J=8.3 Hz), 6.85 (1H, d, J=8.3 Hz), 7.57 (1H, s), 7.64 (1H, d, J=6.9 Hz), 7.73-7.78 (1H, m), 8.07 (1H, s), 8.65 (1H, d, J=7.7 Hz), 9.09 (1H, br s). | Melting Point >220 (dec). (°C). Elemental Analysis as C₃₄H₃₂N₂O₃·CH₃SO₃H· 0.7H₂O Calculated:C, 67.22: H. 6.03: N, 4.48: S, 5.13. Found: C, 67.08: H, 5.94; N, 4.57: S. 5.42. IR (cm⁻¹) (KBr) 3400, 1630, 1508, 1454, 1423, 1332, 1288, 1207, 1123, 1050, 901, 779, 553. Mass (FAB) 517 ((M+H)⁺). |
| 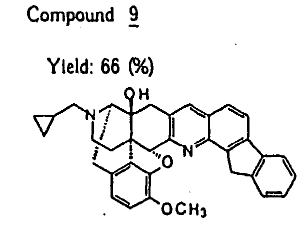 | NMR (ppm) (300 MHz, CDCl₃) 0.15-0.21 (2H, m), 0.56-0.62 (2H, m), 0.83-0.95 (1H, m), 1.87-1.94 (1H, m), 2.35.2.50 (4H, m), 2.67-2.94 (4H, m), 3.22 (1H, d, J=18.6 Hz). 3.36 (1H, d. J=6.6 Hz). 3.79 (3H, s). 4.39 (1H, d. J=22.9 Hz), 4.50 (1H, d, J=22.9 Hz), 5.77 (1H, s). 6.63 (1H, d, J=8.1 Hz), 6.68 (1H, d, J=8.1 Hz), 7.32-7.46 (2H, m), 7.67-7.74 (2H, m), 7.80-7.88 (2H, m). 7.91 (1H, d, J=8.1 Hz). | Mass (EI) 528 (M⁺) |
| 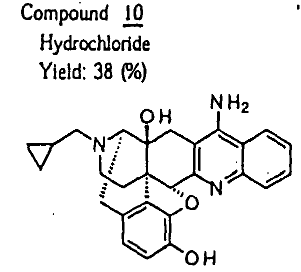 | NMR (ppm) (300 MHz. DMSO-d₆) 0.42-0.53 (2H, m), 0.61-0.75 (2H, m), 1.14 (1H, m). 1.89 (1H. d, J=11.8 Hz). 2.34 (1H, d, J=16.5 Hz). 2.65-2.80 (2H, m), 3.04-3.22 (4H, m), 3.31-3.40 (1H, m), 3.58 (1H, d, 1=20.0 Hz), 4.17 (1H, d. 1=5.8 Hz), 5.88 (1H, s)*,* 6.71 (1H, d, J-8.2 Hz), 6.76 (1H, d, J=8.2 Hz), 7.01 (1H, br s), 7.65 (1H, m). 7.96 (1H, m), 8.07 (1H, d, J=8.2 Hz), 8.43 (1H, br s), 8.50 (1H, d, J=8.5 Hz), 9.24 (2H. m). 9.62 (1H, br s). 14.49 (1H, br s) | Melting Point >240 (°C). Elemental Analysis as C₂₇H₂₇N₃O₃· 1.9HCl· 1.0H₂O Calculated: C, 61.32; H, 5.89; N, 7.95; CI. 12.74. Found: C, 61.59; H, 5.86; N. 7.67: Cl, 12.79. IR (cm⁻¹) (KBr) 3400, 3200, 1650. 1595, 1502, 1460, 1433, 1379, 1323, 1251. 808, 764. Mass (FAB) 442 ((M+H)⁺). |
| 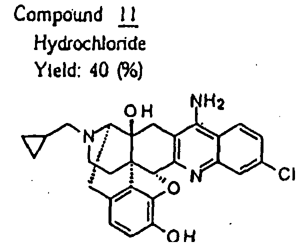 | NMR (ppm) (300 MHz, DMSO-d₆) 0.40-0.54 (2H, m), 0.60-0.77 (2H, m), 1.10 (1H, m), 1.87 (1H. m), 2.31 (1H, d, J= 16.8 Hz), 2.43.2.81 (3H, m). 2.96 (1H, d, J=17.3 Hz), 3.02-3.06 (1H, m), 3.14-3.20 (2H, m), 3.33-3.39 (1H, m). 3.58 (1H, d J=20.0 Hz), 4.16 (1H, d. J=6.6 Hz). 5.79 (1H, s), 6.69-6.77 (2H, m), 7.08 (1 H. br s). 7.69-7.72 (1H, m), 8.03 (1H, s), 8,44-8.68 (1H, br s), 8.48 (1H, d, J=8.8 Hz). 9.25 (1H. br s), 9.49 (1H, br s). 9.63 (1H, s), 14.67 (1H, br s). | Melting Point >220 (dec) °C). Elemental Analysis as C₂₇H₂₆CIN₃O₃·2HCl·0.3H₂O Calculated: C, 58.51; H, 5.20; Cl. 19.19; N. 7.58. Found: C, 58.45; H, 5.30; Cl. 19.27; N, 7.50. IR (cm⁻¹) (KBr) 3400. 3180. 1636, 1605. 1497. 1468. 1431. 1379. 1247 1114, 924, 816. Mass (FAB) 476 ((M+H)*). |
| 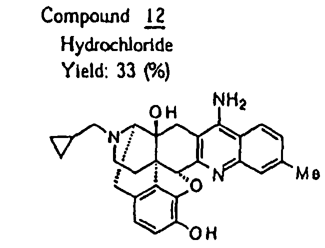 | NMR (ppm) (300 MHz, DMSO-d₆) 0.40-0.54 (2H, m), 0.59-0.75 (2H, m), 1.14 (1H, m). 1.87 (1H, d, J=11.0 Hz), 2.31 (1H, d, J=16.8 Hz), 2.53 (3H, s), 2.63-2.74 (2H, m), 3.00-3.22 (4H, m), 3.34 (1H, m), 3.57 (1H, d, J=19.5 Hz), 9.17 (1H, m), 5.85 (1H, s), 6.70 (1H, d, J=8.2 Hz), 6.76 (1H, d, J=8.2 Hz), 7.01 (1H, br s), 7.49 (1H, d, J=8.8 Hz), 7.82 (1H, s). 8.35 (1H, br s), 8.39 (1H, d, J=8.8 Hz), 9.26 (2H, br s), 9.63 (1H, s), 14.38 (1H, br s). | Melting Point >240 (dec) (°C). Elemental Analysis as C₂₈H₂₉N₃O₃·1.9HCl·0.4H₂O Calculated: C. 63.21; H, 6.01; N. 7.90; Cl, 12.66. Found: C, 63.09; H, 6.11; N, 7.96; Cl. 12.71. IR (cm⁻¹) (KBr) 3900, 3200, 16A2, 1599, 1504, 1468, 1431. 1380, 1319. 1249, 816, 748. Mass (FAB) 456 ((M+H)'). |
| 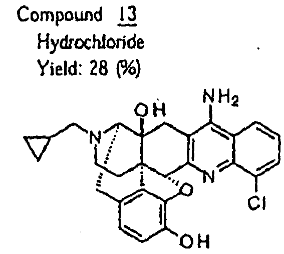 | NMR (ppm) (400 MHz, DMSO-d₆) 0.48 (m. 2H), 0.65 (m. 1H), 0.72 (m, 1H), 1.14 (m, 1H). 1.89 (br d, 1H. J=12.2 Hz), 2.40 (d. 1H, J=17.1 Hz), 2.68 (m, 1 H), 2.78 (m, 1H), 2.98 (d. 1H, J=17.1 Hz), 3.06-3.23 (m, 3H), 3.58 (d, 1H, J=20.0 Hz). 4.19 (d, 1H, J=6.8 Hz), 5.87 (s, 1H), 6.72 (d, 1H, J=8.3 Hz). 6.76 (d. 1H, J=8.3 Hz). 7.04 (br s, H, OH), 7.74 (br d. 1H, J=7.3 Hz), 7.88 (br t. 1H, J=7.8 Hz). 8.08 (br d, 1H. J=8.3 Hz). 8.57 (br s, 1H, NH). 8.81 (br s, 1H, NH). 9.23 (br s. 1H, NH⁺). 9.63 (bt s. 1H. OH), 14.72 (br s, 1H, NH⁺). | Melting Point >250 (°C). Elemental Analysts as C₂₇H₂₅ClN₃O₃·2HCl·1.8H₂O Calculated: C, 55.79; H, 5.61; Cl. 18.30; N. 7.23. Found: C, 55.87; H, 5,61; Cl. 18.00; N, 7.07. IR (cm⁻¹) (KBr) 3392, 1630, 1595. 1545. 1493, 1460, 1402. 1323, 1276, 1249. 1178, 1116, 1052. 1031, 940, 806. Mass (FAB) 461 ((1v1+H)'). |
| 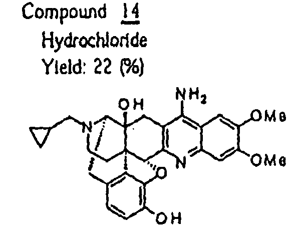 | NMR (ppm) (300 MHz, DMSO-d₆) 0.46 (2H. m), 0.58-0.78 (2H, m), 1.09 (1 H, m), 1.80-1.90 (1M, m), 2.29 (1H, d, J=16.8 Hz), 2.54-3.42 (7H. m), 3.44-3.62 (1H, m), 3.89 (3H, s), 3.95 (3H, s), 4.14 (1H, m), 5.72 (1H, s). Me 6.70 (1N, s). 6.70 (1H, s), 7.02 (1H, br s), 7.33 (1H, s), 7.73 (1H, s). 8.05 (1H, br s), 8.84 (1H, br s), 9.24 (1H, br s), 9.59 Me (1H, br s), 14.21 (1H, br s). | Melting Point >230 (dec) (°C). Elemental Analysis as C₂₉H₃₁N₃O₅· 1.9HCl· 0.7H₂O Calculated:C, 59.70; H. 5.93; N. 7.20; Cl, 11.54, Found: C, 59.82; H, 5.85; N. 7.16; Cl. 11.39. IR (cm⁻¹) (KBr) 3400, 3200, 1657, 1605, 1516. 1466, 1439, 1421, 1290. 1247. 1218. Mass (FAB) 502 ((M+H)⁺). |
| 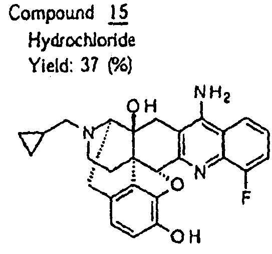 | NMR (ppm) (300 MHz, CD₃OD) 0.61 (m. 2H), 0.85 (m, 1H). 0.94 (m, 1H), 1.25 (m. 1H). 2.07 (m, 1H), 2.54 (d, 1 H, J = 16.7 Hz), 2.86 (m. 1 H), 3.00 (d, 1H, *J*=16.7 Hz), 3.02-3.15 (m, 2H), 3.30-3.44 (m, 2H), 3.48 (m, 1H), 3.63 (d, 1H, *J*=20.3 Hz), 4.36 (d, 1H, *J*=6.6 Hz), 5.76 (s, 1H), 6.77 (d, 1H, *J*=8.2 Hz), 6.83 (d, 1H, *J*=8.2 Hz), 7.38 (br dd, 1H, *J*=13.7, 7.9 Hz). 7.79 (br d, 1H, *J*=7.5 Hz), 7.90 (m. 1H), | Melting Point >267 (dec) (°C). Elemental Analysis as C₂₇H₂₈FN₃O₃ · 1.8HCl ·0.5H₂O Calculated: C, 60.71: H. 5.43; Cl, 11. 95: F. 3.56. N, 7.87. Found: C, 60.66; H, 5.52; Cl. 11.85; F. 3.54; N, 7.90. IR (cm⁻¹) (KBr) 3378. 3194, 1642. 1605, 1504, 1466, 1417, 1377, 1325, 1280, 1249, 1181, 1118, 1062. 1038, 922. 899. 808 Mass (FAB) 460 ((M+H)⁺). |
| 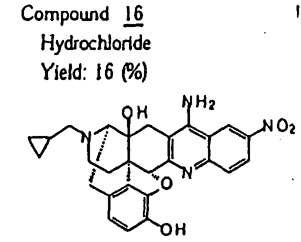 | NMR (ppm) (600 MHz. CD₃OD) 0.58 (m, 2H). 0.83 (m. 1 H), 0.92 (m. 1 H), 1.22 (m. 1H), 2.05 (dd. H, *J=13.5.* 3.1 Hz), 2.54 (d, 1H *J*=16.5 Hz), 2.85 (ddd, 1H, *J*=13.4, 13.4, 4.9 Hz). 3.00 (d, 1H, *J*=16.5 Nz), 3.02-3.1 1 (m, 2H). 3.28.3.40 (m. 2H), 3.48 (m. 1H), 3.61 (d. 1H, *J*=19.8 Hz). 4.34 (br d, 1H, *J*=4.6 Hz), 5.74 (s, 1H), 6.74 (d, 1H, *J*=8.1 Hz). 6.81 (d, 1H, *J*=8.1 Hz), 8.05 (br d, 1H, J=8.5 Hz), 8.54 (m. 1H), 9.38 (br s. 1H). | Melting Point 265-280 (dec) (°C). Elemental Analysis as C₂₇H₂₆N₄O₃· 1.6HCl· 0.6H₂O Calculaled:C. 58.36; H, 5.22; Cl, 10.21: N, 10.08. Found: C, 58.34; H. 5.39: Cl, 10.40; N, 9.86. IR (cm⁻¹) (KBr) 3346, 3174. 1644. 1611. 1506. 1466. 1433. 1381. 1338, 1278, 1178, 1116, 1052. 920. 835. 810. 746. Mass (FAB) 487 ((M+H)'). |
| 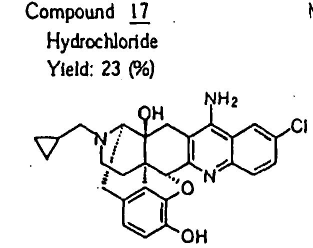 | NMR (ppm) (300 MHz. CD₃OD) 0.55-0.65 (m, 2H). 0.80-0.95 (m, 2H), 1.20-1.30 (m, 1H), 2.02-2.11 1 (m, 1H), 2.55 (d, 1H, *J*=17.0Hz), 2.87 (ddd, 1H, J=13.4, 13.4. 5.0Hz), 3.03 (d. 1 H, *J*=17.0Hz), 3.00-3.25 (m. 2H). 3.28.3.45 (m, 2H), 3.50 (dd, 1H, J=13.4. 7.5Hz), 3.63 (d, 1H, J=20. Hz), 4.38 (d, 1H, J=6.9Hz), 5.80 (s, 1H), 6.78 (d, 1H, J=8.4Hz). 6.84 (d, 1H, J=8.4Hz), 7.89 (dd, 1H, J=9.0. 2.1 Hz). 7.96 (d. 1H, J=9.0Hz), 8.45 (d, 1H, J=2.1Hz). | Melting Point 220 (°C). Elemental Analysis as C₂₇H₂₆N₃O₃·1.9HCl· 1.0H₂O·0.07EtOAc Calculated: C, 57.54; H. 5.39; N. 7.38; Cl. 18.06. Found: C, 57.40; H, 5.49: N, 7.31; Cl, 18.23. IR (cm⁻¹) (KBr) 3400. 1650, 1997, 1473, 1433. 1383, 1321. 1249. 1180, 1116, 926. Mass (FAB) 476 ((M+H)⁺). |
| 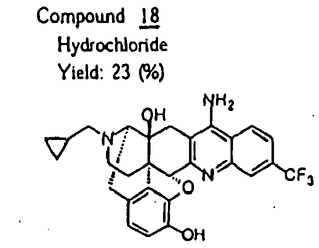 1 | NMR (ppm) (300 MHz, CD₃OD) 0.53-0.61 (m. 2H), 0.79-0.85 (m. 1H), 0.87-0.92 (m, 1H), 1.18-1.25 (m. 1H), 2.01-2.07 (m, 1H), 2.54 (d. 1H, J=16.5Hz), 2.84 (ddd. 1H. J=13.3, 13.3. 5.0Hz), 3.00 (d. 1H, J=16.5Hz), 3.02-3.09 (m, 2H), 3.25.3.40 (m, 2H), 3.46 (dd, 1H, J = 13.3, 7.0Hz), 3.59 (d, 1H, J=19.5Hz).- 4.33 (d, 1H, J=6.7Hz), 5.76 (s, H). 6.73 (d, 1H, J=8.0Hz), 6.88 (d, 1H, J=8.0Hz), 7.84 (dd, 1H, J=9.0. 0.9Hz), 8.23 (s, 1H), 8.51 (d, 1H. J=9.0). | Melting Point 230 (°C). Elemental Analysis as C₂₈H₂₆F₃N₃O₃· 1.8HCl·0.5H₂O Calculated: C, 57.57. H, 4.97; N, 7.19: F. 9.76; CI. 10.92. Found: C. 57.78; H, 4.98; N, 7.20; F, 9.38; Cl. 11.10. IR (cm⁻¹) (KBr) 3400, 1651, 1603, 1508, 1437, 1390, 1328, 1292. 1247, 1181, 1133, 1067, 899. Mass (FAB) 510 ((M+H)⁺). |
| 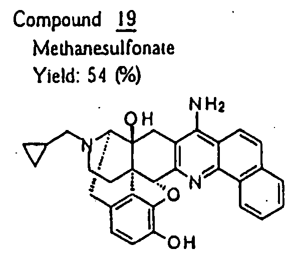 | NMR (ppm) (300 MHz. DMSO-d₆) 0.42-0.56 (2H, m). 0.61-0.80 (2H, m), 1.11 (1H, m), 1.93-2.02 (1H, m), 2.32 (6H, s), 2.35-2.46 (1H, m), 2.61-2.90 (2H, m), 2.97-3.13 (1H, m), 3.04 (1H, d, J-18.4 Hz), 3.17-3.30 (1H, m), 3.28 (1 H, d, J=12.9 Hz). 3.33-3.43 (1 H, m), 3.59 (1 H, d, J= 19.8 Hz) 4.12-4.20 (1 H, m). 6.06 (1 H, br s), 6.72 (1 H s). 6.72 (1H, s), 6.76 (1H, br s). 7.96 (2H, m), 8.05-8.13 (1H, m), 8.15-8.22 (1H, m), 8.30 (1H, d, J=8.5 Hz), 8.42 (1H, br s), 9.15 (1H, br s), 9.27 (1H, br s), 9.31 (1H, d. J=8.2 Hz), 9.50 (1H, brs), 13.65 (1H, br s). | Melting Point >220 (dec). (°C). Elemental Analysis as C₃₁H₂₉N₃O₃·2.0CH₃SO₃H·0.5H2O Calculated: C, 57.21; H. 5.53: N, 6.07; S, 9 26. Found: C. 57.16; H, 5.69: N. 6.02; S. 9.31. !R(cm'')(KBr) . 3400, 3200. 1638. 1620. 1493, 1466. 1423, 1330, 1200, 1050, 818, 785, 559. Mass (FAB) 492 ((M+H)⁺). |
| 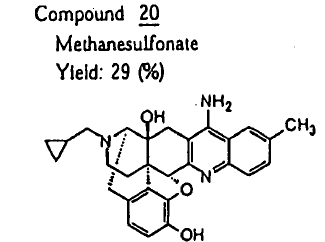 | NMR (ppm) (400 MHz, CD₃OD) 0.30.0.40 (2H, m), 0.60-0.75 (2H, m). 1.00-1.10 (1H, m). 1.82-1.90 (1H, m). 2.44 (1H, d, J=8,4 Hz), 2.51 (3H, s), 2.55-2.65 (2H, m). 2.70 (3.0H. s), 2.69-2.76 (1H, m). 2.81 (1H, d, J=8.4 Hz), 2.95-3.06 (2H. m) 3.30-3.40 (2H, m). 3.78 (1H, br s), 5.58 (1H, s), 6.66 (1H, d, 1-6.6 Hz), 6.68 (1H, d, J=6.6 Hz). 7.64 (1H, d. J=7.0 Hz), 7.75 (1H, d, J=7.0 Hz), 7.97 (1H, s). | Melting Point 215 (°C). Elemental Analysis as C₂₈H₂₉N₃O₃· 1.0MeSO₃H·0.9H₂O Calculated: C, 61.34: H. 6.18: N. 7.40: S. 5.65. Found: C, 61.52: H. 5.99: N. 7.34; S. 5.66. IR (cm⁻¹) (KBr) 3400, 1690, 1508, 1460. 1381, 1328, 1201, 1120, 1048. Mass (FAB) 456 ((M+H)⁺). |
| 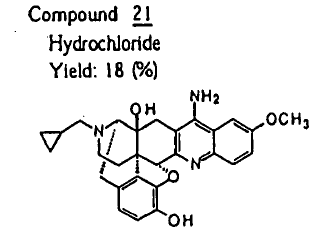 | NMR (ppm) (300 MHz. CD₃OD) 0.50-0.65 (2H. m), 0.80-0.95 (2H, m), 1.08-1.15 (1H, m). 2.01-2.08 (1H, m), 2.55 (1H, d, J=16.8 Hz), 2.78-2.91 (1H, m), 2.96-3.12 (2H, m). 3.02 (1H, d, J=16.8 Hz), 3.25-3.50 (3H, m), 3.62 (1H. d. J=19.5 Hz), 4.00 (3H. s), 4.30-4.36 (1H, m). 5.79 (1H, s), 6.77 (1H, d. J=8.4 Hz), 6.83 (1H, d, J= 8.4 Hz), 7.59 (1H, dd. J=9.3. 2.7 Hz), 7.71 (1H, d, J=2.7 Hz), 7.90 (1H, d, J=9.3 Hz). | Melting Point 185 (°C). Elemental Analysis as C₂₈H₂₉N₃O₄· 1.9HCl·0.5H₂O Calculated: C, 61.16; H. 5.85: N, 7.64: Cl. 12.25. Found: C. 61.23; H. 5.94: N. 7,44; Cl, 12.34. IR (cm⁻¹) (KBr) 3400, 3200, 1659, 1624, 1595. 1506. 1473. 1595. 1506. 1473. 1406, 1251. Mass (FAB) 472 ((M+H)⁺). |
| 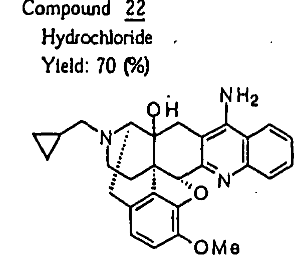 | NMR (ppm) (300 MHz, DMSO-d₆) 0.40-0.54 (2H, m), 0.60-0.77 (2H, m), 1.10 (1H, m). 1.86-1.90 (1H, m), 2.32 (1H, d, J=17.0 Hz), 2.60-2.82 (2H, m), 2.94-3.06 (2H, m), 3.14-3.28 (2H, m), 3.31-3.41 (1H, m), 3.55-3.60 (1H, m), 3.67 (3H, s), 4.18-4.20 (1H, m). 5.84 (1H, s), 6.86 (1H, d, *J*=8.5 Hz), 6.90 (1H, d, J=8.5 Hz), 7.16 (1H, br s), 7.62-7.67 (1H, m), 7.91-8.00 (2H, m), 8,30-8.70 (1H, brs), 8.43 (1H, d, J=8.8 Hz), 9.30 (1.7H, br s), 14.58 (0.6H, br s). | Melting Point >240 (dec). (°C). Elemental Analysis as C₂₈H₂₉N₃O₃·1.94HCl·0.4H₂O Calculated: C, 63.04; H, 6.00; N, 7.88; Cl. 12.89. Found: C, 63.07; H, 6.08; N, 7.88; Cl. 12.92. IR (cm⁻¹) (KBr) 3400, 1640, 1597, 1504, 1991, 1263, 1164, 1127, 1052, 913, 764. Mass (FAB) 456 ((M+H)⁺). |
| 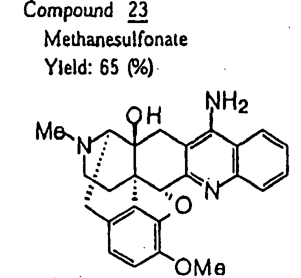 | NMR (ppm) (600 MHz. DMSO-d₆) 1.88-1.94 (m. 1H), 2.33 (s, 6H). 2.34 (d. J= 16.5 Hz, 1 H), 2.64-2.67 (m. 1H). 2.76-2.85 (m. 1H). 2.93 (d, *J*=16.5 Hz. 1H). 2.94-2.99 (m, 3H), 3.15-3.24 (m, 2H), 3.65 (d. *J*=20.1 Hz, 1H). 3.71 (s, 3H), 3.85-3.89 (m, 1H), 5.85 (br s, 1H), 6.66 (br s. 1H). 6.88 (d, J=8.2 Hz, (H), 6.92 (d. *J*=8.2 Hz. 1H), 7.66-7.71 (m, 1H), 7.95-8.01 (m, 2H), 8.45 (d, J=8.5 Hz, 1H), 8.96 (br s, 1H), 9.23 (br s, 1H), 9.35 (br s, 1H), 14.2 (br s. 1H). | Melting Point 245-250 (°C), Elemental Analysis as C₂₅H₂₅N₃O₃· 2.0CH₃SO₃H· 0.65H₂O Calculated: C. 52.36: H, 5.58; N, 6.78; S. 10.35. Found: C, 52.32; H. 5.59; N. 6.78; S. 10.53. IR (cm⁻¹) (KBr) 3450, 1657, 1611, 1597. 1508. 1456, 1286, 1265, 1201, 1060. 785. Mass (FAB) 416 ((MtH)*). |
| 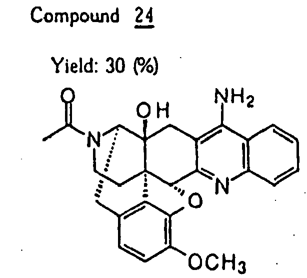 | NMR (ppm) (300 MHz, DMSO.d₆) 1.41-1.60 (m, 1H), 2.05 (s, 1.35H). 2.13 (s, 1.65H), 2.23 (d. *J*=16.2 Hz, 0.45H), 2.26 (d, *J*=16.2 Hz, 0.55H), 2.24-2.36 (m, 1H), 2.70 (d, *J*= 16.2 Hz, 0.45H), 2.74 (d, *J*=16.2 Hz, 0.55H), 2.83 (d, *J*=19.2 Hz. 0.45H), 2.98-3.15 (m, 1H), 3.03 (d, *J*=16.2 Hz. 0.55H), 3.17 (dd, *J*=19.2, 6.9 Hz. 0.45H). 3.28 (dd, *J*=19.2, 6.9 Hz. 0.55H). 3.63 (s, 3H), 3.66-3.76 (m, 0.45H). 4.14 (d. *J*=6.9 Hz, 0.45H). 4.35-4.46 (m. 0.55H), 4.98 (s, 0.45H), 4.99 (d, *J*=6.9 Hz, 0.55H), 5.16 (s, 0.55H), 5.35 (s. 0.95H), 5.36 (s, 0.55H). 6.44 (s, 0.9H). 6.46 (s. 1.1H). 6.22-6.78 (m, 2H). 7.31-7.38 (m, 1H), 7.53-7.60 (m. 1H), 7.73-7.79 (m, 1H), 8.10-8.15 (m, 1 H). | IR (cm-¹) (KBr) 3400, 1618, 1506. 1496, 1278, 1166, 1129, 1052, 909. 762. Mass (EI) 443 (M⁺) |
| 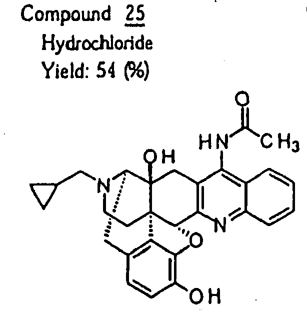 | NMR (ppm) (300 MHz, DMSO-d₆) 0.40-0.58 (2H, m), 0.58-0.77 (2H, m), 1.13 (1N, m), 1.87 (1H, d. J=11.3 Hz). 2.18 (3H, s), 2.42-2.50 (1H, m), 2.59-2.60 (1H, m), 2.73-3.00 (2H, m), 3.00 (1H, d, J=17.3 Hz). 3.06-3.16 (2H, m), 3.40.3.54 (2H, m). 4.15 (1H, d, J=6.3 Hz), 5.73 (1H, s), 3 6.45 (1H, br s), 6.66 (2H, s), 7.65 (1H, m), 7.81 (1H, m), 7.99 (1H, d, J=8.5 Hz). 8.09 (1H, d, J=8.2 Hz), 9.00 (1H, brs), 9.38 (1H, br s), 10.32 (1H, br s). | Melting Point >230 (dec) (°C). Elemental Analysis as C₂₉H₂₉N₃O₄·1.3HCl·0.6H₂O Calculated: C, 64.29; H, 5.86: N, 7.76: CI, 8.51. Found: C, 64.29; H, 5.85: N. 7.70: Cl, 8.60. IR (cm⁻¹) (KBr) 3400. 3200, 1650. 1508. 1464. 1412, 13T3. 1325. ! 249. 772. Mass (FAB) 484 ((M+H)*). |
| 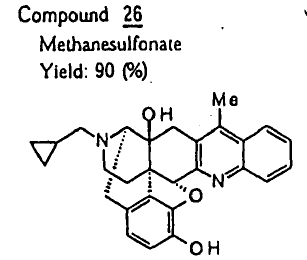 | NMR (ppm) (300 MHz. CD₃OD) 0.57-0.65 (m, 2H), 0.84 (m, 1H), 0.95 (m, 1H). 1.23 (m, 1H), 2.07 (m, 1H), 2.71 (s. 3H), 2.73 (s, 3H), 2.80-2.93 (m, 2H), 3.03-3.15 (m. 2H), 3.26-3.55(m. 4H), 3.59 (d, 1H, J=19.8 Hz), 4.34 (br d, 1H. J=6.6 Hz), 5.81 (s, 1H), 6.73 (d, 1H. J=8.0 Hz). 6.79 (d. 1H, J=8.0 Hz). 7.74 (br t. 1H, J=8.0 Hz), 7.88 (br t. 1H, *J*=8.0 Hz), 8.15 (br d, *J*=8.1 Hz), 8.25 (br d. J-8.7 Hz). | Melting Point 240-250 (dec) (°C). Elemental Analysis as C₂₈H₂₈N₂O₃·1.2MeSO₃H· 1.1 H₂O Calculated: C, 60.92; H, 6.13: N, 4.87: S. 6.68. Found: C, 60.98; H, 6.23; N, 4.61: S. 6.75. IR (cm⁻¹) (KBr) 3398, 1644, 1620, 1506, 1466. 1417, 1383, 1361. 1328, 1241, 1193, 1125, 1114, 1060, 922, 903, 843, 812. 774. Mass (FAB) 941 ((M+H)'). |
| 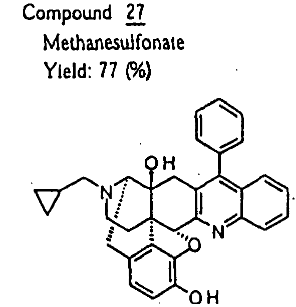 | NMR (ppm) (400 MHz, DMSO-d₆) 0.34-0.42 (1H, m), 0.43-0.52 (1H, m), 0.52-0.61 (1H, m), 0.61-0.70 (1H, m). 1.00-1.11 (1H, m). 1.89 (1H, d, J=11.2 Hz), 2.36 (6.9H.s), 2.45 (1H. m), 2.60-2.93 (4H, m), 3.03-3,17 (2H, m). 3.36-3.46 (2H, m), 3.96 (1H. d, J=6.4 Hz), 5.79 (1H. s), 6.10-6.60 (1H, br s), 6.63 (1H, d, J=8.3 Hz), 6.67 (1H, d, J=8.3 Hz), 7.14-7.16 (1H. m), 7.23 (1H, d, J=8.3 Hz), 7.33 (1H. d, J=7.8 Hz). 7.47-7.57 (3H, m), 7.59-7.64 (1H, m), 7.82 (1H, dd, J-8.3. 8.3 Hz). 8.14 (1H, d, J=8.3 Hz). 8.86 (1H, br s), 9.00-10.00 (1H, br s). | Melting Point 217-219 (°C). Elemental Analysis as C₃₃H₃₀N₂O₃·2.3CH₃SO₃H·0.8 H₂O Calculated: C, 57.44: H, 5.57; N. 3.79; S, 9,99. Found: C. 57.22-, H, 5.69: N, 3.75; S, 10.38. IR (cm⁻¹) (KBr) 3400, 3078, 1638, 1562, 1510, 1460, 1328, 1195, 1050, 934. 847, 812, 785, 706, 553. Mass (FAB) 503 ((M+H)⁺). |
| 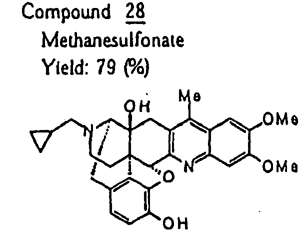 | NMR (ppm) (300 MHz, CD₃OD) 0.55.0.65 (m, 2H), 0.83 (m, 1H), 0.93 (m, 1H), 1.22 (m. 1H), 2.03 (m. 1H), 2.36 (m, 3H), 2.73 (s. 3H), 2.73-2.92 (m, 2H), 3.03-3.13 (m, 2H), 3.24-3.51 (m. 4H). 3.57 (d. 1H, *J*=19.5 Hz), 3.98 (s, 3H), 4.03 (s, 3H), 4.36 (m. 1H), 5.69 (s. 1H), 6.72 (d. 1H. *J*=8.3 Hz), 6.77 (d, 1H, *J*=8.3 Hz). 7.11 (br s. 1H), 7.30 (br s, 1H). | Melting Point 255-270 (dec) (°C). Elemental Analysis as C₃₀H₃₂N₂O₅· MeSO₃H· 0.7H₂O Calculated: C. 61.11: H, 6.19; N. 4.60: S. 5.26. Found: C. 61.05; H, 6.27; N. 4.65; S. 5.33. IR (cm⁻¹) (KBr) 3402, 1647, 1628, 1514, 1506. 1485. 1935. 1386. 1357. 1328, 1290, 1255, 1209. 1118. 1033. 996, 895. 851. 816. 777. Mass (EI) 441 (M⁺) (data of salt-free compound) |
| 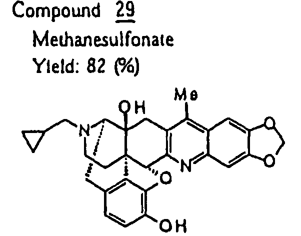 | NMR (ppm) (300 MHz, CD₃OD) 0.55-0.66 (m, 2H), 0.83 (m, 1H). 0.93 (m, 1H), 1.23 (m, 1H), 2.03 (m, 1H), 2.41 (m, 3H), 2.70 (s, 3H), 2.78 (d, 1H, *J*=17.3 Hz), 2.91 (dt, 1H, J=5.2, 13.7 Hz), 3.03-3.15 (m, 2H), 3.27-3.51 (m, 3H) 3.30 (d. 1H, J=17.3 Hz), 3.57 (d, 1H *J*=20.0 Hz), 4.38 (br d, 1H, *J*=6.6 Hz), 5.72 (s, 1H) 6.18 (br s, 2H), 6.72 (d, 1H, *J*=8.3 Hz). 6.78 (d, 1H, *J*=8.3 Hz), 7.28 (s, 1H), 7.34 (s, 1H), | Melting Point >255 (dec) (°C). Elemental Analysis as C₂₉H₂₈N₂O₅·1.2MeSO₃H· 0.4H₂O Calculated: C. 59.75: H. 5.58; N, 4.61; S, 6.34. Found: C, 59.81; H, 5.84: N, 4.67: S, 6.12. IR (cm⁻¹) (KBr) 3442, 1638, 1620. 1506, 1470, 1377, 1328, 1251, 1193, 1118, 1044, 942, 874, 785. Mass (FAB) 485 ((M+H)⁺). |
| 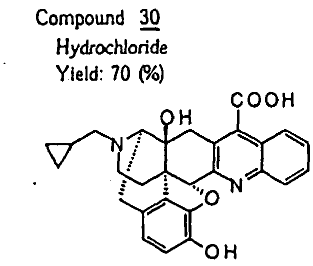 | NMR (ppm) (300 MHz. DMSO-d₆) 0.38.0.54 (2H, m), 0.57-0.77 (2H, m), 1.09 (1H, m), 1.71-1.92 (1H, m). 2.57-2.97 (3H, m), 2.66 (1H, d, J=15.9 Hz), 2.99-3.26 (3H, m), 3.06 (1H, d, J=16.5 Hz), 3.3 2-3.4 (1H. m), 3.46 (1H. d. J=19.2 Hz), .4.18 (1 H, m), 5.70 (1 H, s), 6.61 (1H, br s), 6.65 (1H, s). 6.65 (1H. s), 7.64-7.69 (1H, m), 7.77-7.84 (2H, m). 8.09 (1H, d. J=8.2 Hz). 9.01 (1H, br s); 9.38 (1H, br s). | Melting Point >230 (dec). (°C), Elemental Analysis as C₂₈H₂₆N₂O₅·0.9HCl·1.4H₂O Calculated: C. 63.63; H, 5.66; Cl, 6.04; N, 5.30. Found: C, 63.68: H. 5.76: Cl. 5.88; N. 5.27. IR (cm⁻¹) (KBr) 3400, 1719. 1620, 1508. 1462, 1386, 1321, 1249, 1029, 934, 810. Mass (FAB) 471 ((M+H)'). |
| 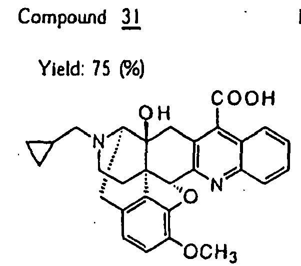 | NMR (ppm) (300 MHz. D₂O) 0.41-0.55 (2H, m), 0.61-0.92 (2H, m), 1.13 (1H, m), 2.09-2.17 (1H. m). 2.T1-2.84 (1H, m). 2.97 (1H, d. J=17.0 Hz), 3.02-3.19 (3H, m), 3.31-3.48 (3H, m). 3.58 (1H. d, J=20.9 Hz), 3.76 (3H, s). 4.42 (1H, d. J=6.0 Hz), 6.14 (1H, s), 6.95 (1H, s), 6.95 (1H, s), 7.85-7.90 (1H, m), 7.97 (1H. d, J=8.5 Hz), 8.05-8.11 (1H, m). 8.19 (1H, d. J=8.5 Hz). | Mass (FAB) 485 ((M+H)'). |
| 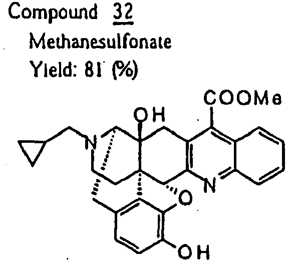 | NMR (ppm) (300 MHz, DMSO-d₆) 0.40-0.56 (2H, m). 0.58-0.80 (2H, m), .1.09 (1H. m), 1.85-1.94 (1H, m), 2.32 (4.5H, s), 2.57-3.02 (3H, m), 2.67 (1H, d, J=17.0 Hz). 2.96 (1H, d, J=17.0 Hz), 3.09-3.27 (2H, m), 3.37-3.50 (1H, m), 3.49 (1H, d, J=19.8 Hz), 4.01 (3H, s), 4.14 (1H, d, J=6.6 Hz), 5.74 (1H, s). 6.52 (1H, br s), 6.66 (1H, s), 6.66 (1H, s). 7.68-7.74 (1H, m), 7.80 (1H, d. J=7.7 Hz), 7.85-7.90 (1H, m). 8.14 (1H, d. J=8.0 Hz), 8.99 (1H, br s), 9.41 (1H, br s). | Melting Point >220 (dec). (°C). Elemental Analysis as C₂₉H₂₈N₂O₅· 1.5CH₃SO₃H**·** 0.5H₂O Calculated: C, 57.44; H. 5.53; N, 4.39; S. 7.54. Found: C, 57.34; H. 5.57; N, 4.40; S. 7.73. IR (cm⁻¹) (KBr) 3400. 1736. 1638, 1962. 1328, 1195, 1060. 774, 557. 542, 524. Mass (FAB) 485 ((M+H)'). |
| 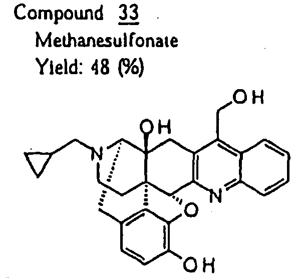 | NMR (ppm) (300 MHz, DMSO-d₆) 0.42.0.57 (2H, m), 0.59-0.80 (2H, m), 1.11 (1H. m). 1.85-1.93 (1H, m). 2.31 (4.5H, s), 2.57-3.02 (3H, m). 2.71 (1H, d, J=16.5 Hz), 3.11-3.27 (2H, m), 3.33.3.53 (1H, m), 3.39 (1H, d, J=16.5 Hz). 3.52 (1H, d, J=19.8 Hz), 4.15 (1H, d. J=6.3 Hz), 4.82 (1 H, d. J=12.5 Hz), 4.98 (1H, d. J=12.5 Hz), 5.71 (1H, s), 6.30 (1H, br s), 6.62 (1H, d. J=8.2 Hz), 6.66 (1H, d. J=8.2 Hz), 7.63-7.68 (1H, m), 7.76-7.81 (1H, m). 8.05 (1H, d, J=8.0 Hz), 8.26 (1H, d.J=8.0 Hz), 8.99 (1H, br s), 9.30 (1H, br s). | Melting Point >210 (dec). (°C). Elemental Analysis as C₂₆H₂₈N₂O₄· 1.5CH₃SO₃H· 0.3H₂O Calculated: C, 58.46: H. 5.75; N. 4.62; S. 7.94. Found: C, 58.42; H. 5.86; N. 4.59; S. 7.86. IR (cm⁻¹) (KBr) 3400, 1638, 1508, 1962, 1328, 1201. 1044, 926, 903. 872, 837, 774. 555. Mass (FAB) 457 ((M+H)'). |
| 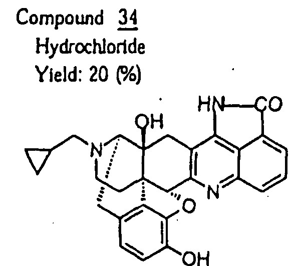 | NMR (ppm) (300 MHz, CD₃OD) 0.55-0.65 (m, 2H), 0.80-1.00 (m,2H),1.17-1.30 (m, 1H), 2.04-2.12 (m, 1H), 2.84 (d, 1H, J=16.8Hz), 2.84-2.95 (m. 1H), 3.03-3.15 (m, 2H), 3.17 (d, 1H, J=16.8Hz), 3.28-3.45 (m, 2H). 3.51 (dd, 1H, J-13.2. 7.2Hz), 3.63 (d, 1H, J=19.8Hz), 4.30-4.37 (m, 1H), 5.77 (s, 1H), 6.76 (d, 1H, J=8.1Hz). 6.83 (d, 1H, J=8.1Hz), 7.95-8.09 (m. 2H), 8.16-8.22 (m. 1H), | Melting Point 235 (°C). Elemental Analysis as C₂₈H₂₆N₃O₄·1.25HCl· 1.0H₂O Calculated: C. 63.32; H, 5.36; N, 7.91. Cl. 8.34. Found: C. 63.23: H, 5.42: N. 7.94: Cl, 8.37. IR (cm⁻¹) (KBr) 1725, 1647, 1508, 1485, 1323. 1249, 1158, 1116, 1064, 1027, 926, 884. Mass (FAB) 468 ((M+H)'). |
| 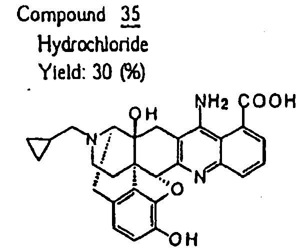 | NMR (ppm) (300 MHz, CD₃OD) 0.55-0.68 (2H, m), 0.80-0.95 (2H, m), 1.13.1.30 (1H, m), 2.00-2.10 (1H, m), 2.52 (1H, d, J=16.5 Hz), 2.80-3.18 (3H, m), 2.93 (1H, d, J =16.5 Hz), 3.30-3.67 (4H, m). 4.36 (1H. d. J=6.6 Hz), 5.80 (1H, s), 6.78 (1H. d, J..8.4 Hz), 6.83 (1H. d. J=8.4 Hz), 7.75-7.85 (3H, m). | Melting Point 215 (°C). Elemental Malysis as C₂₈H₂₇N₃O₅· 1.2HCl·3.3H₂O Calculated: C. 57.12; H, 5.96: N. 7.14: Cl, 7.23. Found: C, 57.26: H, 5.94: N. 6.92: Cl. 7.24. IR (cm⁻¹) (KBr) 3400. 1620, 1572. 1493, 1462, 1383, 1282, 1251. Mass (FAB) 486 ((M+H)⁺). |
| 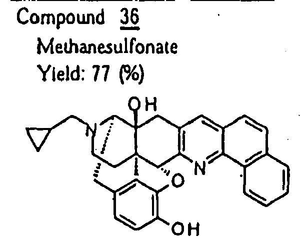 | NMR (ppm) (300 MHz, DMSO-d₆) 0.91-0.56 (2H, m). 0.60-0.79 (2H, m), 1.12 (1H, m), 1.92 (1H, d. J=12.1 Hz), 2.30 (3.3H, s), 2.64-3.00 (3H, m), 2.88 (1H, d. *J*=16.5 Hz), 3.07 (1H, d, J=17.0 Hz), 3.14-3.21 (2H, m), 3.39-3.51 (1H, m), 3.52 (1H, d, J=20.0 Hz), 4.12 (1H, d, J=6.3 Hz), 5.83 (1H, s), 6.47 (1H, br s), 6.65 (2H, s), 7.75-7.85 (2H, m), 7.84 (1H, d, J=9.1 Hz). 7.96 (1H, d, J=8.8 Hz), 8.04,8.07 (1H, m), 8.16 (1H, s), 9.01 (1H, br s), 9.18-9.21 (1H, m), 9.34 (1H, br s). | Melting Point >250 (dec). (°C). Elemental Malysis as C₃₁H₂₈N₂O₃· 1.1CH₃SO₃H·1.5H₂O Calculated: C, 63.28; H, 5.86: N, 4.60; S, 5.79. Found: C, 63.31; H, 5.57:N, 4.53: S, 5.98. IR (cm⁻¹) (KBr) 3400, 1638, 1622, 1508, 1466, 1904, 1328, 1205, 1120, 1044, 818, 758, 553, 526. Mass (FAB) 477 ((M+H)⁺). |
| 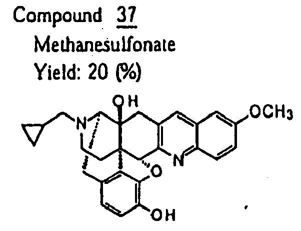 | NMR (ppm) (300 MHz, DMSO-d₆) 0.40-0.55 (2H, m), 0.59-0.78 (2H, m), 1.10 (1H, m), 1.87 (1H, d, J= 10,4 Hz), 2.31 (3.9H, s), 2.56-2.87 (2H, m). 2.79 (1H, d, J=15.9 Hz), 2.90-3.02 (1H, m). 3.00 (1H, d, J=16.8 Hz), 3.10-3.25 (2H. m), 3.36-3.99(1H, m), 3.49 (1H, d, J=19.5 Hz), 3.89 (3H, s). 4.09 (1H, d, J=6.0 Hz), 5.65 (1H, s), 6.42 (1H, br s). 6.64 (1H, s). 6.64 (1H, s). 7.33 (1H, d. J=2.7 Hz), 7.42 (1H, dd, J=9.1. 2.7 Hz), 7.95 (1H, d, J=9.1 Hz), 8.00 (1H, s). 8.99 (1H, br s), 9.33 (1H, br s). | Melting Point 232-238 (dec) (°C). Elemental Analysis as C₂₈H₂₈N₂O₄· 1.3CH₃SO₃H· 0.4H₂O Calculated: C, 59.78: H, 5.82; N, 4.76: S. 7.08. Found: C, 59.67; H, 6.04; N, 4.80: S, 6.97. IR (cm⁻¹) (KBr) 3400. 1626, 1499. 1466, 1423, 1388, 1330, 1274, 1195, 1114, 1060, 785, 555. Mass (EI) 456 (M') (data of salt-free compound) |
| 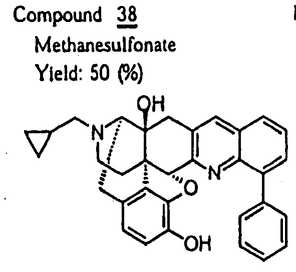 | NMR (ppm) (300 MHz, CD₃OD) 0.47-0.60 (2H, m), 0.73-0.95 (2H, m), 1.07-1.20 (1H, m), 1.90-2.00 (1H, m), 2.68 (3H, s). 2.76 (1H, td, J= 13.2 5.1 Hz), 2.92-3.09 (4H. m), 3.15-3.31 (2H, m), 3.40 (1H, dd. J= 13.2, 7.2 Hz), 3.50 (1H, d. J=20.1 Hz), 4.20 (1H, d. J=6.9 Hz), 5.66 (1H, s), 6.67 (1H, d. J=8.1 Hz), 6.71 (1H, d. J=8.1 Hz), 7.47-7.52 (3H, m), 7.63 (1H, dd, J=8.1, 7.2 Hz), 7.72-7.86 (4H, m). 8.09 (1H, s). | Melting Point 240 (°C), Elemental Analysis as C₃₃H₃₀N₂O₃ · 1.0MeSO₃H· 1.0H₂O Calculated: C, 66.22: H, 5.88; N, 4.54; S. 5.20. Found: C. 66.18. H, 5.93: N, 4.59: S. 5.39. IR (cm⁻¹) (KBr) 3400. 1636, 1508. 1462, 1328. 1193, 1120, 1060. 806, 774. Mass (EI) 502 (M⁺) (data of salt-free compound) |
| 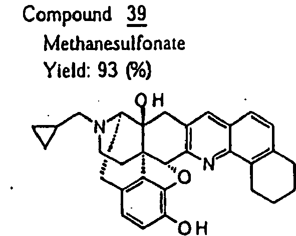 | NMR (ppm) (300 MHz. DMSO-d₆) 0.40-0.55 (2H, m). 0.58-0.78 (2H, m). 1.10 (1H. m), 1.70-1.94 (4H, m). 2.31 (3.3H, s). 2.57-3.03 (6H, m), 2.79 (1H, d. J=16.5 Hz), 2.98 (1H. d. J=16.8 Hz), 3.11-3.30 (4H, m), 3.33-3.49 (1H, m), 3.50 (1H, d. J=20.0 Hz), 4.07 (1H, d, J=6.3 Hz), 5.67 (1H. s). 6.38 (1H, br s). 6.64 (1H, s), 6.64 (1H, s). 7.31 (1H, d. J=8.5 Hz), 7.65 (1H, d, J=8.5 Hz), 7.99 (1H, s). 8.99 (1H. br s). 9.35 (1H, br s). | Melting Point >230 (dec) (°C). Elemental Analysis as C₃₁H₃₂N₂O₃·1.3CH₃SO₃H·0.4H₂O Calculated: C. 63.31; H, 6.25; N. 4.57; S. 6.80. Found: C, 63.35: H, 6.35: N, 4.65: S. 6.75. IR (cm⁻¹) (KBr) 3400, 1638. 1460. 1330, 1210. 1195. 1118. 1060, 816, 785, 557. Mass (EI) 480 (M⁺) (data of salt-free compound) |
| 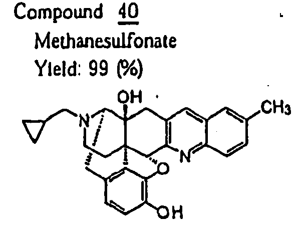 | NMR (ppm) (300 MHz. CD₃OD) 0.50-0.6.1 (2H, m), 0.74-0.92 (2H, m), 1.12-1.22 (1H. m). 2.03-2.10 (1H, m), 2,61 (3H, s), 2.69 (5.4H, s), 2.81-3.12 (5H, m), 3.28-3.38 (2H, m), 3.45 (1H, dd, J=10.8, 7.5 Hz), 3.57 (1H, d, J=20.1 Hz). 4.33 (1H, d, J.6.6 Hz), 6.02 (1H, s). 6.73 (1H, d, J-8.4 Hz), 6.81 (JH. d, 1-8.4 Hz), 7.97 (1H, s). 7.98 (1H, d, J-8.7 Hz), 8.18 (1H, d, 1=8.7 Hz), 8.78 (1H, s). | Melting Point 220 (°C). Elemental Analysis as C₂₈H₂₈N₂O₃· 1.8MeSO₃H· 1.9H₂O Calculated: C, 55.26; H, 6.07; N. 4.32; S. 8.91. Found: C, 55.37: H, 6.02: N. 4.17; S. 8.90. IR (cm⁻¹) (KBr) 3500, 1738, 1713. 1622. 1499, 1460. 1437. 1383. 1193, 1060. Mass (EI) 440 (M⁺) (data of salt-free compound) |
| 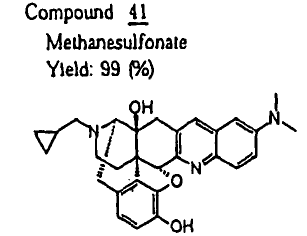 | NMR (ppm) (300 MHz, CD₃OD) 0.54-0.66 (2H, m)., 0.80-0.89 (1H, m). 0,90-0.98 (1H. m), 1.15-1.28 (1H, m), 2.05-2.12 (1H, m), 2.74 (5.4H. s), 2.82-2.95 (1H, m), 3.01 (1H, d. J=16.2 Hz), 3.05-3.17 (2H, m), 3.21 (6H, s), 3.22 (1H, d, J=16.2 Hz), 3,30-3.41 (2H, m), 3.49 (1H, dd. J=13.8, 7.2 Hz), 3.60 (1H, d, J=20.1 Hz). 4.34 (1H, d, J=6.6 Hz), 5.94 (1H, s), 6.78 (1H. d, J=8.2 Hz), 6.84 (1H. d, J=8.2 Hz). 7.06 (1H, d, J=2.7 Hz). 7.83 (1H, dd, J=9.9. 2.7 Hz), 8.09 (1H, d, J=9.9 Hz), 8.47 (1H, s). | Melting Point 245 (°C). Elemental Analysis as C₂₉H₃₁N₃O₃· 1.8MeSO₃H·1.3H₂O Calculated: C, 55.55. H. 6.17; N. 6.31: S. 8.67. Found: C, 55.47; H, 5.91; N, 6.23; S. 8.88. IR (cm⁻¹) (KBr) 3500, 1620, 1510, 1960, 1205. Mass (EI) 469 (M⁺) (data of salt-free compound) |
| 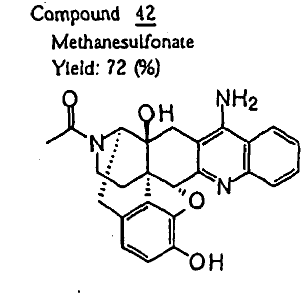 | NMR (ppm) (600 MHz, CD₃OD) 1.72 (dd, *J*=12.6, 2.9 Hz, 0,4H), 1.77 (dd, *J*=12.6, 2.9 Hz, 0.6H), 2.20 (s, 1.8 H), 2.25 (s, 1.2 H), 2.42 (d, *J*=16.3 Hz, 0.4H), 2.43 (d, *J*=16.3 Hz, 0.6H), 2.46 (td, *J*=12.6*,* 5.3 Hz, 0.4H), 2.59 (td, *J*=12.6, 5.3 Hz, 0.6H), 2.70 (s. 0.8H). 2.78 (td, *J= 13.6.* 3.8 Hz, 0.4H). 2.84 (d, *J*=16.3 Hz 1H), 2.98 (d, *J*=18.9 Hz, 0.6H), 3.13 (d, *J=*18.9 Hz, 0.4H), 3.30 (td, *J=*13.6, 3.8 Hz, 0.6H), 3.34 (dd. *J*=18.9*,* 6.8 Hz. 0.6H), 3.43 (dd. *J*=18.9, 6.8 Hz, 0.4H), 3.89 (dd, *J*=13.6*.* 5.3 Hz, 0.6H), 4.41 (d, *J*=6.8 Hz, 0.4H). 4.58 (dd, *J*=13.6*.* 5.3 Hz. 0.4H). 5.21 (d, *J*=6.8 Hz, 0.6H). 5.60 (s, 0.4H). 5.61 (s, 0,6H), 6.66-6.71 (m. 2H). 7.62-7.67 (m, 1H), 7.87-7.95 (m, 2H). 8.30 (d, *J*=8.4 Hz**,** 0.6H), 8.31 (d, *J*=8.4 Hz, 0.4H) | Melting Point 280-285 (°C). Elemental Analysis as C₂₅H₂₃N₃O₄·0.8CH₃SO₃H·2.1H₂O Calculated: C. 56.94; H, 5.63; N. 7.72; S, 4.71. Found: C. 56.80; H, 5.42; N. 7.63; S. 5.04. IR (cm⁻¹) (KBr) 3410. 1640, 1599. 1502, 1460. 1321. 1280. 1203, 1050 Mass (FAB) 430 ((M+H)⁺). |
| 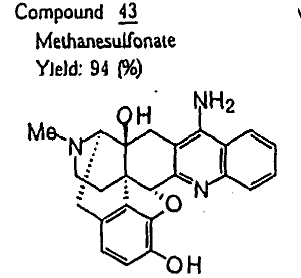 | NMR (ppm) (400 MHz, DMSO-d₆) 1.84-1.94 (m, 1H), 2.33 (s, 5.7H), 2.28-2.38 (m, 1H). 2.56-2.69 (m, 1H), 2.75-2.88 (m, 1H). 2.90 (d, J=17.1 Hz, 1H), 2.92-3.04 (m. 3H), 3.12 (dd. J=20.0, 6.4 Hz. 1H), 3.18-3.26 (m, 1H), 3.60 (d, J=20.0 Hz, 1H). 3.84 (d, J=6.4 Hz, 1H), 5.78 (s, 1H). 6.62 (br s, 1H), 6.71 (d, J=8,3 Hz, 1H), 6.74 (d, J=8.3 Hz, 1H). 7.64-7.73 (m, 1H), 7.93-8.03 (m, 2H). 8.46 (br s, 1 H), 8.50 (d, J=8.3 Hz, 1H), 9.22 (br s, 1H). 9.32 (br s, 1H), 9.53 (br s, 1H). 14.1 (br s. 1H). | Melting Point 256-265 (°C). Elemental Analysis as C₂₄H₂₃N₃O₃· 2.0CH₃SO₃H· 0.2H₂O·0.5EtOAc Calculated: C, 52.44; H, 5.56; N. 6.55; S, 10.00. Found: C, 52.25; H, 5.70; N, 6.58; S, 10.08. IR (cm⁻¹) (KBr) 3400. 1649, 1597, 1504, 1475, 1330, 1199. 1060, 1052. 785. Mass (FAB) 402 ((M+H)⁺). |
| 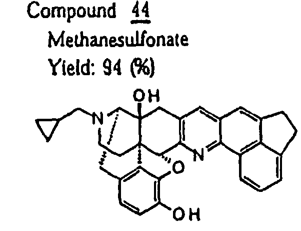 | NMR (ppm) (300 MHz, DMSO-d₆) 0.41-0.57 (2H, m). 0.59-0-79 (2H, m). 1.12 (1H, m), 1.91 (1H. d, J=11.3 Hz), 2.32 (4.3H, s), 2.61-3.03 (3H, m), 2.86 (1 H. d, J=16.5 Hz), 3.03 (1H, d. J-16.8 Hz). 3.12-3.27 (2H. m), 3.37-3.55 (6H. m), 4.10 (1H, d, J=6.0 Hz), 4.17 (1H, br s), 5.81 (1H, s), 6.46 (1H, br s), 6.65 (1H, s). 6.65 (1H, s), 7.57 (1H, s), 7.64 (1H, d. J=6.9 Hz), 7.76 (1H, m), 8.06 (1H, s), 8.63 (1H, d, J=8.0 Hz), 9.00 (1H, br s). | Melting Point >260 (dec) (°C). Elemental Analysis as C₃₃H₃₀N₂O₃· 1.45CH₃SO₃H·0.9H₂O Calculated: C. 62.87: H. 5.76, N, 4.26. S. 7.06. Found: C, 62.71; H, 6.03; N, 4.09; S, 7.26. IR (cm⁻¹) (KBr) 3900. 1626, 1510, 1466, 1423, 1379. 1325. 1199. 1118, 1052, 785, 561. Mass (EI) 502 (M⁺) (data of salt-free compound) |
| 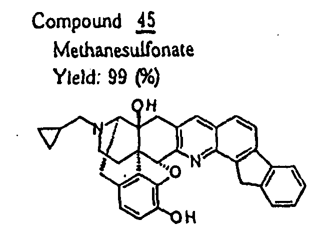 | NMR (ppm) (300 MHz, CD₃OD) 0.53-0.66 (2H, m), 0.80-1.00 (2H, m). 1.18-1.20 (1H, m), 2.06-2.15 (1H, m), 2.74 (3.8H, s), 2.80-3.08 (5H, m), 3.25-3.40 (2H, m), 3.49 (1H, dd, J=12.9, 7.2 Hz), 3.62 (1H, d, J=19.5 Hz), 4.15-4.30 (3H, m), 5.85 (1H, s), 6.81 (1H, d. J=8.4 Hz), 6.86 (1H. d, *J*=8.4 Hz). 7.33.7.43 (2H, m). 7.55 (1H, *d*. J=6.3 Hz), 7.80-7.87 (2H. m). 8.03 (1H, d, J=8.4 Hz), 8.09 (1H, s). | Melting Point 240 (°C). Elemental Analysis as C₃₁H₃₀N₂O₃· 1.25MeSO₃H•0.5H₂O Calculated:C, 65.77; H, 5.64; N. 4.35; S. 6.23. Found: C. 65.76: H. 5.67: N. 4.31: S. 6.15. IR (cm⁻¹) (KBr) 3450, 1642, 1613, 1508, 1464, 1330, 1210, 1118. 1048. Mass (FAB) 515 ((M+H)⁺). |
| 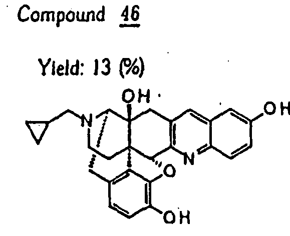 | NMR (ppm) (300 MHz, CD₃OD) 0.13.0.24 (2H, m), 0.49-0.62 (2H, m), 0.92 (1H, m), 1.68-1.77 (1H, m), 2.29-2.62 (4H, m). 2.65-2.82 (4H, m), 3.20 (1H, d. J=18.1 Hz), 3.36 (1H, d, J=6.3 Hz), 5.48 (1H, s), 6.58 (1H, s), 6.58 (1H, s), 6.98 (1H, s), 7.22-7.28 (1H, m), 7.69 (1H, s), 7.85 (1H, d, J-8.8 Hz). | Mass (EI) 442 (M⁺) |
| 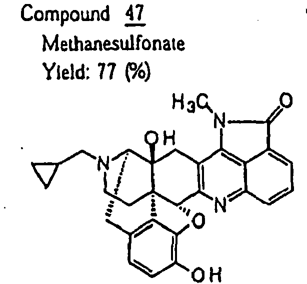 | NMR (ppm) (300 MHz, CD₃OD) 0.50-0,62 (2H, m), 0.76-0.98 (2H, m). 1.15-1.24 (1H. m), 2.02-2.10 (1H, m), 2.68 (6.2H, s), 2.82.2.95 (1 H, m), 2.99-3.13 (3H, m), 3.30-3.50 (3H, m). 3.58 (1H, d. J=20.4Hz), 3.65 (3H, s), 3.78 (1H, d, *J*= 17.1 Hz), 4.43 (1H, d, J=6.9Hz), 5.98 (1H, s), 6.74 (1H, d, J=8.4Hz), 6.83 (1H, d, J=8.9Hz), 8.15-8.30 (3H, m). | Melting Point >220 (dec) (°C). Elemental Analysis as C₂₉H₂₇N₃O₄· 2.05MeSO₃H· 0.6H₂O Calculated: C, 54.10; H, 5.32; S, 9.54: N, 6.10. Found: C, 59.19; H, 5.37; S, 9.55; N, 5.93. IR (cm⁻¹) (KBr) 3400. 1742, 1638, 1491, 1402, 1195, 1120, 1060, 1011, 936, 882. Mass (FAB) 482 ((M+H⁺). |
| 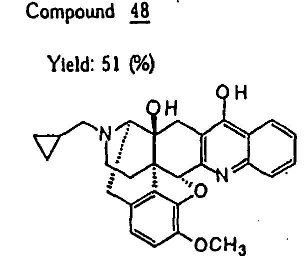 | NMR (ppm) (300 MHz, CDCl₃) 0.16-0.22 (m, 2H), 0.55-0.64 (m, 2H), 0.91 (m, 1H), 1.68 (br s. 1H, OH), 1.79 (m, 1H), 2.26 (d, 1H, *J*=17.0 Hz), 2.80-2.53 (m, 4H), 2.75-2.97 (m. 2H), 3.07 (d. 1H, *J*=17.00 Hz), 3.16 (d, 1H, *J=*17.7 Hz), 3.42 (d, 1H, *J*=6.3 Hz), 3.7 4 (s. 3H), 5.45 (s, 1H), 6.67 (s, 2H). 7.13 (d, 1H, *J*=8.0 Hz), 7.17 (dd, 1H. *J=*8.0, 8.0 Hz). 7.45 (dd. 1H. J=8.0 Hz), 8.15 (d. 1H, J=8.0 Hz), 8.86 (br s, 1H, OH), | Mass (EI) 456 (M') |
| 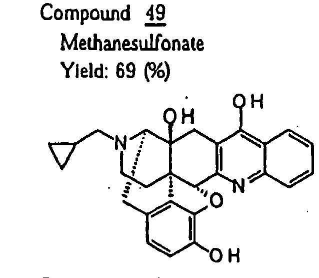 | NMR (ppm) (500 MHz. DMSO-d₆) 0.44 (m, 1H), 0.49 (m, 1H), 0.63 (m, 1H), 0.74 (m, 1H), 1.09 (m, 1H), 1.83 (br d, 1H, *J*=10.7 Hz), 2.02 (d, 1H, *J*=17.1Hz), 2.32 (s, 3H), 2.61 (ddd, 1H, *J*=13.2, 13.2. 4.6 Hz), 2.74 (m, 1H), 2.93 (m, 1H), 3.03 (d, 1H. *J*=17.1 Hz), 3.13 (m, 1H), 3.25 (dd, 1H, *J*= 19.8, 6.6 Hz), 3.39 (m. 1H), 3.44 (d. 1H, J=19.8 Hz), 4.11 (d, 1H, *J*=6.6 Hz), 5.55 (s, 1H), 6.36 (br s, 1H, OH), 6.64 (d, 1H, *J*=8.1 Hz), 6.69 (d, 1H, *J*=8.1 Hz), 7.29 (m, 1H). 7.64-7.69 (m, 2H), 8.04 (d. 1H, *J*=8.0 Hz), 8,93 (m, 1H, NH+), 9.40 (br s. 1H, OH). 12.10 (s, 1H, OH). | Melting Point 255-285 (°C). Elemental Analysis as C₂₇H₂₆N₂O₄·1.2MeSO₃H· 1.1H₂O Calculated: C, 58.63; H. 5.76; N. 4.85: S, 6.66. Found: C, 58.77: H, 5.67; N, 4.66: S, 6.78. IR (cm⁻¹) (KBr) 3350, 3252, 1636, 1613, 1555. 1512, 1479. 1421. 1367. 1328, 1195, 1120, 1050, 926. 886, 812, 768. Mass (FAB) 443 ((M+H)'). |
| 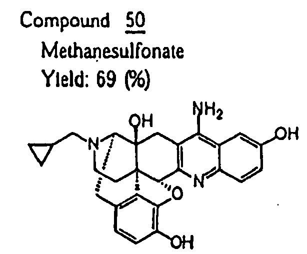 | NMR (ppm) (300 MHz, CD₃OD) 0.50-0.62 (2H, m). 0.76-0.98 (2H, m), 1.15-1.24 (1H, m), 2.02-2.10 (1H, m), 2.68 (6.2H, s), 2.82-2,95 (1H, m), 2.99-3.13 (3H, m), 3.30-3.50 (3H, m), 3.58 (1H, d. 1-20.4 Hz). 3.65 (3H, s) 3.78 (1H, d, J=17.1 Hz). 4.43 (1H, d, J=6.9 Hz). 5.98 (1H, s), 6.74 (1H, d, J-8.4 Hz), 6.83 (1H, d. J-8.4 Hz). 8.16-8.30 (3H, m). | Melting Point >210 (dec) (°C). Elemental Analysis as C₂₇H₂₇N₃O₄· 2.0MeSO₃H· 1.2H₂O Calculated: C, 51.88; H, 5.62; S. 9.55: N. 6.26. Found: C, 51.93; H. 5.59: S. 9.52; N, 6.14. IR (cm⁻¹) (KBr) 3900. 3250. 1651. 1622. 1597. 1506, 1642. 1408, 1330, 1195, 1120. Mass (FAB) 458 ((M+H)⁺). |
| 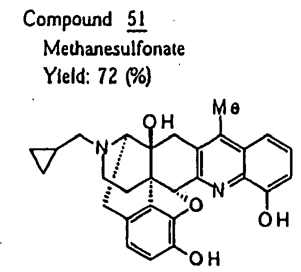 | NMR (ppm) (500 MHz, DMSO-d₆) 0.46 (m. 1H), 0.52 (m, 1H), 0.64 (m, 1H), 0.75 (m, 1H), 1.11 (m, 1H). 1.91 (br d, 1H, *J*=10.3 Hz), 2.31 (s, 3H), 2.52 (s. 3H), 2.63 (d. 1H, *J*=17.3 Hz), 2.65 (m, 1H), 2.83 (m. 1H), 2.98 (m, 1H), 3.16 (m, 1H), 3.21 (d, 1H, *J*=17.3 Hz), 3.24 (dd. 1H, *J*=19.8. 7.0 Hz), 3.43 (m, 1H), 3.51 (d. 1H, J=19.8 Hz). 4.14 (d, 1H. 7=6.4 Hz), 5,79 (s, 1H), 6.35 (br s, 1H, OH), 6.65 (s, 2H). 7.12 (d. 1H. *J*=7.5 Hz), 7.48 (dd. 1H, J=8.3. 7.5 Hz). 7.55 (d. 1H, J=8.3 Hz), 9.01 (m, 1H, NH+). 9.31 (br s. 1H, OH), 9.78 (br s, 1H, OH). | Melting Point 235-270 (°C). Elemental Analysis as C₂₈H₂₈N₂O₄·1.2MeSO₃H· 1.1H₂O Calculated: C, 59.27; H, 5.96; N, 4.73, S. 6.50. Found: C, 59.28; H. 6.05; N, 9.44; S, 6.58. IR (cm⁻¹) (KBr) 3376, 1638. 1508. 1468. 1421, 1363. 1328. 1203. 1178. 1122, 1044, 977. 808. 774. Mass (FAB) 457 ((M+H)⁺). |
| 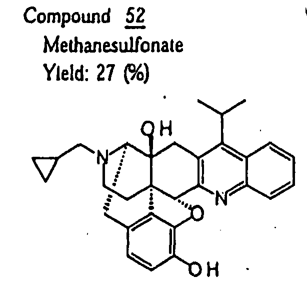 | NMR (ppm) (300 MHz. DMSO-d₆) 0.40.0.58 2H, m), 0.60-0.70 (1H, m), 0.70-0.81 (1H, m), 1.10-1.18 (1H, m), 1.42 (3H, d. J=7.1 Hz). 1.45-1.56 (3H, m), 1.82-1.92 (1H. m), 2.33 (6.4H, s), 2.57-2.65 (1H,m), 2.66 (1H, d. J=17.0 Hz), 2.70-2.90 (1H, m), 2.93-3.05 (1H, m), 3.12-3.32 (2H, m), 3.29 (1H, d J=17.0 Hz), 3.35-3.42 (1H, m). 3.44 (1H, d J=15.7 Hz), 3.62 (1H, br s). 9.12-4.20 (1H, m). 5.75 (1H, s), 6.37 (1H, br s), 6.66 (2H, s). 7.65 (1H, dd J=7.1. 7.4 Hz), 7.81 (1H, dd J=7.4, 8.2 Hz), 8.09 (1H, d J=7.1 Hz), 8.37 (1H, d J=8.2 Hz), 9.00 (1H, br s). | Melting Point 210 (°C). Elemental Analysis as C₃₀H₃₂N₂O₃·2.1CH₃SO₃H·0.5MeOH Calculated: C, 57.04; H, 6.23: N. 4.08; S, 9.81. Found: C, 57.19; H. 6.05; N. 4.06: S. 9.59. IR (cm⁻¹) (KBr) 3380. 1638, 1509. 1462, 1328, 1291, 1210. 1195, 1060. 785. Mass (EI) 468 (M⁺) (data of salt-free compound) |
| 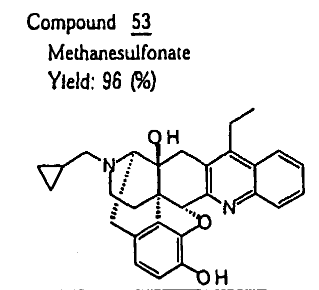 | NMR (ppm) (300 MHz, DMSO-d₆) 0.40.0.58 (2H, m), 0.60.0.70 (1H. m). 0.71-0.82 (1H, m). 1.08-1.15 (1H, m), 1.20 (3H, t, J=7,4 Hz), 2.82-2.94 (1H, m), 2.36 (8H, s), 2.60-2.70 (1H, m), 2.70 (1H, d, J=17.6 Hz), 2.72.2.90 (1H, m), 2.95-3.10 (2H, m); 3.10-3.30 (3H, m), 3.27 (1H, d J=17.6 Hz). 3.32-3.45 (1H, m), 3.52 (1H, d J=19.5 Hz), 9.20-4.22 (1H, m). 5.88 (1H, s). 6.40 (3H. br s), 6.68 (2H, s). 7.80 (1H, dd J=7.4. 8.2 Hz), 7.96 (1H, dd J=7.4. 7.6 Hz). 8.19 (1H, d J=8.2 Hz). 8.30 (1H, d J=7.6 Hz). 9.05 (1H, br s). | Melting Point 163 (°C). Elemental Analysis as C₂₉H₃₀N₂O₃·2.6CH₃SO₃H·0.7H₂O Calculated: C. 52.93; H, 5.88; N. 3.91; S. 11.63. Found: C. 52.83; H, 5.75; N, 3.88; S, 11.86. IR (cm⁻¹) (KBr) 3400, 1638, 1615, 1574, 1509, 1960, 1927, 1394, 1339, 1284, 1245, 1154, 1122. 1060. 1035. Mass (EI) 454 (M⁺) (data of salt-free compound) |
| 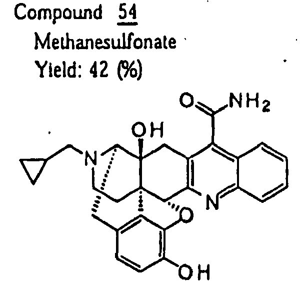 | NMR (ppm) (300 MHz, CD₃OD) 0.52-0.57 (2H, m), 0.74-0.91 (2H, m). 1.15 (1H, m), 2.01-2.06 (1H, m), 2.68 (3H, s), 2.77-2.88 (1H, m), 2.92-3.17 (4H, m), 3.21.3.27 (1H, m). 3.32-3.34 (1H, m). 3.39-3.46 (1H. m). 3.53 (1H, d, J=19.5 Hz), 4.27 (1H, d, J=6.3 Hz). 5.71 (1H, s), 6.66 (1H, d, J=8.2 Hz), 6.74 (1H, d. J=8.2 Hz), 7.65-7.70 (1H, m), 7.80-7.85 (1H, m), 7.89-7.91 (1H, m). 8.13 (1H, d, J=8.5 Hz). | Melting Point >230. (°C). Elemental Analysis as C₂₈H₂₇N₃O₄·CH₃SO₃H·0.8H₂O Calculated: C. 60.05; H, 5.66: N, 7.24; S, 5.53. Found: C, 60.00; H, 5.70; N. 7.17; S. 5.76. IR (cm⁻¹) (KBr) 3400, 1665, 1504, 1462, 1330. 1197, 1060. 783, 774, 563, 536. Mass (FAB) 470 ((M+H)⁺). |
| 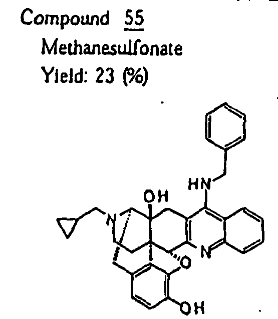 | NMR (ppm) (300 MHz, DMSO-d₆) 0.91-0.55 (2H, m), 0.60-0.79 (2H, m), 1.07 (1H, m). 1.85-1.94 (1H, m), 2.35 (7H, s), 2.46.2.68 (2H, s), 2.72-2.85 (1H m), 2.97-3.26 (4H, m), 3.33-3.42 (1H, m), 3.48-3.58 (1H, m), 4.04 (1H, brs), 4.16 (1N, d, J=6.3 Hz), 4.92 (1H, d, J=17.3 Hz), 5.08 (1IH, d, J=17.3 Hz), 5.77 (1H, s), 6.65 (1H, br s), 6.72 (1H, s), 6.72 (1H, s), 7.22-7.36 (5H, m). 7.48-7.55 (1H, m), 7.89-7.98 (2H, m), 8.14 (1 H, m), 8.86 (1 H, br s), 9.10 (1 H, br s). 9.54 (1H, br s). | Melting Point 185-191 (°C). Elemental Analysis as C₃₄H₃₃N₃O₃· 2.4CH₃SO₃H· 0.6H₂O Calculated: C, 56.55; H. 5.71; N, 5.44; S, 9.95. Found: C, 56.72; H, 5.78; N. 5.41; S, 9.94. IR (cm⁻¹) (KBr) 3300, 1638, 1578, 1535, 1510, 1460, 1421, 1330. 1199, 1044. 785, 774, 536. Mass (FAB) 532 ((M+H)⁺). |
| 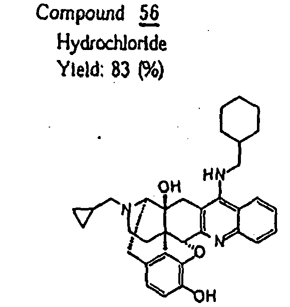 | NMR (ppm) (300 MHz. CD₃OD) 0.50.0.60 (2H, m). 0.72-1.01 (4H, m). 1.05-1.30 (4H, m), 1.45-1.95 (7H, m), 2.00-2.08 (1H, m), 2.64 (1H, d, J=16.5 Hz), 2.80 (1H, td. J=13.5, 4.9 Hz), 3.00 (1H, d, J=16.5 Hz), 3.05-3.10 (2H, m), 3.25-3.50 (2H, m), 3.50-3.65 (2H, m), 3.79 (1H, dd, J=14.1. 5.8Hz). 4,37 (1H,d, J=6.0 Hz), 5.75 (1H,s), 6.74 (1H, d, J=8.2 Hz), 6.81 (1H, d,J=8.2 Hz), 7.60-7.67 (1H, m), 7.93 (2H, d, J=3.6 Hz). 8.36 (1H, d. J=8.5 Hz). | Melting Point >200 (dec) (°C). Elemental Analysis as C₃₄H₃₉N₃O₃· 1.9HCl·0.7H₂O Calculated: C, 65.91; H, 6.88; Cl, 10.87: N, 6.78. Found: C, 65.92; H, 7.06; Cl, 10.97; N, 6.65. IR (cm⁻¹) (KBr) 3900, 2932, 1639, 1609, 1576, 1526, 1508, 1460, 1359. 1330, 1259, 1183, 160, 1125, 1112, 1065, 1029. 926. Mass (FAB) 538 ((M+H)⁺). |
| 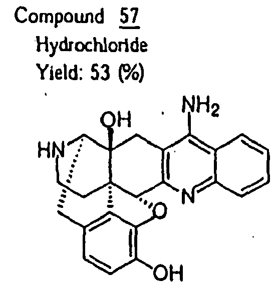 | NMR (ppm) (300 MHz, CD₃OD) 1.96-2.02 (1H, m). 2.40-2,46 (1H, m), 2.75-2.82 (1H, m), 2.90.2.96 (1H, m),3.01-3.15 (1H, m),3.29-3.30 (2H, m), 3.43-3.52 (1H, m), 4.01 (1H, d, J-6.0 Hz), 5.73 (1H, s), 6.71-6.79 (2H, m), 7.62-7.69 (1H, m), 7.92-7.94 (2H. m), 8.31-8.34 (1H, m). | Melting Point >235 (dec) (°C). Elemental Analysis as C₂₃H₂₁N₃O₃· 2HCl· 1.35H₂O· 0.25CH₃OH Calculated: C. 57.03; H, 5.50; Cl, 14.48; N. 8.58. Found: C. 56.76; H, 5.78; Cl, 14.86; N. 8.50. IR (cm⁻¹) (KBr) 3400, 1651. 1502, 1468 1431. 1375, 1319, 1247, 1162. 1085, 1096, 925, 799, 762. Mass (FAB) 388 ((M+H)'). |
| 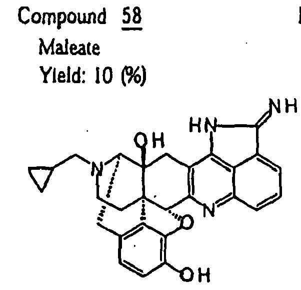 | NMR (ppm) (300 MHz, CD₃OD) 0.55-0.66 (2H, m). 0.80-0.99 (2H, m), 1.17-1.24 (1H, m), . 2.03-2.08 (1H, m), 2.80.2.92 (2H, m), 2.86 (1H, d, J=17.1 Hz), H 3.03-3.15 (2H, m), 3.25-3.40 (2H, m). 3.36 (1H, d. J=17.1 Hz), 3.47 (1H, dd, J=13.8. 7.5 Hz), 3.60 (1H, d, J=20.1 Hz), 4.31 (1H, d. J=6.3 Hz), 5.71 (1H, s), 6.74 (1H, d, J=7.8 Hz), 6.80 (1H, d, J=7.8 Hz), 7.92 (1H, dd, J=8.9. 6.9 Hz), 8.20 (1H, d. J=8.4 Hz), 8.21 (1H, d.J=8.9 Hz). | Melting Point >190 (dec) (°C). IR (cm⁻¹) (KBr) 3300, 1470, 1357. 1280. 1251, 1176, 1116. 1067. 1033. Mass (FAB) 467 ((M+H)*). |

### [Example 46]

### Infarction inhibiting action in rat model of middle cerebral artery ischemia

It is the well known fact that, in the acute stage of human cerebral infarction, significant cerebral edema is caused by cerebral ischemia accompanied with a grave lesion of the intracerebral blood vessels, and that when the blood flow is reopened in the acute stage of cerebral infarction, cerebral edema is significantly worsened. It is also known that, in this way, in the acute stage of cerebral infarction, the lesion proceeds to the peripheral tissue from the core of infarction, and death of the nerve cells is extended with the passage of several days. This possibly not only extends and makes grave aftereffects, and causes loss of motor and mental function, but also finally causes the critical influences on the life. As an in vivo experimental model of cerebral infarction which is capable of precisely evaluating the clinical effect of a medicine in conformity with clinical conditions of the disease of a patient of cerebral infarction, the middle cerebral artery occlusion (MCAo)-recirculation model comprising an embolus with a yarn using Wister rats is known [Document: Japan Journal of Stroke, vol. 8, 1 (1986)]. It is apparent that, in this model, a compound exhibiting the infarction inhibiting action is useful as an agent for curing·preventing cerebral stroke, traumatic cerebral diseases, cerebral edema, and cerebral neurodegenerative diseases. This action was evaluated by applying the MCAo model by the method which will be described below.

In rats of 10 weeks old, after etherization, a median incision of the cervical region was made up to the right carotid artery bifurcation under 1.0% halothane anesthetization with care to preserve the vagus nerves. The common carotid artery and the external carotid artery were separated from the periphery connective tissue with the right carotid artery bifurcation as the center, and each of the arteries was ligated by a 6-0 silk yarn (Eto yarn). Further, a yarn was wound on the internal carotid artery origin in preparation for ligation and fixing after insertion of the embolus. Next, the common carotid artery was incised, and the embolus was inserted from the common carotid artery to the internal carotid artery by about 15 to 16 mm, and ligated and fixed to the internal carotid artery by the silk yarn at the end thereof near the nylon yarn. In this operation, the end of the embolus was inserted into the anterior cerebral artery by about 1 to 2 mm beyond the middle cerebral artery bifurcation, and the inlet of the middle cerebral artery was occluded by the body (resin part) of the embolus for 1 hour. In recirculation, the embolus with a yarn was removed to recirculate the blood flow to the middle cerebral artery. 0.3 mg/kg or 3 mg/kg of each of test compounds was intraperitoneally administered 10 minutes before occlusion and 1 hour after recirculation of the blood flow. One day after occlusion and recirculation, the whole body was perfused with physiologic saline through the heart, and the brain was extracted. The extracted brain was cooled with ice and water for 5 minutes, and cut at intervals of 2.0 mm to form 7 sections of the cerebral coronal surface. Each of the sections was stained with TTC (Triphenyltetrazolium Chloride), and fixed by a 5% neutral buffer formalin solution. In each of the sections, the infarction area in the right cerebral hemisphere was measured by an image analyzer (Olympus), and the infarction was evaluated by volume (mm³). The infarction volume was compared with the infarction volume of a control group to calculate the rate of inhibition of infarction. The results obtained are shown the table below.

**[Table]**

| Action to inhibit infarction in rat model of middle cerebral artery ischemia | | | | | |
|---|---|---|---|---|---|
| Compound | Rate of inhibition of infarction (%) | | Compound | Rate of inhibition of infarction (%) | |
| | 0.3 mg/kg | 3 mg/kg | | 0.3 mg/kg | 3 mg/kg |
| 1 | 19 | 60 | 22 | | 60 |
| 2 | | 17 | 25 | 32 | 50 |
| 10 | 56 | 66 | 26 | 41 | 55 |
| 12 | | 25 | 34 | 58 | 65 |
| 13 | | 16 | 36 | 62 | 89 |
| 14 | 16 | 21 | 37 | 55 | |
| 15 | | 13 | 38 | 29 | |
| 16 | | 18 | 39 | | 25 |
| 17 | 13 | 42 | 40 | 18 | |
| 18 | | 39 | 41 | 20 | |
| 19 | 22 | 32 | 42 | | 35 |
| 20 | 31 | | 44 | 12 | |

### Industrial Applicability

It was made apparent that these compounds of the present invention have the action to protect the cerebral nerve cells from various damages caused by occurrence of cerebral ischemia to inhibit evolution of infarction, and the action to prevent worsening of disease conditions of cerebral infarction. Therefore, it was found that the compounds of the present invention are useful as agents for curing·preventing cerebral stroke, traumatic cerebral diseases, cerebral edema, and cerebral neurodegenerative diseases.

## Claims

1. A quinolinomorphinan derivative or pharmacologically acceptable acid addition salt thereof represented by the following formula (II): 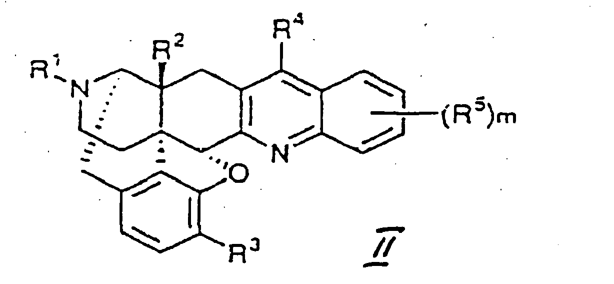 wherein
R¹ represents hydrogen alkyl having 1 to 5 carbon atoms, cycloalkylalkyl having 4 to 7 carbon atoms, cycloalkenylalkyl having 5 to 7 carbon atoms, aryl having 6 to 12 carbon atoms, aralkyl having 7 to 13 carbon atoms, alkenyl having 2 to 7 carbon atoms, alkanoyl having 1 to 5 carbon atoms, furan-2-ylalkyl (wherein an alkyl moiety has 1 to 5 carbon atoms), or thiophene-2-ylalkyl (wherein an alkyl moiety has 1 to 5 carbon atoms);
R² and R³ independently represent hydrogen, hydroxy, alkoxy having 1 to 5 carbon atoms, alkanoyloxy having 1 to 5 carbon atoms, aralkyloxy having 7 to 13 carbon atoms, or arylcarbonyloxy having 7 to 13 carbon atoms;
m represents an integer of 0 to 4;
R⁵ is each of m substituents on the benzene ring, which independently represent R¹⁸, or two R⁵ substituted at adjacent carbons form together a fused ring structure A (wherein residual 0 to 2 substituents R⁵ independently represent R¹⁸ or another fused ring structure A);
the fused ring structure A is a benzo, indeno, naphtha, pyrido, or cycloalkeno having 5 to 7 carbon atoms, which, is substituted by 0 to 4 substituents R⁹, or unsubstituted dioxoleno;
R⁹ and R¹⁸ (1) independently represent fluoro, chloro, bromo, iodo, nitro, hydroxy, alkyl having 1 to 5 carbon atoms, alkoxy having 1 to 5 carbon atoms, isothiocyanato, trifluoromethyl, trifluoromethoxy, cyano, phenyl, hydroxyalkyl having 1 to 3 carbon atoms, SR⁶, SOR⁶, SO₂R⁶, (CH₂)ₖCO₂R⁷, SO₂NR⁷R⁸, CONR⁷R⁸, (CH₂)ₖNR⁷R⁸, or (CH₂)ₖN(R⁷)COR⁸ (wherein k represents an integer of 0 to 5) and R⁶ represents alkyl having 1 to 5 carbon atoms, and R⁷ and R⁸ independently represent hydrogen, alkyl having 1 to 5 carbon atoms, or cycloalkylalkyl having 4 to 6 carbon atoms), and/or (2) R⁹ and R¹⁸ substituted at adjacent carbons with a ring junction therebetween form together any one of ethano, propane and o-benzeno bridged structures R⁹-R¹⁸;
R⁴ represents hydrogen, alkyl having 1 to 5 carbon atoms, hydroxyalkyl having 1 to 5 carbon atoms, aryl having 6 to 12 carbon atoms (which may be substituted by at least one substituents R¹⁷), NR¹⁰R¹¹, OR¹², COOR¹³ or CONR¹⁴R¹⁵, or any one of bridged structures R⁴-R⁵ of N(R¹⁶)CO, N(R¹⁶)C(=NH), N(R¹⁶)CH₂, o-benzeno, ethano, propano and butano, which are formed together by R⁴ and R⁵ substituted at the peri position;
R¹⁷ represents fluoro, chloro, bromo, iodo, nitro, amino, hydroxy, alkyl having 1 to 5 carbon atoms, alkoxy 1 to 5 carbon atoms, alkanoyloxy having 1 to 5 carbon atoms, trifuoromethyl, trifuoromethoxy or cyano;
R¹⁰, R¹¹, R¹² and R¹⁶ independently represent hydrogen, alkyl having 1 to 5 carbon atoms, cycloalkylalkyl having 4 to 7 carbon atoms, aralkyl having 7 to 13 atoms, or alkanoyl having 1 to 5 carbon atoms; and R¹³, R¹⁴ and R¹⁵ independently represent hydrogen, alkyl having 1 to 5 carbon atoms, aryl having 6 to 12 carbon atoms, or aralkyl having 7 to 13 carbon atoms,
wherein when R⁴ is hydrogen, and (1) when m is 1, R⁵ is R¹⁸ which represents hydroxy, and (2) when m is an integer of 2 to 4, R⁵ is R¹⁸ at least one of which represents hydroxy, or two R⁵ form together a fused ring structure A, and the residual 0 to 2 R⁵ independently represent R¹⁸ (wherein when the fused ring structure A is benzo, pyrido, or cycloalkeno having 5 to 7 carbon atoms, at least one R¹⁸ represents hydroxy); or at least one R⁹ and one R¹⁸ substituted at adjacent carbons with a ring junction therebetween form together a bridged structure R⁹-R¹⁸ which is any one of ethano, propano and o-benzeno), or must form another fused ring structure A); and
formula (II) includes (+) form, (-) form and (±) form].

2. A quinolinomorphinan derivative or pharmacologically acceptable acid addition salt thereof according to Claim 1, wherein R⁴ is hydrogen, alkyl having 1 to 5 carbon atoms, or NR¹⁰R¹¹, or R⁴ and R⁵ substituted at the peri position form together a bridged structure R⁴-R⁵ of N(R¹⁶)CO or N(R¹⁶)C(=NH).

3. A quinolinomorphinan derivative or pharmacologically acceptable acid addition salt thereof according to Claim 1. wherein R⁴ is hydroxyalkyl having 1 to 5 carbon atoms, aryl having 6 to 12 carbon atoms (which may be substituted by at least one substituent R¹⁷), OR¹², COOR¹³, or CONR¹⁴R¹⁵.

4. A quinolinomorphinan derivative or pharmacologically acceptable acid addition salt thereof according to Claim 2, wherein R⁵ is each of m substituents on the benzene ring, which are independently R¹⁸.

5. A quinolinomorphinan derivative or pharmacologically acceptable acid addition salt thereof according to Claim 2, wherein two R⁵ groups substituted at adjacent carbons form together a fused ring structure A (the remaining 0 to two R⁵ groups independently represent R¹⁸ or form another fused ring structure A).

6. A quinolinomorphinan derivative or pharmacologically acceptable acid addition salt thereof according to Claim 3. wherein R⁵ is each of m substituents on the benzene ring, which are independently R¹⁸.

7. A quinolinomorphinan derivative or pharmacologically acceptable acid addition salt thereof according to Claim 4, wherein R⁴ is hydrogen.

8. A quinolinomorphinan derivative or pharmacologically acceptable acid addition salt thereof according to Claim 4 wherein R⁴ is alkyl having 1 to 5 carbon atoms.

9. A quinolinomorphinan derivative or pharmacologically acceptable acid addition salt thereof according to Claim 4, wherein R⁴ is N¹⁰R¹¹.

10. A quinolinomorphinan derivative or pharmacologically acceptable acid addition salt thereof according to Claim 4, wherein R⁴ and R⁵ substituted at the peri position form together a bridged structure R⁴-R⁵of N(R¹⁶)CO or N(R¹⁶)C(=NH).

11. A quinolinomorphinan derivative or pharmacologically acceptable acid addition salt thereof according to Claim 5, wherein R⁴ is hydrogen.

12. A quinolinomorphinan derivative or pharmacologically acceptable acid addition salt thereof according to Claim 5, wherein R⁴ is NR¹⁰R¹¹.

13. A quinolinomorphinan derivative or pharmacologically acceptable acid addition salt thereof according to Claim 9, wherein R¹⁰ and R¹¹ are hydrogen.

14. A quinolinomorphinan derivative or pharmacologically acceptable acid addition salt thereof according to Claim 10, wherein R¹⁶ is hydrogen.

15. A medical composition containing an effective amount of quinolinomorphinan derivative or pharmacologically acceptable acid addition salt thereof according to anyone of the preceding claims.

## Patentansprüche

1. Chinolinomorphinan-Derivat oder pharmakologisch akzeptables Säureadditionssalz davon, repräsentiert durch die folgende Formel (II): 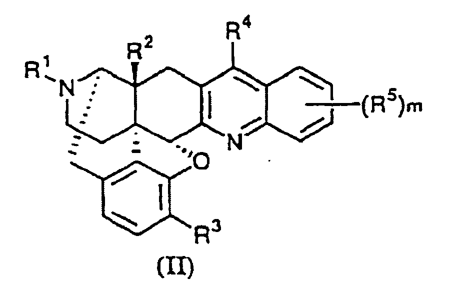 worin
R¹ Wasserstoffalkyl, das 1 bis 5 Kohlenstoffatome hat, Cycloalkylalkyl, das 4 bis 7 Kohlenstoffatome hat, Cycloalkenylalkyl, das 5 bis 7 Kohlenstoffatome hat, Aryl, das 6 bis 12 Kohlenstoffatome hat, Aralkyl, das 7 bis 13 Kohlenstoffatome hat, Alkenyl, das 2 bis 7 Kohlenstoffatome hat, Alkanoyl, das 1 bis 5 Kohlenstoffatome hat, Furan-2-ylalkyl (worin eine Alkylhälfte 1 bis 5 Kohlenstoffatome hat) oder Thiophen-2-ylalkyl (worin eine Alkylhälfte 1 bis 5 Kohlenstoffatome hat) repräsentiert;
R² und R³ unabhängig Wasserstoff, Hydroxy, Alkoxy, das 1 bis 5 Kohlenstoffatome hat, Alkanoyloxy, das 1 bis 5 Kohlenstoffatome hat, Aralkyloxy, das 7 bis 13 Kohlenstoffatome hat, oder Arylcarbonyloxy, das 7 bis 13 Kohlenstoffatome hat, repräsentiert;
m eine ganze Zahl von 0 bis 4 repräsentiert;
R⁵ jeder der m-Substituenten an dem Benzolring ist, die unabhängig R¹⁸ repräsentieren, oder zwei R⁵, die an benachbarten Kohlenstoffatomen substituiert sind, zusammen einen fusionierte Ringstruktur A bilden (worin die restlichen 0 bis 2 Substituenten R⁵ unabhängig R¹⁸ oder eine andere fusionierte Ringstruktur A bilden);
die fusionierte Ringstruktur A ein Benzo, Indeno, Naphtha, Pyrido oder Cycloalkeno ist, das 5 bis 7 Kohlenstoffatome hat und mit 0 bis 4 Substituenten R⁹ substituiert ist, oder ein unsubstituiertes Dioxoleno ist;
R⁹ und R¹⁸ (1) unabhängig Fluor, Chlor, Brom, Iod, Nitro, Hydroxy, Alkyl, das 1 bis 5 Kohlenstoffatome hat, Alkoxy, das 1 bis 5 Kohlenstoffatome hat, Isothiocyanato, Trifluormethyl, Trifluormethoxy, Cyano, Phenyl, Hydroxyalkyl, das 1 bis 3 Kohlenstoffatome hat, SR⁶, SOR⁶, SO₂R⁶, (CH₂)ₖCO₂R⁷, SO₂NR⁷R⁸, CONR⁷R⁸, (CH₂)ₖNR⁷R⁸ oder (CH₂)ₖN(R⁷)COR⁸ (worin k eine ganze Zahl von 0 bis 5 repräsentiert) und R⁶ Alkyl, das 1 bis 5 Kohlenstoffatome hat, repräsentiert und R⁷und R⁸ unabhängig Wasserstoff, Alkyl, das 1 bis 5 Kohlenstoffatome hat, oder Cycloalkylalkyl, das 4 bis 6 Kohlenstoffatome hat, repräsentieren) repräsentieren und/oder (2) R⁹ und R¹⁸, die an benachbarten Kohlenstoffatomen mit einer Ringverbindung dazwischen substituiert sind, zusammen irgendeine der Ethano-, Propano- oder o-Benzeno- überbrückten Ringstrukturen R⁹-R¹⁸ bilden;
R⁴ Wasserstoff, Alkyl, das 1 bis 5 Kohlenstoffatome hat, Hydroxyalkyl, das 1 bis 5 Kohlenstoffatome hat, Aryl, das 6 bis 12 Kohlenstoffatome hat (die mit mindestens einem Substituenten R¹⁷ substituiert sein können), NR¹⁰R¹¹ OR¹², COOR¹³ oder CONR¹⁴R¹⁵ oder irgendeine der überbrückten Ringstrukturen R⁴-R⁵ von N(R¹⁶)CO, N(R¹⁶)C(=NH), N(R¹⁶)CH₂, o-Benzeno, Ethano, Propano und Butano repräsentiert, die zusammen durch R⁴ und R⁵, substituiert in der peri-Position, gebildet werden;
R¹⁷ Fluor, Chlor, Brom, Iod, Nitro, Amino, Hydroxy, Alkyl, das 1 bis 5 Kohlenstoffatome hat, Alkoxy, das 1 bis 5 Kohlenstoffatome hat, Alkanoyloxy, das 1 bis 5 Kohlenstoffatome hat, Trifluormethyl, Trifluormethoxy oder Cyano repräsentiert;
R¹⁰, R¹¹, R¹² und R¹⁶ unabhängig Wasserstoff, Alkyl, das 1 bis 5 Kohlenstoffatome hat, Cycloalkylalkyl, das 4 bis 7 Kohlenstoffatome hat, Aralkyl, das 7 bis 13 Kohlenstoffatome hat, oder Alkanoyl, das 1 bis 5 Kohlenstoffatome hat, repräsentieren;
und R¹³, R¹⁴ und R¹⁶ unabhängig Wasserstoff, Alkyl, das 1 bis 5 Kohlenstoffatome hat, Aryl, das 6 bis 12 Kohlenstoffatome hat, oder Aralkyl, das 7 bis 13 Kohlenstoffatome hat, repräsentieren,
worin, wenn R⁴ Wasserstoff ist, und (1), wenn m 1 ist, R⁵ R¹⁸ ist, der Hydroxy repräsentiert, und (2), wenn m eine ganze Zahl von 2 bis 4 ist, R⁵ R¹⁸ ist, wobei mindestens einer davon Hydroxy repräsentiert, oder zwei R⁵ zusammen eine fusionierte Ringstruktur A bilden, und die restlichen 0 bis 2 R⁵ unabhängig R¹⁸ repräsentieren (worin, wenn die fusionierte Ringstruktur A Benzo, Pyrido oder Cycloalkeno ist, die 5 bis 7 Kohlenstoffatome hat, mindestens ein R¹⁸ Hydroxy repräsentiert), oder mindestens ein R⁹ und ein R¹⁸, die an benachbarten Kohlenstoffatomen mit einer Ringverbindung dazwischen substituiert sind, zusammen irgendeine überbrückte Ringstruktur R⁹-R¹⁸ bilden, die irgendeine aus Ethano-, Propano- oder o-Benzeno- ist), oder eine andere fusionierte Ringstruktur A bilden müssen); und
die Formel (II) die (+)-Form, (-)-Form und (±)-Form einschließt.

2. Chinolinomorphinan-Derivat oder pharmakologisch akzeptables Säureadditionssalz davon nach Anspruch 1, worin R⁴ Wasserstoff, Alkyl, das 1 bis 5 Kohlenstoffatome hat, oder NR¹⁰R¹¹ ist oder R⁴ und R⁵, die in der peri-Position substituiert sind, zusammen eine überbrückte Ringstruktur R⁴-R⁵ von N(R¹⁶)CO oder N(R¹⁶)C(=NH) bilden.

3. Chinolinomorphinan-Derivat oder pharmakologisch akzeptables Säureadditionssalz davon nach Anspruch 1, worin R⁴ Hydroxyalkyl, das 1 bis 5 Kohlenstoffatome hat, Aryl, das 6 bis 12 Kohlenstoffatome hat, (die mit mindestens einem Substituenten R¹⁷ substituiert sein können), OR¹², COOR¹³ oder CONR¹⁴R¹⁵ ist.

4. Chinolinomorphinan-Derivat oder pharmakologisch akzeptables Säureadditionssalz davon nach Anspruch 2, worin R⁵ jeder der m-Substituenten an dem Benzolring ist, die unabhängig R¹⁸ sind.

5. Chinolinomorphinan-Derivat oder pharmakologisch akzeptables Säureadditionssalz davon nach Anspruch 2, worin zwei R⁵ -Gruppen, die an den benachbarten Kohlenstoffatomen substituiert sind, zusammen eine fusionierte Ringstruktur A bilden (wobei die restlichen 0 bis zwei R⁵-Gruppen unabhängig R¹⁸ repräsentieren oder eine weitere fusionierte Ringstruktur A bilden).

6. Chinolinomorphinan-Derivat oder pharmakologisch akzeptables Säureadditionssalz davon nach Anspruch 3, worin R⁵ jeder der m-Substituenten an dem Benzolring ist, die unabhängig R¹⁸ sind.

7. Chinolinomorphinan-Derivat oder pharmakologisch akzeptables Säureadditionssalz davon nach Anspruch 4, worin R⁴ Wasserstoff ist.

8. Chinolinomorphinan-Derivat oder pharmakologisch akzeptables Säureadditionssalz davon nach Anspruch 4, worin R⁴ Alkyl ist, das 1 bis 5 Kohlenstoffatome hat.

9. Chinolinomorphinan-Derivat oder pharmakologisch akzeptables Säureadditionssalz davon nach Anspruch 4, worin R⁴ NR¹⁰R¹¹ ist.

10. Chinolinomorphinan-Derivat oder pharmakologisch akzeptables Säureadditionssalz davon nach Anspruch 4, worin R⁴ und R⁵, die in der peri-Position substituiert sind, zusammen eine überbrückte Ringstruktur R⁴-R⁵ von N(R¹⁶)CO oder N(R¹⁶)C(=NH) bilden.

11. Chinolinomorphinan-Derivat oder pharmakologisch akzeptables Säureadditionssalz davon nach Anspruch 5, worin R⁴ Wasserstoff ist.

12. Chinolinomorphinan-Derivat oder pharmakologisch akzeptables Säureadditionssalz davon nach Anspruch 5, worin R⁴ NR¹⁰R¹¹ ist.

13. Chinolinomorphinan-Derivat oder pharmakologisch akzeptables Säureadditionssalz davon nach Anspruch 9, worin R¹⁰ und R¹¹ Wasserstoff sind.

14. Chinolinomorphinan-Derivat oder pharmakologisch akzeptables Säureadditionssalz davon nach Anspruch 10, worin R¹⁶ Wasserstoff ist.

15. Medizinische Zusammensetzung, die eine wirksame Menge eines Chinolinomorphinan-Derivats oder eines pharmakologisch akzeptablen Säureadditionssalzes davon entsprechend irgendeinem der vorhergegangenen Ansprüche umfasst.

## Revendications

1. Dérivé de quinoléinomorphinane ou sel d'addition avec un acide pharmacologiquement acceptable de celui-ci, représenté par la formule (II) : 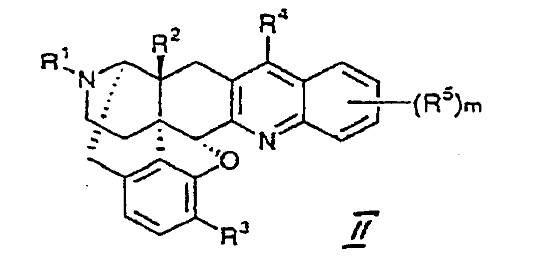 dans laquelle
R¹ représente un atome d'hydrogène, un groupe alkyle comportant de 1 à 5 atomes de carbone, cycloalkylalkyle comportant de 4 à 7 atomes de carbone, cycloalcénylalkyle comportant de 5 à 7 atomes de carbone, aryle comportant de 6 à 12 atomes de carbone, aralkyle comportant de 7 à 13 atomes de carbone, alcényle comportant de 2 à 7 atomes de carbone, alcanoyle comportant de 1 à 5 atomes de carbone, furan-2-ylalkyle (dans lequel la partie alkyle comporte de 1 à 5 atomes de carbone), ou thiophène-2-ylalkyle (dans lequel la partie alkyle comporte de 1 à 5 atomes de carbone) ;
R² et R³ représentent indépendamment un atome d'hydrogène, un groupe hydroxy, alkoxy comportant de 1 à 5 atomes de carbone, alcanoyloxy comportant de 1 à 5 atomes de carbone, aralkyloxy comportant de 7 à 13 atomes de carbone, ou arylcarbonyloxy comportant de 7 à 13 atomes de carbone ;
m représente un entier de 0 à 4 ;
R⁵ représente chacun parmi m substituants sur le cycle benzénique qui représentent indépendamment R¹⁸, ou deux groupes R⁵ substitués au niveau des atomes de carbone adjacents, forment ensemble une structure cyclique condensée A (dans laquelle de 0 à 2 substituants R⁵ restants représentent indépendamment R¹⁸ ou une autre structure cyclique condensée A) ;
la structure cyclique condensée A est un groupe benzo, indéno, naphta, pyrido ou cycloalcéno comportant de 5 à 7 atomes de carbone, qui est substitué par 0 à 4 substituants R⁹, ou dioxoléno non substitué ;
R⁹ et R¹⁸ (1) représentent indépendamment un atome de fluore, chlore, brome, iode, un groupe nitro, hydroxy, alkyle comportant de 1 à 5 atomes de carbone, alkoxy comportant de 1 à 5 atomes de carbone, isothiocyanato, trifluorométhyle, trifluorométhoxy, cyano, phényle, hydroxyalkyle comportant de 1 à 3 atomes de carbone, SR⁶, SOR⁶, SO₂R⁶, (CH₂)ₖCO₂R⁷, SO₂NR⁷R⁸, CONR⁷R⁸, (CH₂)ₖNR⁷R⁸ ou (CH2)kN(R⁷)COR⁸ (dans lesquels k représente un entier de 0 à 5), et R⁶ représente un groupe alkyle comportant de 1 à 5 atomes de carbone, et R⁷ et R⁸ représentent indépendamment un atome d'hydrogène, un groupe alkyle comportant de 1 à 5 atomes de carbone, ou cycloalkylalkyle comportant de 4 à 6 atomes de carbone, et/ou R⁹ et R¹⁸ (2), substitués au niveau des atomes de carbone adjacents avec une jonction de cycle entre ceux-ci, forment ensemble une quelconque des structures pontées R⁹-R¹⁸ éthano, propano et o-benzéno ;
R⁴ représente un atome d'hydrogène, un groupe alkyle comportant de 1 à 5 atomes de carbone, hydroxyalkyle comportant de 1 à 5 atomes de carbone, aryle comportant de 6 à 12 atomes de carbone (qui peut être substitué par au moins un substituant R¹⁷), NR¹⁰R¹¹, OR¹², COOR¹³ ou CONR¹⁴R¹⁵, ou une quelconque des structures pontées R⁴-R⁵ choisies parmi N(R¹⁶)CO, N(R¹⁶)C(=NH), N(R¹⁶)CH₂, o-benzéno, éthano, propano et butano, qui sont formées ensemble par R⁴ et R⁵ substitués à la position péri ;
R¹⁷ représente un atome de fluore, chlore, brome, iode, un groupe nitro, amino, hydroxy, alkyle comportant de 1 à 5 atomes de carbone, alcoxy comportant de 1 à 5 atomes de carbones, alcanoyloxy comportant de 1 à 5 atomes de carbones, trifluorométhyle, trifluorométhoxy ou cyano ;
R¹⁰, R¹¹, R¹² et R¹⁶ représentent indépendamment un atome d'hydrogène, un groupe alkyle comportant de 1 à 5 atomes de carbone, cycloalkylalkyle comportant de 4 à 7 atomes de carbone, aralkyle comportant de 7 à 13 atomes de carbone, ou alcanoyle comportant de 1 à 5 atomes de carbone ; et R¹³, R¹⁴ et R¹⁵ représentent indépendamment un atome d'hydrogène, un groupe alkyle comportant de 1 à 5 atomes de carbone, aryle comportant de 6 à 12 atomes de carbone, ou aralkyle comportant de 7 à 13 atomes de carbone,
dans lequel, lorsque R⁴ représente un atome d'hydrogène, et (1) lorsque m est égal à 1, R⁵ est R¹⁸ qui représente un groupe hydroxy, et (2) lorsque m est un entier de 2 à 4, R⁵ est R¹⁸, dont au moins un représente un groupe hydroxy, ou deux groupes R⁵ forment ensemble une structure cyclique condensée A, et les 0 à 2 groupes R⁵ restants représentent indépendamment R¹⁸ (dans lequel, lorsque la structure cyclique condensée A est un groupe benzo, pyrido ou cycloalcéno comportant de 5 à 7 atomes de carbone, au moins un groupe R¹⁸ représente un groupe hydroxy), ou au moins un groupe R⁹ et au moins un groupe R¹⁸, substitués au niveau des atomes de carbone adjacents avec une jonction de cycle entre ceux-ci, forment ensemble une structure pontée R⁹-R¹⁸ qui est un quelconque groupe choisi parmi les groupes éthano, proprano et o-benzéno, ou ils doivent former une autre structure cyclique condensée A ; et
la formule (II) comprend la forme (+), la forme (-) et la forme (±).

2. Dérivé de quinoléinomorphinane ou sel d'addition avec un acide pharmacologiquement acceptable de celui-ci selon la revendication 1, dans lequel R⁴ représente un atome d'hydrogène, un groupe alkyle comportant de 1 à 5 atomes de carbone, ou NR¹⁰R¹¹, ou R⁴ et R⁵, substitués à la position péri, forment ensemble une structure pontée R⁴-R⁵ de type N(R¹⁶)CO ou N(R¹⁶)C(=NH).

3. Dérivé de quinoléinomorphinane ou sel d'addition avec un acide pharmacologiquement acceptable de celui-ci selon la revendication 1, dans lequel R⁴ représente un groupe hydroxyalkyle comportant de 1 à 5 atomes de carbone, aryle comportant de 6 à 12 atomes de carbone (qui peut être substitué par au moins un substituant R¹⁷), OR¹², COOR¹³ ou CONR¹⁴R¹⁵.

4. Dérivé de quinoléinomorphinane ou sel d'addition avec un acide pharmacologiquement acceptable de celui-ci selon la revendication 2, dans lequel R⁵ représente chacun parmi m substituants sur le cycle benzénique, qui représentent indépendamment R¹⁸,

5. Dérivé de quinoléinomorphinane ou sel d'addition avec un acide pharmacologiquement acceptable de celui-ci selon la revendication 2, dans lequel deux groupes R⁵, substitués au niveau des atomes de carbone adjacents, forment ensemble une structure cyclique condensée A (les 0 à 2 groupes R⁵ restants représentent indépendamment R¹⁸ ou forment une autre structure cyclique condensée A).

6. Dérivé de quinoléinomorphinane ou au sel d'addition avec un acide pharmacologiquement acceptable de celui-ci selon la revendication 3, dans lequel R⁵ représente chacun parmi m substituants sur le cycle benzénique, qui représentent indépendamment R¹⁸.

7. Dérivé de quinoléinomorphinane ou sel d'addition avec un acide pharmacologiquement acceptable de celui-ci selon la revendication 4, dans lequel R⁴ est un atome d'hydrogène.

8. Dérivé de quinoléinomorphinane ou au sel d'addition avec un acide pharmacologiquement acceptable de celui-ci selon la revendication 4, dans lequel R⁴ est un groupe alkyle comportant de 1 à 5 atomes de carbone.

9. Dérivé de quinoléinomorphinane ou sel d'addition avec un acide pharmacologiquement acceptable de celui-ci selon la revendication 4, dans lequel R⁴ représente NR¹⁰R¹¹,

10. Dérivé de quinoléinomorphinane ou sel d'addition avec un acide pharmacologiquement acceptable de celui-ci selon la revendication 4, dans lequel R⁴ et R⁵, substitués à la position péri, forment ensemble une structure pontée R⁴-R⁵ de type N(R¹⁶)CO ou N(R¹⁶)C(=NH).

11. Dérivé de quinoléinomorphinane ou sel d'addition avec un acide pharmacologiquement acceptable de celui-ci selon la revendication 5, dans lequel R⁴ est un atome d'hydrogène.

12. Dérivé de quinoléinomorphinane ou sel d'addition avec un acide pharmacologiquement acceptable de celui-ci selon la revendication 5, dans lequel R⁴ représente NR¹⁰R¹¹

13. Dérivé de quinoléinomorphinane ou sel d'addition avec un acide pharmacologiquement acceptable de celui-ci selon la revendication 9, dans lequel R¹⁰ et R¹¹ sont des atomes d'hydrogène.

14. Dérivé de quinoléinomorphinane ou sel d'addition avec un acide pharmacologiquement acceptable de celui-ci selon la revendication 10, dans lequel R¹⁶ est un atome d'hydrogène.

15. Composition médicale contenant une quantité efficace d'un dérivé de quinoléinomorphinane ou d'un sel d'addition avec un acide pharmacologiquement acceptable de celui-ci selon l'une quelconque des revendications précédentes.
